(19) Europäisches Patentamt
European Patent Office
Office européen des brevets

(11) EP 3 768 336 B1

(12) EUROPEAN PATENT SPECIFICATION

(45) Date of publication and mention of the grant of the patent:
**06.09.2023 Bulletin 2023/36**

(21) Application number: **19714730.9**

(22) Date of filing: **19.03.2019**

(51) International Patent Classification (IPC):
***A61L 27/22*** *(2006.01)* ***A61L 27/24*** *(2006.01)*
***A61L 27/56*** *(2006.01)* ***A61L 27/58*** *(2006.01)*
***A61L 27/60*** *(2006.01)*

(52) Cooperative Patent Classification (CPC):
(C-Sets available)
**A61L 27/56; A61L 27/22; A61L 27/225; A61L 27/227; A61L 27/24; A61L 27/58; A61L 27/60** (Cont.)

(86) International application number:
**PCT/GB2019/050769**

(87) International publication number:
**WO 2019/180423 (26.09.2019 Gazette 2019/39)**

(54) **SCAFFOLD**

GERÜST

ÉCHAFAUDAGE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.03.2018 GB 201804366**

(43) Date of publication of application:
**27.01.2021 Bulletin 2021/04**

(73) Proprietor: **Oxford University Innovation Limited**
**Oxford, Oxfordshire OX2 0JB (GB)**

(72) Inventors:
- **LIM, Xuxin**
  **Headington, Oxford OX3 7DQ (GB)**
- **DYE, Julian**
  **Headington, Oxford OX3 7DQ (GB)**

(74) Representative: **HGF**
**HGF Limited**
**1 City Walk**
**Leeds LS11 9DX (GB)**

(56) References cited:
**WO-A1-2013/164635 WO-A1-2017/182676**
**WO-A1-2017/183710 US-A1- 2009 017 092**

- **ANDREA BARBETTA ET AL: "Enzymatic Cross-Linking versus Radical Polymerization in the Preparation of Gelatin PolyHIPEs and Their Performance as Scaffolds in the Culture of Hepatocytes", BIOMACROMOLECULES, vol. 7, no. 11, 1 November 2006 (2006-11-01), pages 3059-3068, XP55596037, US ISSN: 1525-7797, DOI: 10.1021/bm060533l**
- **M DE COLLI ET AL: "A biomimetic porous hydrogel of gelatin and glycosaminoglycans cross-linked with transglutaminase and its application in the culture of hepatocytes", BIOMEDICAL MATERIALS, vol. 7, no. 5, 26 July 2012 (2012-07-26), page 055005, XP55596018, GB ISSN: 1748-6041, DOI: 10.1088/1748-6041/7/5/055005**
- **LIM XUXIN ET AL: "Manufacture and characterisation of EmDerm-novel hierarchically structured bio-active scaffolds for tissue regeneration", JOURNAL OF MATERIALS SCIENCE: MATERIALS IN MEDICINE, SPRINGER NEW YORK LLC, UNITED STATES, vol. 29, no. 6, 5 June 2018 (2018-06-05), pages 1-12, XP036597954, ISSN: 0957-4530, DOI: 10.1007/S10856-018-6060-6 [retrieved on 2018-06-05]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61L 27/227, C08L 89/06**

## Description

### FIELD OF THE INVENTION

This invention relates to a method for preparing a porous scaffold for supporting the growth of biological tissue. The present invention also relates to a porous protein scaffold for supporting the growth of biological tissue. Disclosed is also the use of the porous protein scaffolds obtained for surgical implantation into a wound site or tissue defect or other site to support the repair or regrowth of the tissue. The disclosure further relates to uses of the porous protein scaffold and to a tissue-engineered construct obtained therefrom.

### BACKGROUND

Hierarchical interconnected porous architecture and nano-scaled structure are fundamental requirements of three-dimensional protein-based bio-intelligent scaffolds, essential for the functions of cell conductivity, nutrient perfusion, angiogenesis and vasculogenic differentiation and neuronal ingrowth. Such structural requirements are also fundamental to the development of biomaterials, which may be used in applications such as wound healing and tissue regeneration within a patient. However, there is a need for effective, controllable, scalable methods of producing such nanostructured scaffolds.

One method that may be used for achieving controlled porosity in protein hydrogels is controlled freezing and lyophilisation. In this method, pores are formed by material exclusion from the ice crystal porogen. This may result in dense lamellar structured material, largely devoid of nanoscale structure. This is shown by many of the current scaffolds, notably acellular collagen scaffolds. Cellularisation and vascularisation into such materials may be relatively slow.

Foam formation may be another method for achieving controlled porosity in protein hydrogels. However, this may also have some limitations, due to a large exposed air interface for protein denaturation, intrinsic bubble instability and foam drainage during gelation and cross-linking. These can cause difficulty in achieving a biologically acceptable degree of homogeneity and can result large pore defects due to collapse of foam bubbles during manufacture.

Recent attention has been focussed on the powerful and highly controllable bottom-up manufacture methodologies. Electrospinning is an established method of forming micro and nano-scale fibre meshes, but may not be amenable to manufacturing structures at the thickness and consistency for the commercial scale-up required for marketing three-dimensional scaffolds. While 3D-printing and rapid-prototyping are also emerging technologies which are able to create soft scaffold structures, the concept of building macroscale structure from a nano-scaled filament at the scale required for commercial manufacture, remains challenging. Therefore, new methods of controllable rapid and versatile manufacture of nanostructured regenerative biomaterials would represent a significant advance in healthcare technology.

US 2009/017092 describes a method for preparing an artificial extra cellular matrix, which involves making an oil in water emulsion from a water solution of extra cellular matrix molecules, a fluorocarbon and a biocompatible emulsifier.

WO 2017/182676 describes a method of forming a scaffold, which includes preparing a high internal phase emulsion (HIPE) comprising an aqueous solution of: proteins selected from the group consisting of collagen and mixtures of collagen and chitosan.

A. Barbetta et al. (Biomacromolecules 2006, 7(11), 3059-3068) describes porous biodegradable scaffolds based on gelatin A3.

M. De Colli et al. (Biomed. Mater. 2012, 7(5), 055005) describes a blended gelatin and glycosaminoglycan scaffold, which was obtained used a concentrated emulsion templating technique known as high internal phase emulsion (HIPE).

WO 2013/164635 describes a process or preparing an extracellular matrix composition which comprises: (a) mixing an aqueous solution of fibrinogen with a coagulating agent and a bulking agent and a foaming agent; and (b) causing the mixture to foam and coagulate.

WO 2017/183710 describes a method for producing a porous gelatin sheet having cell proliferation properties.

It is among the objects of embodiments of the present invention to develop an improved method for producing a porous protein scaffold for supporting the growth of biological tissue.

### SUMMARY OF THE INVENTION

The invention provides a method of preparing a porous protein scaffold for supporting the growth of biological tissue. The method comprises: providing an oil-in water emulsion comprising oil droplets dispersed in a continuous phase comprising a pH-buffered aqueous protein solution, wherein the oil-in-water emulsion comprises a non-ionic surfactant or a mixture of non-ionic surfactants, wherein the non-ionic surfactant is in an amount of 0.01 to 10 volume % of the total volume of the oil phase in the oil-in-water emulsion, wherein a HLB of the non-ionic surfactant or the surfactant mixture is 10 to 16; gelling the protein around the oil droplets; removing the oil droplets from the continuous

phase after formation of a scaffold; and incubating the scaffold with an excipient solution. The porous protein scaffold is typically a porous native protein scaffold.

The invention also provides a porous protein scaffold. The porous protein scaffold is obtainable from the method of the invention using a gellable protein selected from at least one of collagen, fibrinogen, fibronectin, laminin and elastin, wherein the porous protein scaffold comprises an interconnected fibrous structure of proteins and has an average pore size in the range of 80 to 200 microns.

The invention also relates to uses and methods involving the porous protein scaffold.

The porous protein scaffold may be for use in repairing tissue or tissue regeneration in a human or an animal. For example, the porous protein scaffold may be used in wound healing.

A method of repairing tissue or tissue regeneration in a human or an animal is described. The method comprises applying or implanting the porous protein scaffold at a site, such as a wound, in a human or animal body. Typically, the site is an area of the human or animal body in need of tissue regeneration or repair.

The invention also relates an *in vitro* or ex *vivo* method of (i) manufacturing or engineering a tissue or (ii) manufacturing a tissue-engineered construct. Each method comprises applying cells to the porous protein scaffold of the invention.

The invention further provides a tissue-engineered construct. The tissue-engineered construct is obtainable from a method of the invention. Additionally or alternatively, the tissue-engineered construct comprises cells or a tissue supported on the porous protein scaffold.

The invention also relates to uses and methods involving the tissue-engineered construct.

The tissue-engineered construct may be for use in the treatment of the human or animal body.

A method of treating a human or an animal is described. The method comprises applying or implanting the tissue-engineered construct at a site, such as a wound, in the human or animal body.

## BRIEF DESCRIPTION OF THE DRAWINGS

Embodiments of the invention are further described below, by way of example, with reference to the accompanying drawings.

Figure 1 provides scanning electron microscopy images of a number of emulsion-templated EmDerm (emulsion-templated dermal) scaffolds in accordance with an embodiment of the invention.

Figure 2 provides graphs showing scaffold degradation time of EmDerm scaffolds fabricated using emulsions made with varying surfactant concentrations according to embodiments of the present invention.

Figure 3 provides graphs showing proliferation of cell types in EmDerm scaffolds post lyophilisation with different excipients according to embodiments of the present invention. For the histograms shown in A., the first three bars (from left to right on the x-axes) relate to the excipient "P68", the second three bars relate to the excipient "M" and the final three bars relate to "NIL" excipient. For the histograms shown in B., the first three bars (from left to right on the x-axes) relate to the excipient "PVA", the second three bars relate to the excipient "M", the next three bars relate to the excipient "P68" and the final three bars relate to the "PEG" excipient. For the histograms shown in C., the first three bars (from left to right on the x-axes) relate to the excipient "PVA", the second three bars relate to the excipient "M", the next three bars relate to the excipient "PEG" and the final three bars relate to "NIL" excipient.

Figure 4 provides graphs showing proliferation of cell types in EmDerm scaffolds according to embodiments of the present invention. In the histograms shown in A. to C. the % surfactant on each x-axis from left to right is 0.1, 0.3, 0.5 and 0.7 respectively.

Figure 5 provides graphs showing proliferation of cell types in EmDerm scaffolds according to embodiments of the present invention in comparison with commercial comparator scaffolds. In the histograms shown, the first three bars (from left to right on the x-axes) relate to the scaffold type "0.1C-P68", the second three bars relate to the scaffold type "0.1CF-P68", the next three bars relate to the scaffold type "0.1F-P68", the fourth set of three bars relate to the scaffold type "MATRIDERM" and the final three bars relate to the "INTEGRA" scaffold type.

Figure 6 provides wide-field microscope images of EmDerm scaffolds according to embodiments of the present invention cultured with different cell types for 7 days.

Figure 7 provides light microscopy images of Oil Red O stained emulsions made with differing HLB values according to embodiments of the present invention.

Figures 8A and 8B provide light microscopy images of Oil Red O stained emulsions with varying mixing speeds and surfactant concentrations, and summary measurements of droplet diameter according to embodiments of the present invention. Figure 8A shows the effect of shear rate on droplet size of HLB 13 emulsion mixtures with 0.75% surfactant mix and 0.8% Kollidon concentration. Increasing shear rate causes significant reduction in droplet sizes (mean ± SD, *p<0.05, **P<0.01, ****P<0.0001). Figure 8B shows the combined effect of shear rate and surfactant concentration on droplet diameter. In the top histogram, the first bar in each group of bars represents 0.25 SA, the second bar represents 0.5 SA, the third bar represents 0.75 SA, the fourth bar represents 1.0 SA and the fifth bar represents 1.5 SA. In the bottom histogram, the first bar in each group of bars represents "1500", the second bar represents "2000", the third bar represents "2500" and the fourth bar represents "3000".

Figure 9 provides light microscopy images of Oil Red O stained emulsions with differing surfactant concentrations according to embodiments of the present invention.

Figure 10 provides light microscopy images of Oil Red O stained emulsions with varying PVP concentrations according to embodiments of the present invention.

Figure 11 provides light microscopy images of Oil Red O stained emulsions at varying temperatures according to embodiments of the present invention.

Figure 12A to 12F provide emulsion stability profiles for emulsions over varying HLB values, PVP concentrations and surfactant concentrations according to embodiments of the present invention.

Figure 13 provides viscoelasticity characterisation of emulsions according to embodiments of the present invention.

Figure 14 provides confocal microscopy images of BSA-FITC emulsions according to embodiments of the present invention.

Figure 15 provides relative fluorescence intensities in the aqueous and oil phase of emulsions according to embodiments of the present invention. In each of the histograms, the aqueous phase is represented by the first bar (left to right along the x-axis) and the oil phase is represented by the second bar.

Figure 16 provides FTIR spectra of emulsions according to embodiments of the present invention.

Figure 17 provides circular dichroism spectra of bovine serum albumin mixed with an emulsion according to embodiments of the present invention.

Figure 18 provides tryptophan fluorescence assays for fibrinogen mixed with emulsions with varying surfactant and PVP concentrations according to embodiments of the present invention.

Figure 19 provides enzymatic assays for LDH mixed with emulsions with varying surfactant and PVP concentrations according to embodiments of the present invention.

Figure 20 is a schematic illustration of an emulsification apparatus described in Example 4;

Figure 21 is a graph illustrating how emulsion droplet diameter may be controlled by impellor speed as described in Example 4 (e.g. directly controlled by theoretical shear rate of the impellor at the corresponding speed).

Figure 22 are a series of histograms showing the cell proliferation outcome of co-cultures comparing EmDerm and controls.

Figure 23 shows a series of images of the co-culture of cells on EmDerm-C scaffolds where (A) is for HDF/HDE and (B) is for MSC/HDE.

Figure 24 shows a series of images of the co-culture of cells, where (C) is for HEK/HDF on the EmDerm-C scaffold and (A) is for HDF/HDE on the EmDerm-CF scaffold.

Figure 25 shows a series of images of the co-culture of cells on the EmDerm-CF scaffold where (B) is for MSC/HDE and (C) is for HEK/HDF.

Figure 26 shows a series of images of the co-culture of cells on the EmDerm-F scaffold where (A) is for HDF/HDE and (B) is for MSC/HDE.

Figure 27 shows a series of images of the co-culture of cells on the Integra scaffold where (A) is for HDF/HDE and (B) is for MSC/HDE.

Figure 28 shows a series of images of the co-culture of cells on the Integra or the Matriderm scaffolds where (C) is for HEK/HDF on the Integra scaffold and (A) is for HDF/HDE on the Matriderm scaffold.

Figure 29 shows a series of images of the co-culture of cells on the Matriderm scaffold where (B) is for MSC/HDE and (C) is for HEK/HDF.

Figure 30 shows SEM images of keratinocytes seeded on EmDerm-C (A), EmDerm-CF (B), Integra (C) and Matriderm (D).

Figure 31 is a schematic representation of an *in vivo* experimental plan (not according to the claimed invention).

Figure 32 shows a day 7 biopsy of EmDerm-C with abraded on the left and non-abraded on the right.

Figure 33 shows a day 7 biopsy of EmDerm-CF with abraded on the left and non-abraded on the right.

Figure 34 shows a day 7 biopsy of EmDerm-F with abraded on the left and non-abraded on the right.

Figure 35 shows a day 14 biopsy of EmDerm-C (abraded).

Figure 36 shows a day 14 biopsy of EmDerm-CF (abraded).

Figure 37 shows a day 14 biopsy of EmDerm-F (abraded).

Figure 38 shows a day 14 biopsy of EmDerm-C (non-abraded).

Figure 39 shows a day 14 biopsy of EmDerm-CF (non-abraded).

Figure 40 shows a day 14 biopsy of EmDerm-F (non-abraded).

Figure 41 are histograms showing the mean number of blood vessels in biopsies.

Figure 42 are histograms showing the mean grade of inflammation in biopsies.

## DESCRIPTION

In accordance with an aspect of the present invention, there is provided a method of preparing a porous protein scaffold for supporting the growth of biological tissue. The method comprises providing an oil-in water emulsion comprising droplets of oil dispersed in a continuous phase comprising a pH-buffered aqueous protein solution. The oil-in-water emulsion comprises a non-ionic surfactant in an amount of 0.01 to 10 volume % of the total volume of the oil (i.e. hydrophobic phase) in the oil-in-water emulsion. The method further comprises gelling the protein around the oil droplets, removing the oil droplets from the continuous phase after formation of a scaffold; and incubating the scaffold with an excipient solution.

The method of the present disclosure provides an effective method for the manufacture of scaffolds for supporting the growth of biological cells. In particular, the method of the present disclosure employs an oil-in-water emulsion that can act as a template for making a porous protein scaffold. By gelling the protein around the oil droplets, a scaffold having a desired pore structure may be produced. As the oil droplets are not directly involved in the protein gelation, the oil can subsequently be removed (e.g. eluted) to provide a porous protein scaffold for supporting cell growth.

Emulsion templating methods are known. However, to date, such methods have not been employed for the manufacture of porous protein scaffolds, in particular where the protein is in a native secondary and tertiary configuration. Without wishing to be bound by any theory, this is believed to be because of difficulties associated with the stability and compatibility between an oil emulsion system and the scaffold protein, protein denaturation as well as the subsequent removal of the oil phase. For example, it has been found that if ionic surfactants, for example, sodium dodecyl sulphate

(SDS) or dodecyl trimethylamine chloride (DTMA), are used as the sole surfactants in the emulsion, gelation of the protein may be inhibited or prevented. In addition, ionic surfactants, such as decanoic acid, may fail to form stable emulsions, resulting in increased risk of separation during the emulsion templating process.

It has now been found that the problems of denaturation and/or emulsion breakdown and/or instability can be reduced by using non-ionic surfactants in certain amounts. The amount of non-ionic surfactant relative to the amount of oil in an oil-in-water emulsion may also be controlled to control the oil droplet size and, hence, pore size of the final scaffold.

Unlike methods in the prior art, the method of the invention can produce a porous protein scaffold where the protein is not denatured. This allows the production of a porous protein scaffold where the microstructure of the proteins is preserved, such that they form an interconnected fibrous structure. The production of the scaffold may be achieved by enzymatic activity in the aqueous phase, by non-enzymatic chemical reaction or by thermally controlled gelation of protein molecules. The interconnected fibrous structure may facilitate the formation of functional structures within the tissue that is grown on the scaffold, such as vasculature when creating a dermal layer of skin. In contrast, the protein scaffolds in the prior art are generally smooth and featureless.

The step of gelling the protein around the oil droplets may be performed using a gelation agent. The gelation agent may be added to the oil-in water emulsion to bring about gelling of the protein around the oil droplets. Alternatively, the gelation agent may be included in the oil-in water emulsion at the outset. Thus, the oil-in water emulsion may comprise the gelation agent.

The gelation agent may be an enzymatic gelation agent, such as thrombin.

The gelation agent may be a non-enzymatic gelation agent. The non-enzymatic gelation agent may be a chemical agent or a cross-linking agent, such as genipin. The non-enzymatic gelation agent may be an ionic cross-linking agent, such as calcium chloride.

*Proteins*

As described above, the method of the present disclosure involves providing an oil-in water emulsion comprising droplets of oil dispersed in a continuous phase comprising a pH-buffered aqueous protein solution.

The aqueous protein solution may comprise a native aqueous protein solution. A native protein is a protein that is in its properly folded and/or assembled conformation or form which is associated with its specific biological activity, i.e. is operative and functional. In other words, a native protein has not been altered by, for example, a denaturing agent such as heat, chemicals, or enzyme action, which could result in partial or complete unfolding. The tertiary, folded structure of a native protein renders the protein capable of performing its biological function. In other words, a native protein is a characterized protein that possesses the ability to perform one or more biological functions that said protein would be able to perform within its native environment.

Examples of proteins that may be used as, for example, the primary structural component of a scaffold of the invention include collagen, fibrinogen, fibronectin, laminin and elastin.

Typically, the protein is a gellable protein, such as a gellable protein selected from at least one of collagen, fibrinogen, fibronectin, laminin and elastin.

It is preferred that the protein includes collagen or fibrinogen. More preferably, the protein includes collagen.

The collagen may be any type or form of extracted dissolved or suspended collagen solution. The collagen may be any form of collagen solution which is gellable. The collagen may be fibrillar (Type I, II, III, V, XI) or non-fibrillar (IX, XII, XIV, XIX, XXI, VIII, X, IV, XV, XVIII, XIII, XVII, VI, VII). The collagen may be acid-dissolved collagen, wherein the acid is preferably acetic acid. In one embodiment, the protein is type I collagen, for example acid extracted type I collagen.

The collagen may, for example, be human collagen, porcine collagen, bovine collagen or rat tail collagen. Preferably, the collagen is human collagen, porcine collagen or bovine collagen. More preferably, the collagen is human collagen or porcine collagen.

The aqueous protein solution may comprise a single protein, or a mixture of one or more proteins, or a mixture of a protein or proteins with peptides. In one embodiment, a peptide or denatured protein such as a gelatin or silk fibroin may be used in a blend with a native protein.

In addition, other biological materials or macromolecules, such as glycosaminoglycans (e.g. chondroitin sulphate, dermatan sulphate, heparin sulphate and hyaluronic acid) and polysaccharides, for example, chitosan and alginate may be used in combination with the protein in the continuous phase. These other biological macromolecules may act to improve the specificity of the scaffold. For example, extracellular matrix glycosaminoglycans may be useful as coacervate constituents of compound formulations with the primary protein constituent.

Other biological materials can be added to the scaffold. These other biological materials may act to improve the specificity of the scaffold. Other biological materials may include hydroxyapatite, tri-calcium phosphates and other minerals, such as amorphous calcium phosphate.

The biological material may be selected from at least one of glycosaminoglycans, alginates, polysaccharides

and calcium phosphate particles, such as hydroxyapatite.

The amount of structural scaffold protein in the final aqueous phase of the oil-in-water emulsion mixture may be in the range of 0.01 to 5% w/v, preferably 0.05 to 4% w/v, for example 0.1 to 3% w/v. For example, a preferred range may be 1mg/ml (0.1% w/v) to 5 mg/ml (0.5% w/v) for collagen type I, and 10 mg/ml (1% w/v) to 50 mg/ml (5% w/v) for fibrinogen.

In a preferred embodiment, the aqueous protein solution may comprise a mixture of two or more proteins. When the aqueous protein solution comprises a mixture of two or more proteins, it is preferred that at least one of the proteins is collagen.

For example, a mixture of collagen and fibrinogen solutions may be used to create an interpenetrating collagen and fibrin scaffold. Preferably, the collagen is collagen type I. The collagen and fibrinogen reagent mixture may be present in a ratio by mass of 1:50 to 10:1, preferably 1:25 to 5:1, for example, 1:20 to 2:1. In a preferred example, a combination of 0.2% w/v collagen and 2% w/v fibrinogen are blended from separate solutions at a 1:1 volume ratio to give a ratio by mass of 1:10.

The final proportion of continuous phase in the oil-in-water emulsion may be 25 to 75 volume % of the total volume of the oil-in-water emulsion. Preferably the final proportion of continuous phase in the oil-in-water emulsion may be 25 % to 50 % volume % of the total volume of the oil-in-water emulsion. More preferably the final proportion of continuous phase in the oil-in-water emulsion may be 25% to 35% volume % of the total volume of the oil-in-water emulsion. The final proportion of continuous phase in the oil-in-water emulsion may be the volume % of the total volume of the oil-in-water emulsion used for casting. Preferably, the amount of protein in the oil-in-water emulsion may be 0.1% to 5% by mass of the total aqueous component of the oil-in-water emulsion.

The aqueous phase of the oil-in-water emulsion may be manufactured in stages, from component solutions. For example, the surfactant solution may be prepared from manufactured forms or concentrates by addition to water to form a surfactant solution. Each protein or proteins may be dissolved in, diluted into, or otherwise prepared in, separate aqueous buffer e.g. as a concentrated protein solution. Separate buffer solution may be used as a diluent. Separate stabilising agent solution may be used. Thereafter these component solutions may then be added to the scaffold formation mixture in appropriate proportions to achieve the final aqueous phase composition.

*Oil (hydrophobic) phase*

Any suitable oil may be used in the method of the present invention. Examples of suitable oils include light mineral oils, decane or short chain hydrocarbon oils (with hydrocarbon chain lengths in the range C8-C18, preferably C8 - C12). Examples of light mineral oils include pharmaceutical grade light mineral oil, with low viscosity, and nontoxic, triglycerides preferably with hydrocarbon chain lengths of fatty acyl groups in the range C8-C18, preferably C8 - C12 (e.g. glyceryl trioctanoate), cyclohexane, toluene, short chain hydrocarbons, and perfluorocarbon oil, or mixtures thereof, are also compatible. In a particularly preferred embodiment, the oil is decane.

Perfluorocarbons optionally in combination with phospholipids may also be used as the oil/hydrophobic phase.

The oil phase may be present in the oil-in-water emulsion in an amount of at least 25% of total volume of emulsion, preferably at least 50%, for example at least 75%. Preferably, the oil phase may be present in the oil-in-water emulsion in an amount of at least 100 volume % of the volume of the continuous phase, more preferably in an amount from 100 vol % to 300 vol % of the volume of the continuous phase. In a preferred embodiment, the oil phase may be present in the oil-in-water emulsion at about 300 vol % of the continuous phase. In constituting the final mixture, the oil phase may be present in at least 66 % of the volume of the continuous phase in the case where the continuous phase is mixed with the oil phase in two or more steps, such as addition of concentrated surfactant and aqueous buffer solution prior to addition of protein solution.

*Surfactant*

A non-ionic surfactant is used to form the oil-in-water emulsion. Preferably, the non-ionic surfactant may be used to reduce the risk of the protein in the continuous phase adsorbing onto the oil droplet interface, and/or the surfactant denaturing the protein components. The non-ionic surfactant may increase the likelihood of the protein remaining in bulk aqueous phase. The non-ionic surfactant may also reduce the risk of the protein being denatured during the scaffold-manufacturing process.

The surfactant can be any suitable non-ionic surfactant. A non-ionic surfactant consists of a hydrophilic head, a hydrophobic tail, and has no charge. Examples of non-ionic surfactants include esters or ethers of a polyol. Suitable polyols include sugar alcohols and their derivatives. In one example, the polyol is sorbitan.

The non-ionic surfactant may comprise an ester or ether of sorbitan and a fatty acid. The non-ionic surfactant may comprise an ester of sorbitan and a fatty acid. Suitable fatty acids include Csto $C_{20}$ fatty acids, for example, $C_{10}$ to $C_{18}$ or $C_{12}$ to $C_{16}$ fatty acids. The non-ionic surfactant may comprise an ester or ether of sorbitan and a fatty acid selected

from at least one of a $C_{10}$ to $C_{18}$ fatty acid, preferably a $C_{12}$ (lauric acid), $C_{14}$ (myristic acid) $C_{16}$ (palmitic acid) or $C_{18}$ (stearic acid). The non-ionic surfactant may comprise a mono-, di- or tri ester or ether of a fatty acid selected from at least one of a $C_{10}$ to $C_{18}$ fatty acid, preferably a $C_{12}$ (lauric acid), $C_{14}$ (myristic acid) $C_{16}$ (palmitic acid) or $C_{18}$ (stearic acid).

In one example, the non-ionic surfactant may comprise a mono-, di- or tri ester of a fatty acid selected from at least one of a $C_{10}$ to $C_{18}$ fatty acid, preferably a $C_{12}$ (lauric acid), $C_{14}$ (myristic acid) $C_{16}$ (palmitic acid) or $C_{18}$ (stearic acid). Preferably, the non-ionic surfactant comprises a monoester of sorbitan and lauric acid. Suitable esters of sorbitan and fatty acids are sold under the trademark Span™. For example, the non-ionic surfactant may comprise sorbitan monolaurate, Span 20™.

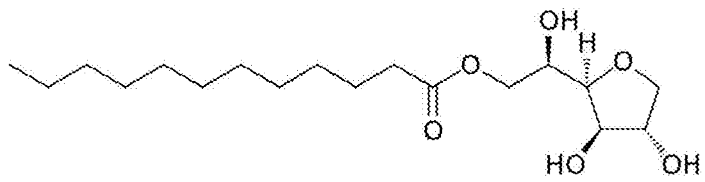

Span 20™ structure

The non-ionic surfactant may comprise an ethoxylated ester of a polyol. The non-ionic surfactant may comprise an ethoxylated ester of sorbitan or a polysorbate. Suitable polysorbates are sold under the trademark Tween ™.

The non-ionic surfactant may comprise an ethoxylated fatty acid ester of sorbitan. The fatty acid may be selected from at least one of a $C_8$ to $C_{20}$ fatty acid, for example, a $C_{10}$ to $C_{18}$ or $C_{12}$ to $C_{16}$ fatty acid. In one example, the non-ionic surfactant may comprise ethoxylated sorbitan monolaurate, Tween 20™.

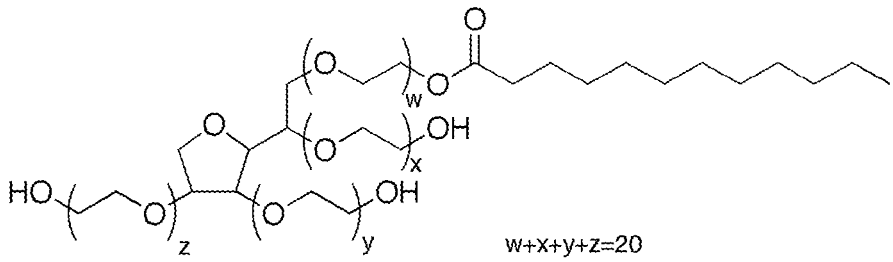

Tween 20™ structure

A mixture of non-ionic surfactants may be employed. The mixture may comprise sorbitan ester(s) and ethoxylated sorbitan ester(s). In one example, a mixture of Span™ and Tween™ surfactants may be employed. Preferably, a mixture of sorbitan monolaurate, Span 20™, and ethoxylated sorbitan monolaurate, Tween 20™ may be employed.

The ratio of sorbitan ester(s) to ethoxylated sorbitan ester(s) may be controlled to provide a suitable hydrophile-lipophile balance (HLB). The HLB is a measure of the degree to which the surfactant is hydrophilic or lipophilic. For example, the HLB value of Span 20 is 8.6 and of Tween 20 is 16.7.

The non-ionic surfactant or surfactant mixture comprising the non-ionic surfactant(s) has an HLB value of at least 10. The non-ionic surfactant or surfactant mixture comprising the non-ionic surfactant(s) has an HLB value of up to 16. The HLB is 10 to 16. In preferred examples, the HLB may be between 11 and 14, preferably 12 to 13.5, more preferably 12.5 to 13.

Other suitable non-ionic surfactants include ethoxylated non-ionic surfactants with a hydrocarbon tailgroup of 6 to 20 carbon atoms, preferably 8 to 15 carbon atoms, for example 10 to 12 carbon atoms. Preferably the surfactants have at least 12 moles, particularly preferred at least 16 moles, and still more preferred at least 20 moles, such as at least 25 moles of ethylene oxide per mole of alcohol.

Suitable ethoxylated surfactants include the sorbitan esters (Span™ family), polyethoxylated sorbitan esters (Tween™ family), polyoxypropylene-polyoxyethylene block co-polymer (Poloxamer family), polyethylene oxide aromatic hydrocarbon group, fatty acid/alcohol ethoxylates and fatty acid esters of glycerol.

The non-ionic surfactant may be used in combination with a co-surfactant. The cosurfactant can be an ionic surfactant, such as an anionic surfactant or a cationic surfactant. The co-surfactant may comprise up to 10% by weight of total surfactant, preferably up to 7.5% by weight, for example up to 5% by weight.

Examples of ionic surfactants which may be used as co-surfactants are sodium dodecyl sulphate (SDS) or

dodecyl trimethylamine chloride (DTMA) or decanoic acid.

The co-surfactant may have a hydrocarbon chain length similar to, or identical to, that of the non-ionic surfactant. In one example, the co-surfactant may have a hydrocarbon chain length in the range $C_{10}$-$C_{14}$, when the principle non-ionic surfactant has a hydrocarbon chain length of $C_{12}$.

By blending non-ionic surfactant(s) with anionic or cationic surfactants, it may be possible to alter the zeta potential ($\psi$) of the emulsion droplets. This can provide an increase in emulsion stability, as the magnitude of the zeta potential indicates the degree of electrostatic repulsion between adjacent, similarly charged particles within a dispersion.

A cationic surfactant may be used as a cosurfactant, for example, where the protein is fibrinogen. In another example, an anionic surfactant may be used as a cosurfactant, for example, where the protein is collagen.

The oil-in-water emulsion comprises a non-ionic surfactant in an amount of 0.01 to 10 volume % of the total volume of the oil phase in the oil-in-water emulsion. Preferably, the oil-in-water emulsion comprises a non-ionic surfactant in an amount of 0.05 % to 1 % volume %, more preferably 0.1% to 0.5 % volume % of the total volume of the oil in the oil-in-water emulsion. Preferably, the oil-in-water emulsion comprises a non-ionic surfactant in an amount of 0.1% to 0.7% volume % of the total volume of the oil in the oil-in-water emulsion. For example, a molar concentration of 1% Tween 20 ™ equates to 8.2 μM in the oil phase. A molar concentration of 1% Span 20 ™ equates to 29 μM in the oil phase.

Where a blend of surfactants is employed, the total amount of surfactant may be in the range of 0.05 -2 % mass of the oil phase. For example, the molar concentration of a preferred surfactant blend of the invention with an HLB of 12.5, where the total surfactant is 0.1%, equates to 0.393 μM Tween 20™ plus 1.50 μM Span 20™, in the oil phase.

Where a blend of surfactants is employed, the HLB of the surfactant blend may be between 11 and 14, preferably 12 to 13.5, more preferably 12.5 to 13.

The surfactant may be employed in combination with a stabilising agent. A suitable stabilising agent may be polyvinylpyrrolidone. The stabilising agent may be employed in an amount of 0.1 to 10 % w/v of the aqueous phase, for example, 0.5 to 5 % w/v of the oil-in-water emulsion.

*Method of preparing* a *porous protein scaffold*

The method of the present invention employs an oil phase emulsified in a pH-buffered aqueous phase, containing the protein component or components which will form the scaffold structure. Emulsions of this type are generically referred to as oil-in-water type (O/W) emulsions. To be useful for templating, emulsions of the invention are desirably stable for at least the time for protein gelation, and preferably for subsequent cross-linking to occur, and should desirably have a controllable droplet size. In at least some embodiments, the stability of emulsions of the emulsions of the invention may be achieved by the formulation described above, employing non-ionic surfactants, preferably with polyol headgroups, and more preferably by the HLB of the surfactant or surfactant blend employed.

The droplet size may be controlled by the shear rate of the emulsification system.

In accordance with the method of the present invention, protein gelation is used to form a protein network around, and separate from, the oil droplets. In one example, the step of protein gelation may include non-ionic, ionic or covalent crosslinking processes, or a mixture thereof. In another example, the step of protein gelation may include enzymatic or non-enzymatic chemical reaction, ionic or non-ionic crosslinking processes, including thermally controlled molecular self-assembly, or a mixture thereof.

Protein gelation may be performed over a period of time under controlled conditions of temperature and humidity. Typically, the onset of physical gelation will be designed to occur within 5-10 minutes of casting, and completion of the gelation process may occur after up to 60 minutes after casting, for example 30 minutes after casting. Protein gelation may be performed, at for example 37 °C, in a humidified chamber, or tray covered to minimise evaporative loss from the scaffold surface.

In one example, an additional chemical crosslinking step may be performed. Preferably, the crosslinking step occurs after primary structure formation, i.e. after protein gelation. The additional crosslinking step may join (e.g. covalently) protein molecules together to create an insoluble matrix. It understood in the art that cross-linking between proteins may allow control of the bulk proteolytic degradation rate of a protein scaffold. Cross linking can increase the physical strength and chemical and biochemical stability of the protein scaffold. It is believed that this may allow control of the degradation rate and profile of the scaffold. Crosslinking may also increase the mechanical properties of the scaffold.

Any suitable protein cross-linking agent for the protein component of the scaffold may be used. For example, di-aldehydes (e.g. glyoxal, glutaraldehyde), di-isocyanates (e.g. hexamethylene di-isocynanate), succinimides (e.g. N-hydroxysuccinimide (NHS)), carbodiimides (e.g. 1-ethyl-3-(-3-dimethylaminopropyl) carbodiimide hydrochloride (EDC), N,N dicylohexylcarbodiimide), imidoesters (e.g. dimethyl adipimidate (DMA), dimethyl suberimidate (DMS), epichlorhydrin, 1,4-butanediol diglycidyl ether, genipin or enzymes with protein cross-linking function (transglutaminase, factor XIII).

Compatible cross-linking reagents include 1-ethyl-3-(-3-dimethylaminopropyl) carbodiimide hydrochloride (EDC) and sulfo-N-hydroxysuccinimide (NHS). In one embodiment, EDC and NHS may be used in combination. Any suitable molar ratio of EDC and NHS may be employed. For example, the molar ratio of EDC to NHS may be 1-10 : 1,

for example, 1 - 5 : 1, or 2 - 3 : 1. In one example, the molar ratio of EDC to NHS may be 5 : 2. In one example, EDC:NHS (5:2 molar ratio) in the range 1-100 mM for EDC, preferably in the range 10-35 mM. may be used.

Other suitable cross-linking reagents include glutaraldehyde. For example, glutaraldehyde in the range 10 to 500 mM, more particularly in the range 15 to 50 mM may be used.

An alcoholic solution may be used in the additional crosslinking step. Examples of suitable alcohols include $C_1$-$C_6$ alcohols, preferably $C_1$-$C_4$ alcohols, for example $C_1$-$C_3$ alcohols. In a preferred embodiment, the alcohol is ethanol. The alcohol may be used in combination with an aqueous pH buffer, in the range 50% to 95%. In one embodiment, a crosslinking solution of 75-85% ethanol with an aqueous pH buffer in the region of pH 6-8, preferably in the range of pH 7-7.5, for example pH 7.4, can be used.

Enzymatic cross-linking reagents may also be used. An example is transglutaminase. Enzymatic cross-linking using transglutaminase dissolved in an aqueous buffer at a concentration between 0.001 and 1000 Unit/ml can also be used.

In accordance with the method of the present invention, emulsion oil droplets are removed from the scaffold after formation. The oil droplets may be removed by elution from the scaffold. Any suitable solvent may be used for this step. For example, an alcohol solvent may be used. The alcohol may be an aliphatic alcohol. In one embodiment, the alcohol is a $C_1$-$C_{12}$ alcohol, preferably a $C_1$-$C_6$ alcohol, for example a $C_1$-$C_4$ alcohol. Examples of suitable alcohols include methanol, ethanol, propane-1-ol, propane-2-ol, butane-1-ol, butane-2ol, tert-butanol and mixtures thereof. The elution is performed by washing the scaffold with an excess volume of the alcohol or alcohol solution. The washing step may be aided by gentle agitation, such as achieved by rotary orbital motion.

Following tne removal of oil droplets, tne scaffold is incubated with an excipient solution. The excipient may be a water-soluble organic hydrophilic excipient. Examples of suitable excipients include polyols such as sugars (e.g. mannitol, sorbitol, dextrose, sucrose), polymers such as polyvinyl alcohol (PVA), polyethylene glycol (PEG) or polyoxyethylene-polyoxypropylene glycol block co-polymer (e.g. pluronic P68), polyvinylpyrrolidone (PVP) (e.g. Kollidon™), or polyethylene glycol-polyvinyl alcohol copolymers (e.g. Kollicoat™). A mixture of excipients may be used. The concentration of the excipient in solution, and then may be up to 10 volume %, for example from 1 to 5 volume %.

The scaffold may be incubated for any suitable amount of time, for example for 5 to 10 minutes. The excipient may reduce bulk shrinkage of the scaffold during drying and may also preserve the nano-structure of the scaffold during a freeze-drying process. The concentration of excipient used is in the range 0.5 - 2 M aqueous for sugars, or in the range of 0.5-5% w/v aqueous for polymeric excipients.

Following incubation, excess excipient solution is removed from the scaffold, for example by draining, while maintaining saturation of the scaffold with the excipient solution. The excipient itself coats the scaffold structure, and can be removed after freeze-drying, for example can be removed by washing. Removal of the excipient is desirable before use of the scaffold in, for example, treatment of wounds.

In accordance with a preferred method of the present invention, freeze-drying of the scaffold is performed. Freeze-drying (or lyophilisation) is a dehydration process that works by freezing the scaffold which is saturated in water or excipient solution, and then reducing the surrounding pressure. This allows the removal of solvent (water) and remaining volatile oil from the scaffold. Thus, freeze-drying advantageously improves the storage and shelf life of the scaffold. Controlling the freeze-drying parameters allows for preservation of the scaffold nanostructure that has been formed by emulsion-templating. Preferably, the freeze-drying is performed at between -20 to -40 °C, and at a pressure below the corresponding vapour pressure at the selected drying temperature. In one example, freeze-drying is performed at -40 °C. In an example, freeze-drying is performed at < 200 mTorr.

In some examples, the scaffold is prepared in a clean room under sterile conditions.

*Scaffold*

The porous scaffold formed by the emulsion templating method disclosed comprises a 3-dimensional protein-based structure, in which the protein of the scaffold is a protein which provides cell adhesion sites, and which is designed to have a pore dimension to allow cell ingress into the 3-dimensional structure. The scaffold structure thus provides a 3-dimensional structure able to accommodate cells therein.

The porous scaffold may comprise a structure with a substantial proportion of pores of sufficient dimension to allow cells to pass through and thus to penetrate the structure. The porous structure of the scaffold is preferably a structure of interconnecting pores, extending through at least part of the structure in one dimension. The pore size is typically measured by the pore diameter or shortest distance between scaffold lamellae forming the pore wall.

The range of pore dimensions (shortest pore diameter) suitable is greater than 20 $\mu$m and less than 350 $\mu$m. The scaffold has an average pore size in the range of 80 to 200 microns, preferably in the range of between 90 to 120 microns, for example between 100 to 110 microns.

The pores may be regularly or irregularly shaped. For example, the pores may define chambers or tunnels extending through at least a portion of the scaffold. The scaffold may take the form of a matrix. In one embodiment, the

scaffold takes the form of a matrix comprising a plurality of pores.

Scaffolds of the present disclosure have an average pore size in the range 80 to 200 microns, preferably in the range 90-120 microns or 80 to 100 microns. Figure 1 shows scanning electron microscopy images of a number of emulsion-templated EmDerm scaffolds (emulsion-templated dermal scaffolds). In the histograms shown in D. to F. the % surfactant on each x-axis from left to right is 0.1, 0.3, 0.5 and 0.7 respectively. The pore size may be measured using a cross-section of the SEM image, as shown in Figure 1.

The scaffold comprises an interconnected fibrous structure of the proteins.

In general, the protein(s) is/are not denatured. The protein(s) may be presented in its/their nature secondary and/or tertiary configuration.

The scaffold may be a collagen, fibrinogen, fibronectin, laminin, or elastin scaffold. Alternatively, a composite scaffold can be formed using two or more proteins. The scaffold or composite scaffold may also include a colligative agent such as gelatin or fibroin.

Examples of composite scaffolds include collagen-fibrinogen scaffolds, collagen-chondroitin sulphate scaffolds, collagen-hyaluronic acid scaffolds. Examples of fibrin composite scaffolds include fibrin-chondroitin sulphate, fibrin-hyaluronic acid, and fibrin-gelatin.

It is preferred that the scaffold comprises collagen.

The scaffold may have a suitable tensile strength to support cell adhesion and to be physically handled and manipulated. For example, the scaffold may have an ultimate tensile strength (UTS) of between 0.01 to 100 MPa. Preferably the scaffold UTS is between 1-20 MPa. For example, the UTS of one scaffold of the invention is in the range 10-16 MPa.

The scaffold may have a Young's modulus of between 0.01 to 100 MPa, preferably 0.5 to 3 MPa, for example 1 to 2 MPa.

The stability of the scaffold of the invention is sufficient to support cellular ingress and proliferation within the structure for sufficient time as to fulfil the intended purpose of the scaffold.

The stability of the scaffold in a proteolytic environment can be measured by the rate of weight loss over incubation time in a test proteolytic solution. For example, a solution of tissue culture grade trypsin, 0.25% w/v in phosphate buffered saline or versene buffer is suitable. In an assay in which a sample of scaffold is incubated in such a trypsin solution at 37°C, and the trypsin is replaced every 24hr. The preferred stability, as measured by 50 % weight loss, may be 4-10 days, for example, 5-7 days.

*Uses of the scaffolds*

Scaffolds of the invention may have a variety of applications. These include for example, use as acellular implants, also referred to as tissue repair scaffolds, to promote histologically organised tissue reconstruction and wound healing. Another exemplary use is for a cell-assisted tissue repair scaffold, in which a scaffold of the invention is seeded with therapeutic cells prior to surgical implantation for tissue reconstruction and wound healing. Another example of use of the scaffolds of the invention is the creation of in vitro tissue-engineered tissues, in which cells are seeded into or onto the scaffold, in aseptic conditions, in a physiological cell culture medium, and supported in an environment which allows cells to organise on or within the scaffold, to form a tissue structure or organoid or organ-like structure. These are commonly referred to as tissue-engineered constructs, tissue equivalents or skin equivalents. Such tissue engineered constructs may be used for implantation as advanced therapy medicinal products (ATMPs), or used for non-clinical investigational purposes, such as drug screening or therapy evaluation.

The porous protein scaffold may be for use in repairing tissue or tissue regeneration in a human or an animal. For example, the porous protein scaffold may be used in wound healing. The wound may be associated with tissue loss, such as, for example, a burn, a blast wound, a de-gloving injury. The wound may be a surgical resection wound, such as from a removal of a skin cancer. The wound may be a chronic wound, such as an ulcer or a pressure sore.

The porous protein scaffold may be used in a method of repairing tissue, or for the tissue regeneration, in a human or an animal. The method comprises applying or implanting the porous protein scaffold at a site, such as a wound, in the human or animal body. Typically, the site is an area of the human or animal body in need of tissue regeneration or repair.

The introduction of the scaffold can aid regrowth of tissue at the site in which it is applied or implanted.

After applying or implanting the porous protein scaffold, a dressing material may be applied over the site with the porous protein scaffold.

The porous protein scaffold may be secured at the site with sutures or staples.

Before applying or implanting the porous protein scaffold, the method may involve soaking or washing the porous protein scaffold in a saline solution (i.e. a sterile saline solution) prior to application or implantation.

The porous scaffold may be used in combination with other skin substitutes. For example, the porous scaffold may be used in combination with a membrane, for example an electrospun membrane or silicone sheet. The membrane

can be used to provide a semipermeable or microporous barrier on top of the scaffold. The membrane may be porous, which may allow nutrients to diffuse through the membrane portion into the porous scaffold so as to facilitate cell propagation and growth. In one example, the interfibre pores of an electrospun membrane have a mean diameter of less than 10 μm.

The porous protein scaffold can be used as a tissue repair scaffold or composite material. The porous scaffold is designed to accommodate cells. For example, the porous protein scaffold may comprise openings or pores for accommodating cells. Accordingly, cells may be seeded onto the scaffold, and the scaffold may facilitate growth of new tissue.

The scaffold can be used to create scaffolds for tissue engineering purposes. For example, the scaffold may be used for engineering solid organs e.g. artificial liver, heart or kidney. The scaffold may also be used for tissue reconstruction of dermis, fascia, tendon, ligament, pericardium, periosteum and soft tissue such as fat and muscle grafts.

The invention provides an *in vitro* or ex *vivo* method of (i) manufacturing or engineering a tissue or (ii) manufacturing a tissue-engineered construct. Each method comprises applying cells to the porous protein scaffold, preferably in a culture container. This step produces a porous protein scaffold seeded with cells.

The cells may be derived from skin, soft tissue or bone.

The cells may be fibroblasts, keratinocytes, melanocytes, Langerhans cells, Merkel cells, or stem cells.

The cells may be human cells or porcine cells, preferably human cells.

The method may comprise applying cells and a culture medium to the porous protein scaffold.

After applying the cells, the method involves growing cells and/or tissue on the seeded porous protein scaffold. This step may be performed using techniques known in the art.

The scaffold made in accordance with the method of the present invention can also be used as a stem cell carrier delivery system for wound healing and regeneration. For example, the scaffold may be used for the treatment of burns, such as partial to full thickness burns. The scaffold can also be used for treatment of wounds, such as non-healing, chronic wounds.

*Tissue-engineered construct*

The invention further provides a tissue-engineered construct. The tissue-engineered construct comprises cells or a tissue supported on the porous protein scaffold.

The cells or tissue may be stratified on the porous protein scaffold.

The tissue-engineered construct may comprise a first layer and a second layer.

The first layer may comprise fibroblasts and keratinocytes supported on a first porous protein scaffold. This first layer may provide part of a skin scaffold having a stratified epithelium.

The second layer may comprise fibroblasts and stem cells, preferably mesenchymal stem cells, supported on a second porous protein scaffold. This second layer may provide part of a skin scaffold having a vascularized dermal construct.

The first porous protein scaffold may have the same or a different composition as the second porous protein scaffold. Both the first and second porous protein scaffolds are in accordance with the invention.

The tissue-engineered construct may be used in the treatment of the human or animal body. For example, the tissue-engineered construct may be used in wound healing. The wound may be associated with tissue loss, such as, for example, a burn, a blast wound, a de-gloving injury. The wound may be a surgical resection wound, such as from a removal of a skin cancer. The wound may be a chronic wound, such as an ulcer or a pressure sore.

Disclosed is a method of treating a human or an animal. The method comprises applying or implanting the tissue-engineered construct at a site, such as a wound, in the human or animal body. The method comprises applying or implanting the tissue-engineered construct at a site, such as a wound, in the human or animal body. Typically, the site is an area of the human or animal body in need of tissue regeneration or repair.

After applying or implanting the tissue-engineered construct, a dressing material may be applied over the site with the tissue-engineered construct.

The tissue-engineered construct scaffold may be secured at the site with sutures or staples.

## EXAMPLES

### Example 1

*Materials*

Type I rat tail collagen 5mg/ml in acetic acid (First Link, UK), bovine fibrin (Sigma Aldrich), bovine thrombin (Sigma Aldrich) were purchased. Decane, Tween 20 and Span 20 were also obtained from Sigma Aldrich to make up

oil-in-water emulsions. 1-ethyl-3-(-3-dimethylaminopropyl) carbodiimide hydrochloride (EDC) and sulfo-N-hydroxysuccinimide (NHS), pure ethanol, pure isopropanol were also obtained from Sigma-Aldrich for cross-linking and oil elution. 2-(N-morpholino) ethanesulfonic acid (MES) and sodium chloride were bought from Sigma Aldrich, as well as the Cell Counting Kit (CCK-8) assay. Polyvinyl alcohol (PVA) (99% hydrolysed, MW 89,000 to 98,000), polyethylene glycol (PEG) (MW 6000), Pluronic F-68 (P68) and mannitol were all obtained from Sigma Aldrich. 0.25% Trypsin-EDTA solution for cell culture was also purchased from Sigma-Aldrich.

*Manufacture of Scaffolds*

Emulsion mixtures comprising of decane, Span 20/Tween 20 at a calculated HLB of 13 and an aqueous buffer (25 mM MES, 150 mM NaCl pH 7.4) were mixed in a 60 ml syringe with the tip removed, for 15 seconds at 1000 rpm using a Caframo high-speed mixer. Emulsions with 0.1%, 0.3%, 0.5% and 0.7% surfactant concentration were prepared. To manufacture scaffolds from various protein types, each of the emulsions were added to protein solutions designated as follows: collagen (Col), fibrinogen plus thrombin (Fbn) or a mixture of collagen and fibrin solution at a 1:1 ratio (ColFbn). The gelling solution of collagen was used at 4.5 mg/ml, fibrinogen was prepared at 20 mg/ml and thrombin at 10 units/ml in in 25 mM MES/150 mM NaCl (pH 7.4) buffer. Each emulsion-scaffold was left to gel at 37°C for 30 minutes and then cross-linked with EDC:NHS (5:2 molar ratio) in 80% ethanol. The scaffolds were then washed in 80% isopropanol then deionized water three times for 15 minutes. At this point scaffolds were further incubated with an 1% w/v excipient solution (e.g. P68, PVA, PEG or mannitol (M)). All scaffolds were freeze-dried at -40°C (for fibrin scaffolds) and -30 °C (for collagen scaffolds)

The possible effects of the different excipients used, on functional parameters and scaffold morphology, were compared using scaffolds made at large pore size with 0.1% surfactant concentration. The effect of surfactant concentration was compared between scaffolds made using Pluronic F68 as the excipient.

*Pore Size of Scaffolds*

Each of the scaffolds was cut using a scalpel so that the cross-section of each scaffold was lying horizontally on the carbon tape mounted on the aluminium stubs for scanning electron microscopy imaging. Three images were taken per scaffold and the diameters of 20 random pores were averaged out to calculate estimated mean pore size of each scaffold. Scaffold morphology (e.g. fibrous or smooth) was also noted.

*Biocompatibility of Scaffolds*

Scaffolds were seeded with three cell types to determine the biocompatibility of each scaffold with the various cell types - human dermal fibroblasts (HDF), human dermal endothelial cells (HDE) and GFP-labelled human mesenchymal stem cells (MSC). Each scaffold was cut into 6mm discs using a punch biopsy and washed with PBS (phosphate buffered saline) three times before incubating overnight in media of DMEM (Dulbecco's Modified Eagle's medium) media with 10% FBS (fetal bovine serum) and penicillin/streptomycin antibiotics. On the following day, each of the scaffolds was seeded with 5000 cells/well and left overnight for cells to attach onto the scaffold material. On the next day, the seeded scaffolds were transferred into a new well plate and allowed to incubate up to 14 days. Media was changed on alternate days. On Day 3, 7 and 14, a cell proliferation assay using CCK-8 (Sigma, UK) was done to determine cell growth in each scaffold. On Day 14, the scaffolds were fixed in normal buffered formalin for further imaging.

*Wide-field Imaging of Seeded Scaffolds*

Each of the seeded scaffolds was stained using fluorescent antibodies for downstream imaging. Scaffolds seeded with human dermal fibroblasts were stained with 1:6 phalloidin (Alexa Fluor 488, Thermo Fisher) and scaffolds seeded with human dermal endothelial cells were stained with 1:250 mouse anti-human CD31 (Dako) and 1: 1000 rabbit anti-human vWF (Dako). Secondary staining was done using goat anti-mouse (Alexa Fluor 488, Thermo Fisher) and goat anti-rabbit (Alexa Fluor 568, Thermo Fischer). Scaffolds seeded with mesenchymal stem cells were not stained as they were GFP expressing cells. Z-stacks were taken to visualize cell migration through the scaffold by wide-field imaging at 20X magnification. Images were deconvoluted using AutoQuant X3 (Media Cybernetics) and visualised using Bitplane (Imaris software, Version 9.1).

*Statistical Analysis*

Data between different scaffolds and time-points were analysed using two-way analysis of variance (ANOVA) to evaluate statistical significance between comparisons. A p-value of less than 0.05 is considered to be statistically

significant. Where applicable, * denotes a p-value of <0.05, ** denotes a p-value of <0.01 and *** denotes a p-value of <0.001. All statistical analysis was performed using GraphPad Prism Version 4.0 for Windows.

***Results***

*Scaffold Structure and Porosity*

The application of the decane oil-in-water emulsions was successful in forming scaffolds from each type of scaffold protein, neutralised type I collagen acetic acid extract, which gels spontaneously on warming from a 4°C solution to 37°C; and fibrinogen, enzymatically coagulated with thrombin 37°C. Pore interconnectivity of scaffolds was obtained by controlling the oil:aqueous phase ratio to ≥0.5. Importantly, the formed scaffolds demonstrate a nanoscale fibrous architecture derived from the hydrogelatinous state. The use of an excipient was needed to preserve this structure on lyophilisation and reduce shrinkage.

Pore size of the EmDerm scaffolds inversely correlated with the surfactant concentration used to create the emulsion. There was an inverse relationship between surfactant concentration, the mixing speed (applied shear rate), and emulsion droplet diameter, i.e. increasing surfactant concentration and / or shear rate may decrease droplet diameter. For scaffolds made from emulsions with 0.1 % surfactant, a mean pore size of around 100 μm was obtained. This decreased to approximately 40 μm with 0.7% surfactant.

*Mechanical Properties of Scaffolds*

The measurement of the tensile properties of each EmDerm scaffold type (Table 1) showed that the collagen-fibrin scaffolds had the highest Young's modulus and Ultimate Tensile Strength (UTS), approximately 1 to 2MPa and 12 to 16 MPa respectively. This is followed by collagen scaffolds, which had a Young's modulus of about 1 to 2 MPa and UTS of about 7 to 9 MPa. Fibrin scaffolds had a Young's modulus of about 1 to 2 MPa and UTS of about 4 to 5 MPa. Change in porosity of the scaffolds and excipients used did not appear to have a significant impact on the mechanical properties of the scaffolds, as compared to the type of material the scaffolds are made of.

Table 1. Mechanical strength characterisation of scaffolds.

| Scaffold | Young's modulus (MPa) | Ultimate Tensile Strength (MPa) |
|---|---|---|
| **CF scaffolds** | | |
| **0.1CF** | 2.03 ± 0.81 | 12.82 ± 3.45 |
| **0.3CF** | 2.43 ± 0.45 | 14.07 ± 2.67 |
| **0.5CF** | 2.55 ± 0.98 | 15.15 ± 3.92 |
| **0.7CF** | 2.89 ± 0.72 | 16.47 ± 5.39 |
| **CF-M** | 1.89 ± 0.58 | 12.55 ± 3.89 |
| **CF-P68** | 1.46 + 0.33 | 13.25 ± 2.66 |
| **CF-PEG** | 1.74 ± 0.76 | 11.53 ± 1.96 |
| **Collagen scaffolds** | | |
| **0.1C** | 1.95 + 0.49 | 1.62 |
| **0.3C** | 1.45 ± 0.76 | 7.87 ± 1.45 |
| **0.5C** | 1.71 ± 0.21 | 7.99 ± 1.02 |
| **0.7C** | 2.14 ·+ 0.34 | 8.85 ± 0.97 |
| **C-M** | 1.27 ± 0.48 | 9.65 ± 2.81 |
| | 1.25 + 0.74 | 8.26 ± 1.78 |
| **C-PEG** | 1.70 ± 0.51 | 8.87 + 1.24 |
| **Fibrin scaffolds** | | |
| | | |
| **0.3F** | 1.16 ± 0.29 | 4.81 ± 0.72 |

(continued)

| Fibrin scaffolds | | |
|---|---|---|
| **0.5F** | 1.18 ± 0.41 | 4.77 ± 0.42 |
| **0.7F** | 1.12 ± 0.29 | 4.62 ± 0.58 |
| **F-M** | 1.26 ± 0.35 | 4.25 ± 0.63 |
| **F-P68** | 1.48 ± 0.32 | 4.59 ± 0.22 |
| **F-PEG** | 1.37 ± 0.2 | 5.13 ± 0.51 |
| **F-PVA** | 2.01 ± 0.11 | 4.43 ± 0.49 |

*Hydrolytic Degradation of Scaffold*

Scaffold degradation by hydrolysis was determined by measuring residual dry weight of scaffolds soaked in PBS (phosphate buffered saline) for extended periods (Figure 2 A-C). Collagen scaffolds showed a large drop in dry mass of 40-50% over the first week. This is largely due to the dissolution of excipients during the initial hydration of the scaffold. At the end of 5 weeks, all scaffolds retained about 40 - 60% of their dry mass. Although collagen scaffold stability was achieved at the shortest cross-linking time (Fig 2A), collagen-fibrin and fibrin scaffolds which were cross-linked for a longer duration degraded slower compared to scaffolds which were cross-linked for 30 minutes (Fig 2B,C) although this was not statistically significant in this experiment. Notably, fibrin scaffolds which were cross-linked for only 30 minutes degraded completely by 5 weeks.

*Enzymatic Degradation of Scaffold*

Collagen and collagen-fibrin scaffolds remained incompletely degraded by Day 7 when incubated in trypsin solution (fig 2A, B). All fibrin scaffolds which were cross-linked for less than 2 hours were completely degraded within 7 days. Those were cross-linked for 0.5 hours were completely degraded by 3 days followed by those cross-linked for 1 hour on Day 5 and those cross-linked for 1.5 hours on Day 7 (Fig 2C). As with the hydrolytic degradation, the mass of each scaffold decreased significantly over the first day. Dissolution of excipients is likely to contribute to the initial drop of scaffold dry mass.

*Biocompatibility of Scaffolds*

The assessment of the biocompatibility of EmDerm scaffolds was investigated by measuring the proliferation of three cell types central to skin reconstruction, HDF, HDE and MSC. Net proliferation of each cell type occurred over the 14 days of the assay in each scaffold material (Figures 3-5). Importantly, there was no significant effect of varying the excipient used, compared to the pluronic excipient (Fig 3). HDE and MSC proliferation in collagen scaffold made without an excipient (C-GEL) was lower than EmDerm collagen scaffolds with excipient (C-P68 and C-M) (Fig 3A). Also, HDF showed lower proliferation on CF-PEG (Fig 3B) and F-M (Fig 3C) scaffolds.

While proliferation was marked in scaffolds with high porosity, there was a general trend for proliferation to be progressively lower with decreased porosity (Fig 4). This effect was most pronounced for HDF in C scaffolds at the lowest porosity, corresponding to 0.5 & 0.7% surfactant mix in the emulsion template (Fig 4A), and in CF scaffolds at the lowest porosity (Fig 4B).

*Comparison with Commercial Scaffolds*

Significantly greater proliferation of each cell type occurred in each of the high porosity EmDerm scaffolds from 0.1% surfactant than the commercial comparator scaffolds, Matriderm and Integra (Fig 5). It is notable that 0.1F-P68 supported greatest HDE proliferation, while 0.1C-P68 promoted the better mesenchymal cell proliferation, both effects being statistically significant (Fig 5).

*Wide-field microscope imaging* of *seeded scaffolds*

Based on the Z-stacks obtained, each cell type was found to infiltrate into the scaffold uniformly over the XY plane and Z plane (Fig 6). Due to limitations in light penetration, cells were only imaged up to a depth of about 100 microns. In particular, cell infiltration into collagen scaffolds was observed to a similar extent as for fibrin-containing

scaffolds, suggestive of effect of the preserved fibre nanostructure in these scaffolds. Interestingly, the endothelial cells seemed to form ring-like structures when seeded onto EmDerm scaffolds, most notably with CF and F (arrows in Fig 6, HDE panels). It is also notable that HDE ingress into the EmDerm C scaffolds, and although show less cytoskeletal spreading, demonstrate some association into annular structures. By contrast, the stromal cell types adopt an elongated spindle-like morphology when seeded on to EmDerm scaffolds, more pronounced with fibroblasts than mesenchymal stem cells (Fig 6).

**Example** 2 - **Emulsification Efficiency** (not according to the claimed invention) **Gelation**

Emulsion mixtures were prepared for fibrin gellation systems.

*Fibrin*

Fibrinogen was dissolved to give a 2% solution of clottable protein in MES/NaCl buffer. The pH was adjusted to 7.4 after complete dissolution of the protein.

An emulsion mixture was prepared comprising 2 μl of calcium chloride, MES/NaCl, either up to 250 μl, decane 500 μl, and a test surfactant (in the range of 0.1 to 1 % of decane volume).

Each of the emulsion mixtures were mixed by vortex and hand shaking so as to establish an emulsion. 250 μl Fibrinogen was added, and the mixture was briefly mixed again. Then 25 μl of thrombin was added, and the mixture was shaken vigorously for 30 seconds and incubated for 30 minutes at 37 °C. Control tests of the aqueous components, calcium chloride, fibrinogen, MES/NaCl buffer and thrombin were run to verify coagulation.

Table 2: Gellation results for fibrin gellation systems

| Test mix | Composition | | Gellation result |
|---|---|---|---|
| | Emulsion | Aqueous | |
| Control | Nil | 1% bFbn/MES/NaCl | Gel |
| Span20/Tween20 | S/T / Decane 1 % | | Gel |
| SDS | SDS/Decane 1% | pH 7.4 | No gel |
| DTMA | DTMA: Decane 1% | + 2.5ul Thrombin (0.25 IU) | No gel |
| Test Mix | Composition | | Gellation result |
| | Emulsion | Aqueous | |
| Control | Nil | 1% | Gel |
| Span20/Tween20 | S/T / Decane 1 % | bFbn/MES/NaCl | Gel |
| SDS | SDS/Decane 1% | pH 7.4 | No gel |
| SDS/(S/T) | DSD/(S/T)/Decane: 0.95:0.1% | + 2.5ul Thrombin (0.25 IU) | No gel |
| DTMA | DTMA/Decane: 1% | | No gel |
| DTMA/(S/T) | DTMA: Decane: (S/T)/Decane: 0.9%:0.1% | | gel |
| | | | |
| Test Mix | Composition | | Gellation result |
| | Emulsion | Aqueous | |
| Control | Nil | 1% | Gel |
| Span20/Tween20 | S/T/ Decane: 1% | bFbn/MES/NaCl | Gel |
| SDS | SDS/Decane: 1% | pH 7.4 | No gel |
| SDS/(S/T) | SDS/(S/T)/Decane: 0.9%:0.1% | + 2.5ul Thrombin (0.25 IU) | No gel |
| DTMA | DTMA/Decane: 1% | | No gel |
| DTMA(S/T) | DTMA: Decane: (S/T)/Decane: 0.9%:0.1% | | gel |
| Decanoic acid (DA) | DA/(S/T)/Decane: 1%/1% | | Partial gel |

The results demonstrate that ionic surfactant cannot be substituted for the non-ionic Span 20/Tween 20 mixture at an optimised HLB for decane. The adverse effects of ionic surfactants may be due to protein denaturation or inhibition of gellation, and due to emulsion instability. However, the incorporation of up to 0.1 % ionic surfactant in combination with 0.9 % of non-ionic Span 20/Tween 20 in these gellation systems can be compatible with gellation and templating.

**Example 3** (not according to the claimed invention)

***Materials***

Mineral oil, Tween 20 and Span 20, 2-(N-morpholino) ethanesulfonic acid (MES), sodium chloride (NaCl), Oil Red O, bovine serum albumin (>95% purity), FITC-BSA and butanol were all purchased from Sigma. Polyvinylpyrrolidone (PVP) excipient (Kollidon KDF90) was obtained from BASF Pharmaceutical Co.

***Emulsion preparation***

*Effect of HLB ratio on emulsification*

Tween 20 and Span 20 were mixed to obtain a range of HLB ratios of 9.5 to 14, since the relative HLB (rHLB) of mineral oil to form an oil-in-water emulsion is described as approximately 10.5. 0.75% of surfactant was added to 5ml of mineral oil. 2% of PVP was added to 2.5ml of 25mM MES/150 mM NaCl buffer (pH 7.4). The solution was vortexed for 15 seconds.

*Effect of PVP concentration on emulsification*

0.5% of Tween 20/Span 20 surfactant mix with an HLB ratio of 13 was added to 5ml of mineral oil. 0%, 0.5%, 2% and 4% of PVP was added to 2.5ml of 25mM MES/150 mM NaCl buffer (pH 7.4). The solution was vortexed for 15 seconds.

*Effect of surfactant concentration on emulsification*

0.25%, 0.5%, 0.75%, 1% and 1.5% of Tween 20/Span 20 surfactant mix with HLB ratio of 13 was added to 5ml of mineral oil. 2% PVP was added to 2.5ml of 25mM MES/150 mM NaCl buffer (pH 7.4). The solution was vortexed for 15 seconds.

*Effect of temperature on emulsification*

0.75% surfactant concentration of Tween20/Span 20 surfactant mix with HLB ratio of 13 were added to 5ml of mineral oil and 2.5ml of 25mM MES/150 mM NaCl buffer (pH 7.4). The mixture was stored at 4, 22 and 37 degree Celsius for an hour and vortexed for 15 seconds.

*Effect of varying surfactant concentration at different shear rates*

0%, 0.25%, 0.5%, 0.75%, 1% and 1.5% of Tween20/Span 20 surfactant mix with HLB ratio of 13 were added to 5ml of mineral oil and 2.5ml of 25mM MES/150 mM NaCl buffer (pH 7.4). The mixture was mixed with a high-speed mixer (Caframo, Canada) at 500, 1000, 1500, 2000, 2500 and 3000 rpm

***Emulsion droplet size analysis***

100 μl of all emulsion mixes were stained with Oil Red O and placed on a glass slide with a cover slip. Droplet diameter was characterized using simple light microscopy and Image J. All measurements are expressed as mean ± standard deviation. Shapiro-Wilk test was done to test for data normality. Non-parametric data was evaluated using one-way ANOVA (Kruskal- Wallis) with Dunn's multiple comparison correction. A value of $p<0.05$ was considered to be statistically significant.

***Emulsion stability analysis***

Stability analysis was performed by measurement of turbidity (Turbiscan emulsion stability analyzer, Formulaction, France). Change in light transmission and backscatter throughout the sample was used to characterize the effect

of HLB ratio, surfactant concentration and PVP concentration on the stability of each emulsion mix. 2ml of each emulsion mix was pipetted into glass cells and 21 scans were done over a period of 10 minutes (1 scan every 30 seconds). Change in light transmission and backscatter throughout the length of the glass cell was plotted over time to obtain time point measurements of emulsion destabilization.

***Emulsion viscoelasticity analysis***

Diffusing wave spectroscopy (DWS Rheolab, LS Instruments, Switzerland) system was used to measure viscoelasticity of several emulsions to evaluate effect of PVP concentration on the emulsion system. Briefly, the DWS Rheolab system is an optical microrheology instrument that utilizes diffusing wave spectroscopy to monitor thermal motion of colloidal fluid systems and thus, allows for calculation of storage, loss and complex moduli. 200 μl of each emulsion sample were vortexed and immediately pipetted into the 2mm path length DWS customized optical cuvettes for measurement of G' (loss modulus), G" (storage modulus) as well as G* (complex modulus).

***Characterization of protein-emulsion interaction***

*Localization of proteins in emulsion mix*

10 μl of 1mg/ml FITC-BSA was added to each emulsion mix and vortexed for 15 seconds. The mixtures were incubated at 37 °C for 30 minutes before imaging under confocal microscopy. Distribution of FITC-BSA for each emulsion mix was recorded and confocal green fluorescence images were taken at 10x magnification.

*Quantification of protein in oil and aqueous phase*

10 μl of 1mg/ml FITC-BSA was added to each emulsion mix and vortexed for 15 seconds. The mixtures were incubated at 37 °C for 30 minutes before centrifuging at 10 000 g for 10 minutes to induce creaming. 200 μl of the aqueous subnatant was carefully removed and transferred into a 96 well plate in triplicates (50 μl per well). 100 μl of the creamed supernatant was removed and mixed with 100 μl of butanol to break up the emulsion before transferring onto a 96 well plate in triplicates (50 μl per well). Relative fluorescence was measured against a control solution containing 10 μg/ml of FITC-BSA in MES/NaCl buffer. Plates were read using the Spectramax i3x Multi-Mode Detection Platform at an excitation wavelength of 495nm and an emission wavelength of 530nm. A standard curve using a range of FITC-BSA concentration was used to quantify amount of proteins in the aqueous and cream phase. All statistical analysis was done using ANOVA on GraphPad Prism (GraphPad Software Inc.).

*Assessing conformation of adsorbed BSA*

Attenuated total reflection - Fourier transform infrared spectroscopy (ATR-FTIR) was used to assess denaturation of adsorbed BSA in the cream phase. Each emulsion mix was prepared as described above, however 3% BSA in MES/NaCl buffer was added to the aqueous phase prior to vortexing and centrifugation at 10 000 g. This concentration would ensure that even if 0.1% of total BSA was adsorbed, it would be above the threshold of detection limit (3mg/ml). Spectra of pure native BSA and denatured BSA (heated to 80 °C for 2 hours) were also measured.

Around 20 μl of cream phase was loaded onto the crystal of a TENSOR 37 spectrometer (Bruker) with a Specac Golden Gate single-reflection ATR unit (Specac Ltd., Orpington, UK). The sample detector was purged with nitrogen and an absorption spectrum was recorded in the range of 1000-4000 $cm^{-1}$, Background measurements were based on 64 scans, and sample measurements were based on 64 scans. The data obtained by FT-IR were processed using the options of OPUS version 6.5. Each emulsion spectra was subtracted from the protein-emulsion spectra to obtain protein-only signals, if present. The spectra were then deconvolved using secondary derivative and linear baseline correction of the amide ! band to discern if any protein peaks from BSA were present.

*Assessing conformation of BSA in aqueous phase of emulsion*

Near-UV circular dichroism (Jasco J-815 Spectropolarimeter) was used to assess for changes in conformation of BSA in the aqueous phase of the emulsion. An HLB 13 emulsion mix with 0.25% surfactant concentration was prepared. BSA solution was prepared at a concentration of 4 mg/ml in a 10mM phosphate buffer (pH 7.4). As controls, native and denatured BSA samples, as well as pure emulsion samples (no proteins) were prepared as well. The emulsion mix was incubated with BSA (at 1:1 volume ratio) for 30 minutes and then centrifuged at 10000 g and filtered three times, using a 0.2μm filter to remove all emulsion particles. The filtrate was analyzed using near-UV circular dichroism. The near UV spectra was collected between 260 nm to 310 nm using a 1cm path length quartz cell in 0.1 nm steps with 4 scans per

sample. All measurements were done at 25 °C and baseline corrected using the 10mM phosphate buffer. To ensure reliable results were obtained, only spectra below 1 kV high tension (HT) dynode voltage were included. Far-UV spectra was not included in this study as the HT voltage exceeded 1kV.

*Assessing intrinsic tryptophan fluorescence of fibrinogen in aqueous phase of emulsion*

Intrinsic fluorescence of protein due to aromatic amino acids such as tryptophan provides another way of assessing tertiary structure of proteins in the aqueous phase of emulsions which may be altered by the presence of surfactants and hydrophobic emulsion droplets. Tryptophan is excited at around 275 nm and has an emission of 350 nm in aqueous environment. Denaturation of proteins result in exposure of tryptophan residues which leads to shifts in the emission peak.

A series of emulsion mixes with different concentrations of surfactant (0.25% SA, 0.5% SA, 0.75% SA) and PVP (1% PVP, 2% PVP, 4% PVP) were prepared with a final concentration of 1% fibrinogen. As controls, native and denatured (in 1% SDS) fibrinogen samples without emulsions were prepared as well. Each emulsion mix was incubated with fibrinogen for 30 minutes and then centrifuged at 10000 g to separate the aqueous phase of the emulsion. 50ul of each sample was pipetted into a 96 well plate in triplicates and plates were read using the Spectramax i3x Multi-Mode Detector at an excitation of 275 nm and emission spectra was collected from 300 nm to 400 nm.

*Assessing function of lactate dehydrogenase in aqueous phase of emulsion*

In addition to examining structural changes of proteins exposed to emulsions, the functional changes of proteins exposed to emulsions were also investigated. A colorimetric assay using lactate dehydrogenase (Sigma) was utilized to investigate the effect of exposure of the enzyme to surfactants and hydrophobic emulsion droplets. A series of emulsion mixes with different surfactant (0.25% SA, 0.5% SA, 0.75% SA) and PVP (1% PVP, 2% PVP, 4% PVP) concentration was prepared. As negative control, LDH in 1% SDS was used.

2 μl of LDH Positive Control was added to 1ml of each emulsion mix and incubated for 30 minutes. The emulsions were then centrifuged at 10000 g to separate the aqueous phase of the emulsion. 50ul of each sample was pipetted into a 96 well plate in triplicates together with 50ul of Reaction Mix per sample, according to the assay protocol. Plates were read using the Spectramax i3x Multi-Mode Detector at OD 450 nm in kinetic mode, every 3 minutes for 30 minutes at 37 °C).

**Results**

***Emulsion droplet size characterization***

*Effect of HLB ratio on droplet size*

At HLB of 9.5 no emulsions were obtained and only colloidal aggregates were formed. At HLB values of 10 and 10.5, small unstable oil-in-water and water-in-oil droplets forming biphasic emulsions were obtained. At HLB of 11, complex emulsions of water-in-oil-in-water (W-O-W) emulsions were formed. From HLB of 11.5 to 15, metastable oil-in-water emulsions were obtained. Therefore, increasing the HLB ratio stabilizes the oil-in-water emulsion phase with a trend towards smaller mean droplet sizes. Figure 7 shows light microscopy of the Oil Red O stained emulsions showing phase transition of the emulsions as HLB value increases (Magnification 10x). Mean droplet diameter decreased significantly as HLB values increased ($p<0.05$).

*Effect of varying surfactant concentration at different shear rate*

Increasing the shear rates for each of the surfactant concentration resulted in a gradual decrease of mean droplet sizes (Figure 8). No emulsions were obtained for shear rates of 500 rpm in this system for all ranges of surfactant concentration. This suggests there is a threshold of shear rate to be reached for emulsion formation above which, emulsion formation is shear rate dependent. Increasing surfactant concentration at 1500 rpm did not significantly change the mean droplet sizes as the emulsions formed were unstable and flocculating/coalescing. This resulted in very large standard deviations of mean droplet diameter. However, from 2000 rpm increasing surfactant concentration results in a trend towards smaller mean droplet sizes. Increasing shear rate at all surfactant concentrations appear to reduce droplet size. However, increasing the shear rate at surfactant concentration of 0.25% appears to decrease the mean droplet size of the oil-in-water emulsion up to 2500 rpm (giving a mean droplet size 85 ± 37 microns).

When no surfactant was added to the mineral oil, 25mM MES/150 mM NaCl buffer (pH 7.4) with 2% PVP, emulsions were extremely unstable. At concentrations of 0.25% to 1.5% of HLB 13 surfactant mix, increase in surfactant

concentration resulted in a trend towards smaller droplets as well. Figure 9 shows light microscopy of the Oil Red O stained emulsions showing decrease in mean droplet diameter as surfactant concentration increased ($p<0.05$).

*Effect of PVP concentration on droplet size*

In the HLB 13 emulsion mixture with 0.5% surfactant mix concentration without PVP, large droplet diameters of about 200 microns were obtained. Increasing the PVP concentration makes droplets smaller and more uniform. This suggests that PVP may have steric blocking properties or surface-active properties or interact with surfactant molecules at the interfacial regions that further stabilize the emulsion. It may also exert an effect by influencing viscoelastic properties of the emulsion. Figure 10 shows light microscopy of Oil Red O stained emulsions showing a decrease in mean droplet diameter as PVP concentration increased ($p<0.05$).

*Effect of temperature on droplet size*

Between 4 and 37 °C, temperature change had negligible effect on droplet size. There was a slight increase in mean droplet size when temperature was changed from 22 °C to 37 °C to about 105 microns. However, this was not a significant effect. Temperatures above 37 °C were not tested as proteins are denatured beyond physiological temperatures. In addition, no phase inversion was observed in temperatures between 4 and 37 °C. Figure 11 shows light microscopy of Oil Red O stained emulsions showing no significant change in mean droplet diameter as temperature is increased ($p>0.05$).

*Effect of varying surfactant concentration at different shear rate*

Increasing the shear rates for each of the surfactant concentration resulted in a gradual decrease of mean droplet sizes (Figure 8). No emulsions were obtained for shear rates of 500 rpm in this system for all ranges of surfactant concentration. This suggests there is a threshold of shear rate to be reached for emulsion formation above which, emulsion formation is shear rate dependent. Increasing surfactant concentration at 1500 rpm did not significantly change the mean droplet sizes as the emulsions formed were unstable and flocculating/coalescing. This resulted in very large standard deviations of mean droplet diameter. However, from 2000 rpm increasing surfactant concentration results in a trend towards smaller mean droplet sizes. Increasing shear rate at all surfactant concentrations appear to reduce droplet size. However, increasing the shear rate at surfactant concentration of 0.25% appears to decrease the mean droplet size of the oil-in-water emulsion up to 2500 rpm (giving a mean droplet size $85 \pm 37$ microns).

***Emulsion stability characterization***

*Effect of HLB value on emulsion stability*

At HLB 9.5 and 10, the emulsion stability measured by the turbiscan was very low, with large increases in backscatter at the bottom of the cells, indication sedimentation. At HLB 10.5, sedimentation was less pronounced. However, at HLB 11, changes in light transmission and backscatter indicated formation of 3 distinct layers indicating phase transition from water-in-oil to oil-in-water emulsions. From HLB 11.5 to HLB 13, changes in light transmission and backscatter due to creaming decreased as HLB values increased, indicating greater stability of the emulsion system. Figure 12 shows Turbiscan emulsion stability profiles over time for emulsions at different HLB values (HLB 11.5 and 13.0).

*Effect of surfactant concentration on emulsion stability*

Emulsions without surfactants, phase separated in under one minute into separate continuous oil and water phases. As surfactant concentration increased, changes in light transmission and backscatter, measured by the turbiscan, due to creaming decreased indicating increase in stability of the emulsions. Figure 12 shows Turbiscan emulsion stability profiles over time for emulsions at different surfactant concentrations).

*Effect of* PVP *concentration on emulsion stability*

Emulsions without PVP creamed rapidly but did not phase separate completely. As the concentration of PVP increased, the emulsions became much more stable with little changes in light transmission and backscatter. Figure 12 shows Turbiscan emulsion stability profiles over time for emulsions without PVP, or with 4% PVP.

***Emulsion viscoelasticity characterization***

*Effect of* PVP *concentration on emulsion viscoelasticity*

Although PVP appears to have an effect on droplet size and stability, emulsions with PVP only (no surfactants) were highly unstable and phase separated without forming emulsions. This suggests that the effect of PVP is unlikely to be due to its surface-active property. One possible explanation is that PVP increases stability of the emulsion through changes in viscoelasticity of the emulsion. We therefore studied the effect of PVP on viscoelastic properties of the emulsion at various frequencies. Microrheology results shows that increasing PVP concentration resulted in an increase in dynamic changes to the loss modulus (G") of the emulsion mix (Figure 13). Storage modulus G' was highest for emulsion with highest PVP concentration (4% PVP), followed by 2% PVP and 0.5% PVP, regardless of range of frequencies. At very low frequencies (<50 Hz), emulsions with highest PVP concentrations (4% PVP) had the highest G" followed by emulsions with 2% PVP and 0.5% PVP. At mid-range frequencies (50-3500 Hz), the emulsions 2% PVP had highest G", followed by emulsions with 0.5% PVP then 4% PVP. At high-range frequencies (>3500 Hz), emulsions with 0.5% PVP had highest G" followed by emulsions with 2% PVP and 4% PVP. This indicates that the shear thinning effect increased with increasing PVP concentration.

***Characterization of protein-emulsion interaction***

*Localization of proteins in emulsion mix*

FITC-BSA formed a shell around each emulsion droplet rather than becoming uniformly dispersed in the oil-in-water emulsion, suggesting that the proteins had either adsorbed onto the interface boundary with the Tween20/Span 20 surfactants or formed an outer shell surrounding the surfactant-coated emulsion droplets. Figure 14 shows confocal microscopy images showing "coating effect" as FITC-BSA and surfactant interact close to the interfacial oil-aqueous regions (10x magnification). To distinguish between the two, the emulsion mix was centrifuged to induce creaming of the emulsions. If FITC-BSA was adsorbed at the interface, they are likely to remain in the creamed layer. However, if FITC-BSA had formed a loose outer shell surrounding each droplet, centrifugation would disrupt the protein-surfactant interaction, allowing the FITC-BSA to return into the aqueous phase.

The protein concentrations in each phase were then quantified to observe the proportion of FITC-BSA in the oil and aqueous phase. Relative fluorescence in the aqueous and oil phase shows that most of the FITC-BSA is concentrated in the aqueous phase rather than oil phase, regardless of HLB value, surfactant concentration, PVP concentration or temperature. Figure 15 shows the quantification of relative fluorescence intensity of FITC-BSA in aqueous phase vs. oil phase, demonstrating that most of the protein remains concentrated in the aqueous phase while the amount of protein in the oil phase is too low to be reliably quantified. Indeed, almost none of the FITC-BSA is detectable in the oil phase, suggesting that the FITC-BSA shells were only loosely bound to the emulsion droplets rather than being primarily adsorbed at the interface. This suggests that the polyethylene oxide and sorbitan surfactant moieties prevent protein adsorption at the o/w interface and protects protein from denaturation at the interface.

*Assessing conformation of adsorbed BSA*

To further confirm our findings, FTIR spectra of the cream phase of emulsions with and without BSA were obtained. The spectra of both emulsions with and without BSA were similar with no demonstrable changes in the Amide I region (1600-1700 $cm^{-1}$). As there were no detectable protein signals, this suggests that almost all of the BSA is desorbed from the surface of emulsion droplets on centrifugation. This is consistent with the fluorescence quantification results. We further validated our results by performing deconvolving the FTIR spectra obtained using second derivative to reveal any hidden peaks. Four separate peaks were seen in the solution containing pure BSA, however only one peak was seen in all emulsion spectra with and without BSA. This is shown in the FTIR spectra of BSA, and emulsions with and without BSA, shown in Figure 16, which show no significant differences in the amide region (1600-1700 $cm^{-1}$). Figure 16 also shows the second derivative of subtracted FTIR spectra of cream phase of the emulsions with BSA at different HLB, surfactant and PVP concentrations, showing only water peaks with no protein signals, compared with the four peaks in BSA. This is likely to be due to the water peaks observed in the 1650 $cm^{-1}$ region, owing to slight differences in amount of water in the dispersed phase of the emulsion cream.

*Assessing conformation of BSA in aqueous phase of emulsion*

Near-UV spectra of native BSA in phosphate buffer and BSA incubated with HLB 13 emulsions demonstrated a change in $\theta$ from 260 nm to 290 nm, indicating that BSA in the aqueous phase of emulsions was still in a folded state.

Meanwhile, the spectra of denatured BSA was a flat line indicating loss of polarization of the aromatic residues (Tryptophan, Tyrosine, Phenylalanine) due to unfolding of the protein molecule. Similarly, samples with only filtered emulsions was a flat line and did not show any changes in $\theta$ from 260 nm to 290 nm, indicating that any residual emulsion particle not removed by the filtration process did not interfere with CD spectra readings (figure 17).

*Assessing intrinsic tryptophan fluorescence of fibrinogen in aqueous phase of emulsion*

Peak emission spectra of tryptophan were approximately 350 nm, regardless of concentration of surfactant or PVP. This indicates that fibrinogen in the aqueous phase of the emulsions are still in their native state. Upon denaturation with 1% SDS, there is a red-shift of the maximum intensity of the peak to 340 nm. This suggests that the fibrinogen is no longer in native state and has unfolded, thus exposing the native tryptophan residues in fibrinogen to surfactants available (figure 18).

*Assessing function of lactate dehydrogenase in aqueous phase of emulsion*

Functional changes to proteins exposed to emulsions were also assessed using an enzyme, lactate dehydrogenase (LDH). It is well known that LDH reduces $NAD^+$ to NADH. The formation of NADH can be quantified by colorimetry. In Figure 19, addition of 0.25% surfactant did not significantly affect LDH enzyme activity. However, at 0.5% surfactant concentration and above, there is a slight decrease in enzyme activity compared to the positive control. Similarly, addition of PVP resulted in decrease in enzyme activity in a concentration dependent manner. No enzymatic reaction was observed for the negative controls with 1% SDS.

**Example 4** - **Control of emulsion template size** (not according to the claimed invention) *Relationship between shear rate, surface tension and emulsion droplet size.*

The control of the emulsion droplet size is a critical aspect of the invention. in a templating emulsion system the size of the oil-in-water droplet as a porogen or pore template directly and closely determines the pore size of resultant scaffolds.

Emulsion dispersions may be considered as intrinsically unstable. This is argued from the consideration of the stability of a pure oil phase with pure water. When shaken, an emulsion will be formed, although this will be unstable and will rapidly separate out. The introduction of a surfactant changes the thermodynamic stability, because the surfactant will lower the surface energy of the interface, and provide a stable boundary layer which opposes the force of separation from a mixture.

*Theoretical relationship.*

The emulsion droplet pressure is given by the Laplace law.

$$\Delta P_d = 2\gamma/r_d$$

where the subscript d refers to the emulsion droplet.

The rate of variation of $r_d$ with $\Delta P_d$ is given by the differential $dP_d/dr = -\gamma/r_d^2$

In an example of an emulsification system, as illustrated in Figure 20, the rotating impellor imparts kinetic energy into the system, in relation to the angular velocity. The maximal angular velocity at the impellor tip is

$$2\pi\omega r_p = \tfrac{1}{2} v^2$$

where $\omega$ is the revolution frequency.

Dynamic pressure = Kinetic energy /unit volume $P_{dyn} = \rho.v^2$ where $\rho$ is the dispersed phase density.

The shear stress can be related to the dynamic pressure gradient $\delta P_d/\delta r$

As an approximation, $dP_{dyn}/dr = \rho.(2\pi.\omega.r_p)^2/r_c$ where $r_c$ is the radial shear zone (radius of mixing cylinder - radius of impellor). The shear stress determines the limit of emulsion droplet size. Thus a theoretical relationship between angular velocity of the impellor, rotational shear stress and droplet size can thus be related to the droplet size:

$$\rho\ .(2\pi.\omega.r_p)^2/r_c = -\gamma/r_d^2$$

And

$$r_d{}^2 = -\gamma r_c/\rho\,.(2\pi.\omega.r_p)^2 \quad \text{so } r_d = -\sqrt{\frac{\gamma r_c}{\rho\,.(2\pi.\omega.r_p)^2}}$$

A modified model considers rebound flow from the vessel wall. Rebound has two effects, to increase the effective velocity term and to decrease the space for the shear gradient, thus increase the shear rate. For elastic rebound, $v_p$= -$v_r$ so the velocity between propelled and rebound flow is double (v = 2$v_p$) and the shear rate is doubled due to rebound (i.e. $\delta P_d/\delta r = \rho.v^2$ / ½ $r_c$).

Thus the dynamic pressure gradient becomes:

$$dP_{dyn}/dr = 2\rho\,.(4\pi.\omega.r_p)^2/r_c.$$

Method

*Measurement of interfacial tension*

The surface tension ($\gamma$) of the aqueous phase buffer, 0.025 M MES in 0.15 M NaCl pH 7.4, pharmaceutical grade mineral oil, with 1% volume to volume Span 20 Tween 20 HLB 12.5 blend, was measured by a dynamic drop process. The density of mineral oil is 855 kg/$m^3$ and viscosity is 14.2-17 cP.

Emulsions were made by blending the above mixture at a 30:70 ratio of aqueous phase to oil phase, in a cylindrical mixing chamber with a rotating impellor driven by a variable speed mixer (Ceframo), as described in para [00110].

Droplet stability over 24 h was confirmed by failure of an emulsion of oil-red oil stained oil droplets spiked with the non-stained emulsion post formation, to coalesce or exchange with non-stained droplets.

Droplet size produced at each mixing speed were recorded within 5 minutes of mixing, by light microscopy. Emulsion droplet diameter were obtained by direct measurement using Image J from light micrograph images (data from fig 8).

Results

The relationship between impellor rpm and droplet size obtained experimentally and by theoretical calculation is shown in Figure 21 and the Table below. D(exp) is the experimental droplet diameter, D*(t0) and D*(teq) are the theoretic diameters based on initial and equilibrium values of $\gamma$, and D#(teq) is the curve for a doubling of shear rate due to rebound. The calculations used values for $\gamma$ obtained initially, on droplet formation, and after equilibration for 30 min. The values plotted for experimental data are mean values from replicate experiments. The model curves for rotational dynamic pressure gradient based on simple geometry are D*(t0) and D*(teq). The curve R#(teq) results from considering flow rebound.

| rpm | D(exp) | D($t_0$) | D($t_{eq}$) | D#(teq) | Re |
|---|---|---|---|---|---|
| 1000 | 130 | 339 | 299 | 106 | 60 |
| 1500 | 90 | 226 | 200 | 71 | 90 |
| 2000 | 60 | 170 | 150 | 53 | 119 |
| 2500 | 52 | 136 | 120 | 42 | 149 |
| 3000 | 40 | 113 | 100 | 35 | 179 |

Experimental and theoretical values of emulsion droplet diameter, and calculated Reynolds number for each condition.

Conclusions

The results obtained illustrate the control of droplet diameter obtained by one concentration of surfactant blend follows a geometrical model for shear rate to within useful practical range. The results demonstrate that a relationship exists between impellor speed (rpm) and droplet size. The experimental relationship obtained follows a similar trend to

the theoretical relationship between shear and interfacial tension. However, the experimental results show a smaller droplet size for a given shear stress, on the basis of converting angular to linear velocity, and impellor to wall space. This suggests that turbulent flow in this system results in higher shear actual than calculated by the overall system geometry. The values for the Reynolds number (Re) of the dispersed phase in the system are in the range 60-170, and thus below the expected value of around 2000 for turbulent flow. However, consideration of rebound flow can closely account for the observed results. Here, if rebound velocity is elastic, $v_p$=-$v_r$ so the velocity between propelled and rebound flow is double (v = 2$v_p$) and the pressure gradient is doubled due to rebound (i.e. $\delta P_d/\delta r = \rho.v^2$ / ½ $r_c$. Thus the dynamic pressure gradient becomes:

$dP_{dyn}/dr = 2\rho .(4\pi.\omega.r_p)^2/r_c$.

This is shown by the R#($t_{eq}$) curve in fig 20.

The model also illustrates the effect of variation in interfacial tension in the system. Although the values illustrated are for the effect of initial versus equilibrium values of $\gamma$ at the same bulk concentration of surfactant, clearly similar effects would be obtained by varying bulk surfactant concentration. Such effects would enable the experimental behaviour obtained for variation in surfactant concentration and shear rate to be accounted for.

**Example 5 - co-culture of porous protein scaffolds**

***Materials and Methods***

*Materials*

Collagen was obtained from FirstLink (Wolverhampton, UK). Bovine fibrin and thrombin were obtained from Sigma. Unless otherwise stated, all reagents were purchased from Sigma. Neonatal Human Epidermal Keratinocytes (HEK), Epilife media and Human Keratinocytes Growth Supplement (HKGS) were purchased from ThermoFisher. Neonatal Human Dermal Fibroblasts (HDF), high glucose Dulbecco's Modified Medium (DMEM) and fetal bovine serum (FBS) were obtained from ThermoFisher. hTERT-Human Microvascular Dermal Endothelial Cells (HDE), Vascular Cell Basal Medium, Microvascular Endothelial Cell Growth Kit (VEGF) were purchased from ATCC. Immortalized Bone-Marrow Mesenchymal Stem Cells (MSC) transfected with GFP were received as a gift from collaborators at Hong Kong University and low glucose Dulbecco's Modified Medium were obtained from Sigma.

*Scaffold preparation*

*(A) Preparation of emulsion-templated collagen scaffolds ("EmDerm-C")*

10ml of the HLB 13 emulsion (prepared as described above) and 10ml of neutralized collagen solution was transferred into a 50ml syringe with an open end and mixed at 1000 rpm. 4ml of the mixture was then poured into five square casting trays and allowed to gel at 37°C for 30 minutes. The emulsion-hydrogel scaffolds were then cross-linked using EDC: NHS (5:2 molar ratio) at room temperature for 1 hour. After cross-linking, each scaffold was washed with deionized water twice to remove excess cross-linker.

Each scaffold was then washed with 80% isopropanol for 15 minutes on a shaker to elute the trapped oil droplets within the collagen gel. Following that, each scaffold was washed with deionized water three times to remove any excess solvent and oil. 5% of P68 was added to the scaffolds and allowed to incubate for 10 minutes. Finally, the scaffolds were quickly rinsed with deionized water to remove any excess excipient solution before freeze-drying at -30°C.

*(B) Preparation of emulsion-templated fibrin scaffolds ("EmDerm-F")*

10ml of the HLB 13 emulsion, 8ml of 2% fibrinogen solution and 2ml of thrombin (10 unit/ml) was transferred into a 50ml syringe with an open end and mixed at 1000 rpm. 4ml of the mixture was then poured into square casting trays and allowed to gel at 37°C for 30 minutes. The emulsion-hydrogel scaffolds were then cross-linked using EDC: NHS (5:2 molar ratio) at room temperature for 1 hour. After cross-linking, each scaffold was washed with deionized water twice to remove excess cross-linker. Each scaffold was then washed with 80% isopropanol for 15 minutes on a shaker to elute the trapped oil droplets within the collagen gel. Following that, each scaffold was washed with deionized water three times to remove any excess solvent and oil. 5% of P68 was added to the scaffolds and allowed to incubate for 10 minutes. Finally, the scaffolds were quickly rinsed with deionized water to remove any excess excipient solution before freeze-drying at -30°C.

(*C*) *Preparation of emulsion-templated collagen-fibrin scaffolds ("EmDerm-CF')*

Composite emulsion-templated collagen-fibrin scaffolds were also developed using the decane emulsion system. 10ml of the HLB 13 emulsion was added to 5ml of neutralized collagen solution and 5ml of 2% w/v fibrinogen-thrombin in a 2:1:1 volume ratio and mixed at 1000 rpm. Each emulsion-hydrogel scaffold were then poured into square casting trays and allowed to gel at 37°C for 30 minutes. The emulsion-hydrogel scaffolds were then cross-linked using EDC: NHS (5:2 molar ratio) at room temperature for 1 hour. After cross-linking, each scaffold was washed with deionized water twice to remove excess cross-linker. Each scaffold was then washed with 80% isopropanol solution for 15 minutes on a shaker to elute the trapped oil droplets within the scaffolds. Following that, each scaffold was washed with deionized water three times to remove any excess solvent and oil. 5% of P68 was added to the scaffolds and allowed to incubate for 10 minutes. Finally, the scaffolds were quickly rinsed with deionized water to remove any excess excipient solution before freeze-drying at -30°C.

Each of the EmDerm-C, EmDerm-CF and EmDerm-F scaffolds were cut into 6mm discs using punch biopsies. Each scaffold was washed in phosphate buffered solution (PBS) three times and UV irradiated for 15 minutes. Each scaffold was incubated in the respective media overnight for 24 hours before used for cell seeding experiments. Collagen was obtained from FirstLink (Wolverhampton, UK). Bovine fibrin and thrombin were obtained from Sigma. Unless otherwise stated, all reagents were purchased from Sigma. Neonatal Human Epidermal Keratinocytes (HEK), Epilife media and Human Keratinocytes Growth Supplement (HKGS).

*Cell culture*

HEK cells were sub-cultured using Epilife media with added HKGS. Only cells between Passage 2 and 7 were used in experiments. HDF cells were sub-cultured using high glucose DMEM (4.5g/L) with 10% FBS and 1% penicillin/streptomycin. Only cells between Passage 2 and 9 were used in experiments. Immortalized HDE cells were sub-cultured using Vascular Cell Basal Medium with Endothelial Cell Growth Kit-VEGF containing the following supplements: rh VEGF (5 ng/ml), rh EGF (5 ng/ml), rh FGF basic (5 ng/ml), rh IGF-1 (15 ng/ml), L-glutamine (10 mM), Heparin sulfate (0.75 Units/ml), Hydrocortisone (1 μg/ml), Ascorbic acid (50 μg/ml), Fetal bovine serum (2%). MSC cells were sub-cultured using low glucose DMEM (1g/L) with 10% FBS and 1% penicillin/streptomycin. All cells were passaged at around 80% confluence using standard Trypsin-EDTA.

*Co-culture of scaffolds*

Each cell type was passaged using 1x Trypsin-EDTA and suspended in their respective medium. 10ul of the cell suspension was added to 10ul of tryphan blue and counted using the Countess Cell Counter. The cell suspensions were then serially diluted to achieve a concentration of 5 x 104 cells/ml. 200ul of each cell suspension mixture (HEK/HDF, HDF/HDE, MSC/HDE) was then added in a dropwise manner onto each respective scaffold (EmDerm-C, EmDerm-CF, EmDerm-F) to achieve an evenly seeded scaffold and left to incubate at 37°C at 5% CO2 for 4 hours in a 96 well plate. Cells were fed every 2-3 days for up to 14 days. Two sets of co-culture experiments were performed - one for cell lysis and one for fixation and imaging.

*Cell Proliferation Assay*

On Day 3, 7 and 14, a cell proliferation assay using CCK-8 (Sigma, UK) was done to determine cell growth in each scaffold. CCK-8 is a highly sensitive colorimetric cell counting assay based on a highly water-soluble tetrazolium salt (WST-8), which is reduced by dehydrogenase in cells to give a yellow-colored formazan dye. The amount of the formazan dye generated by cellular metabolism is directly proportional to the number of living cells. 10μl of CCK-8 solution (neat) was pipetted into each well of 100μl cell media. Each plate was incubated for 4 hours in the incubator at 37 °C and 5% $CO_2$. Subsequently, 50μl of each well's contents were pipetted into a fresh plate and absorbance was measured at 450 nm using the Spectramax i3 Multi-Mode Plate Reader (Molecular Devices, CA, USA). 10μl of CCK-8 was also added to a dilution series of known concentrations of cells to obtain a calibration curve in a 96 well plate. Cell-free media with CCK-8 solution was used as negative controls. Three replicates were tested for each sample. Lastly, the scaffolds were fixed in neutral buffered formalin (NBF) on Day 14 for further imaging.

*Air-Liquid Interface (ALI) Co-Culture of Human Keratinocytes and Dermal Fibroblasts*

5000 cells/well of HDFs were seeded onto each scaffold (EmDerm-C, EmDerm-CF, EmDerm-F, Matriderm and Integra) and cultured for a week in DMEM (4.5g/L glucose) + 10% FBS + 1% penicillin/streptomycin. After a week, 5000 cells/well of HEK were seeded onto the other side of each scaffold and cultured for a week in a transwell plate,

fully immersed in media. Media used was Epilife + HKGS and DMEM (4.5g/L glucose) + 10% FBS + 1% penicillin/streptomycin (at a 2:1 volume ratio). After 7 days, the scaffolds were lifted to air-liquid interface to allow for differentiation and stratification of the keratinocytes. Media was changed every other day. On Day 21 of culture, the scaffolds were fixed in neutral buffered formalin for scanning electron microscopy (SEM) imaging and histological assessment.

*Immunofluorescence staining of co-cultured scaffolds*

Each seeded scaffold was fixed in neutral buffered formalin for at least 24 hours. The scaffolds were washed 3 times with PBS. The fixed cellularized scaffolds were then permeabilized using 0.1% Triton X-100 in PBS for 30 minutes and washed 3 times with PBS. The scaffolds were then blocked with 1% bovine serum albumin (BSA) in PBST (PBS + 0.1% Tween 20) for an hour and washed. Primary antibodies were then added and left overnight on an orbital shaker. On the following day, the primary antibody solution was aspirated, and scaffolds were washed with PBS 3 times. Secondary antibodies were then added and left overnight on an orbital shaker. Cells were then counter-stained with 0.1ug/ml of Hoescht for 5 minutes and then rinsed with PBS three times.

For the HEK/HDF co-cultured scaffolds, 1:100 mouse anti-human pancytokeratin (Abcam) and 1x Phalloidin 488 (ThermoFisher) were used to stain for HEK and HDF respectively. 1:500 goat anti-mouse antibody (AlexaFluor 568, ThermoFisher) was used as a secondary antibody. For the HDF/HDE co-cultured scaffolds, 1:4 mouse anti-human CD31 (Dako) and 1:1000 rabbit anti-human vWF (Dako) were used as primary antibodies. Goat anti-mouse (AlexaFluor 488, ThermoFisher) and goat anti-rabbit (AlexaFluor 568, ThermoFisher) were used as secondary antibodies. Phalloidin 647 was also used to stain actin filaments. For MSC/HDE co-cultured scaffolds, 1:4 mouse anti-human CD31 (Dako) and 1:1000 rabbit anti-human vWF (Dako) were used as primary antibodies. Goat anti-mouse (AlexaFluor 647, ThermoFisher) and goat anti-rabbit (AlexaFluor 568, ThermoFisher) were used as secondary antibodies.

*SEM Imaging Scaffold Preparation*

HEK/HDF scaffolds which were cultured at air-liquid interface were imaged under scanning electron microscopy to observe for stratification of keratinocytes when lifted to air-liquid interface. Fixed scaffolds were dehydrated in an ethanol gradient as follows: 25% ethanol, 50% ethanol, 75% ethanol, 80% ethanol, 90% ethanol and 100% ethanol. Finally, the scaffolds were transferred into wells containing hexamethyldisilazane (HMDS) and left to dry in the fume hood overnight. This is to ensure that the samples are completely dried prior to imaging. Each scaffold was then sputter coated with gold for 120 seconds and imaged using the Zeiss scanning electron microscope.

***Results and Discussion***

*Cell Proliferation Assay*

Cell Proliferation of co-cultures (HEK/HDF, HDF/HDE and MSC/HDE) were measured using the CCK-8 assay. The results are shown in Figure 22. In the HEK/HDF co-culture, EmDerm-C had similar rates of cell proliferation to the commercially available Integra and Matriderm scaffolds (not in accordance with the invention). This is rather unsurprising as all three are made up of collagen scaffolds. HEK/HDF in EmDerm-CF and EmDerm-F had lower cell proliferation rate compared to other scaffolds. EmDerm-F was partially degraded by Day 3 and completely degraded by Day 7. EmDerm-CF had lower cell proliferation compared to the controls - Matriderm and Integra on Day 3. In the HDF/HDE and MSC/HDE co-culture, all the EmDerm scaffolds had comparable rates of cell proliferation compared to both Integra and Matriderm controls. This was consistent from Day 3 to Day 14 of the co-culture experiment.

*Fluorescence Imaging*

*(1) EmDerm-C*

For the co-culture of HDF and HDE, there was good cell growth of both cell types. There were more fibroblasts present compared to endothelial cells due to the faster rate of growth of fibroblasts. The fibroblasts and endothelial cells also appear to grow in clusters with the endothelial cells forming pseudo-tubular structures and nascent vascular networks while the fibroblasts appear to grow around the endothelial cells. See the images shown in in (A) of Figure 23, which show that the HDEs appear to form vascular networks with HDFs growing around these vessel-like structures.

For the co-culture of MSC and HDE, both cell types proliferated well on the scaffolds. However, there were more MSCs compared to endothelial cells as they tend to grow faster and are less fastidious. The endothelial cells did not appear to express or secrete vWF significantly and were proliferating in a disorganized manner. There was no clear formation of early vascular structures noted, as seen in the HDF/HDE co-culture. See the images shown in (B) of Figure

23, which show that the MSCs retained their elongated morphology while the HDEs are interspersed between MSCs.

For the co-culture of HEK and HDF, there was good proliferation of HDF and HEK. HEK cells had flattened, stretched out morphology and mostly grew on the surface of the scaffold, while HDF cells migrated into the scaffold and had a more elongated morphology. See the images in (C) of Figure 24, which show that HEK grew in a sheet-like manner with HDFs scattered in between.

*(2) EmDerm-CF*

For the co-culture of HDF and HDE, the fibroblasts and endothelial cells grew in clusters with both the fibroblasts growing around the networks formed by the endothelial cells. They also appeared to grow around pore structures of the scaffold. See the images in (A) of Figure 24, which show that the HDEs and HDFs appeared to grow in an interdispersed manner around each other.

For the co-culture of MSC and HDS, the MSC and endothelial cells grew around each other and adhered to the walls of the pores of the scaffolds. The MSCs retained their spindle like morphology. There was minimal production of vWF intracellularly and extracellularly. See the images shown in (B) of Figure 25, which show that the MSCs retain their elongated morphology and HDEs were scattered around the scaffold.

For the co-culture of HEK and HDF, there was good proliferation of both cell types. HEK cells grew in sheet-like layer while HDF cells grew in clusters. See the images shown in (C) of Figure 25, which show that the HEKs grew in sheets while HDFs grew in clusters.

*(3) EmDerm-F*

For the co-culture of HDF and HDE, there was formation of vascular networks of endothelial cells with fibroblasts clustering around the endothelial cells. The cells also appeared to grow around the pores of the scaffold. There was minimal expression of vWF. See the images in (A) of Figure 26, which show that the HDEs grew in a network-like manner with HDFs growing in between.

For the co-culture of MSC and HDE, there was good cell growth of both cells. However, the MSCs were much more abundant and grew around scaffold surfaces, while the endothelial were interdispersed between MSCs. There was minimal expression of vWF. See the images in (B) of Figure 26, which show that the MSCs retained their elongated morphology with HDEs scattered amongst the MSCs.

For the co-culture of HEK and HDF, EmDerm-F scaffolds seeded with HEK and HDF were completely degraded after Day 3. Therefore, no images were obtained.

*(4) Integra*

For the co-culture of HDF and HDE, the fibroblasts grew in sheets with the endothelial cells forming network-like structures in between. There was a much higher cell proliferation of fiboblasts as compared to endothelial cells. There was significant production of intracellular vWF as seen in the red channel within the endothelial cells as well. See the images in (A) of Figure 27, which shows that the HDFs grew in sheets with HDEs forming network-like structures.

For the co-culture of MSC and HDE, there was a very high proliferation of MSCs with minimal organization. MSCs appeared to grow in clumps, with loss of their spindle-like morphology. The endothelial cells also appeared to grow interspersed amongst the MSCs with no formation of vascular networks. There was minimal expression of vWF intracellularly or extracellularly. See the images in (B) of Figure 27, which show that the MSCs grew in clusters with HDEs interspersed in between.

For the co-culture of HEK and HDF, there was extensive growth of both HEK and HDF in a sheet-like manner. Due to the high rate of proliferation of cells, there was overcrowding of cells and it is difficult to distinguish HEK from HDFs as HEK cells do not adopt the polygonal spread out morphology as in EmDerm-C and EmDerm-CF scaffolds. See the images in (C) of Figure 28, which show that the HEK and HDFs grew in a sheet-like manner.

*(5) Matriderm*

For the co-culture of HDF and HDE, there was significant growth of fibroblasts throughout the scaffold, with endothelial cells forming network like structures within the scaffold itself. There was minimal expression of vWF as well, intracellularly and extracellularly. See the images in (A) of Figure 28, which show that the HDFs grew in a sheet-like manner while HDEs formed vessel-like structures.

For the co-culture of MSC and HDE, there was good cell growth of both MSC and endothelial cells. However, they tend to grow in clusters around each other. There was minimal production of vWF as well. There was no clear vascular network pattern of endothelial cell growth. See the images in (B) of Figure 29, which show that the MSCs grew

in a haphazard manner with HDEs scattered in between.

For the co-culture of HEK and HDF, there was extensive growth of both HEK and HDF in a sheet-like manner. However, it was difficult to distinguish HEK from HDFs as HEK cells do not adopt the characteristic flattened-out morphology typical of differentiated keratinocytes. See the images in (C) of Figure 29, which show that the HEKs and HDFs grew in a sheet-like manner.

*Scanning Electron Microscopy Imaging*

The HEK cells adopted a very flattened, spread-out morphology when seeded onto scaffolds which is a key characteristic of keratinocytes, indicating good cellular attachment. The cells were also found to grow over each other in a stratified manner. See the images in Figure 30 on EmDerm-C (A), EmDerm-CF (B), Integra (C) and Matriderm (D), which show a flattened, stretched out morphology and partial stratification. For the EmDerm-F scaffold, the keratinocytes appeared to degrade at Day 7, which suggests that fibrin-based EmDerm scaffolds were less suitable to create a bilayer skin construct as compared to collagen-based scaffolds.

The results show that cell proliferation of co-cultured HEK and HDF in EmDerm-C is comparable to the commercially available Integra and Matriderm scaffolds. This may be due to all of these scaffolds being made up of collagen material, which is favourable for growth of keratinocytes and fibroblasts. HEK/HDF growth was slower in EmDerm-CF scaffolds compared to Integra and Matriderm on Day 3, but improved between Day 7 to Day 14. EmDerm-F scaffolds were noted to be partially degraded by Day 3 of HEK/HDF co-culture. This is likely due to release of fibrinolytic degradative enzymes by keratinocytes.

EmDerm-C scaffolds seeded with HDE and HDF appeared to form nascent networks of vascular structures with HDFs growing around these tubular networks. This was also seen in EmDerm-CF and EmDerm-F but in a less organized fashion.

The penetration of cells appeared to be better on EmDerm scaffolds compared to Integra and Matriderm. In both Integra and Matriderm, seeded cells appeared to proliferate and spread on the surface of the scaffolds. In contrast, cells on the EmDerm scaffolds appeared to migrate better into the scaffolds, rather than growing along the scaffold surface. The improvement in depth of cell penetration may be due to the micro- and nano-structure of EmDerm scaffolds, which allows better diffusion of oxygen and nutrients into the scaffold.

Stratification of keratinocytes when cultured at air-liquid interface was also observed in EmDerm-C and EmDerm-CF scaffolds. There was good cellular attachment and proliferation of keratinocytes.

In summary, the EmDerm scaffolds have been shown to be effective dermal skin substitutes, supporting growth of fibroblasts, keratinocytes and endothelial cells. They also promoted the vascularization process by stimulating organization of endothelial cells into nascent vascular networks. The scaffolds also can be used as an epidermal-dermal bilayer skin substitute as they support growth of keratinocytes and fibroblasts, as well as stratification of keratinocytes when grown at air-liquid interface. This may be useful for single stage reconstruction of burns and chronic wounds as both the dermal and epidermal layers can be applied simultaneously.

**Example 6 - co-culture of porous protein scaffolds**

***Materials and Methods***

*Materials*

Type I collagen from rat tail was commercially obtained from FirstLink (Wolverhampton, UK). Bovine fibrin and thrombin were obtained from Sigma UK. Unless otherwise stated, all reagents were obtained from Sigma UK. In order to measure the amount of lipopolysaccharide endotoxin, the Limulus amebocyte lysate (LAL) assay was used (Hycult Biotech, PA, USA). Rats were obtained from a licensed animal provider in the UK.

*Scaffold Preparation*

EmDerm-C, EmDerm-CF and EmDerm-F scaffolds were manufactured as described in Example 5. All scaffolds were made using 0.1% surfactant mix and cross-linked for an hour using EDC-NHS with 5% P68 as excipient prior to freeze-drying. Scaffolds were cut into 8mm discs using punch biopsies. Each scaffold was washed in phosphate buffered solution (PBS) three times and UV irradiated for 15 minutes. The scaffolds were then left overnight in 5% penicillin/streptomycin solution. The scaffolds were then given one final rinse with normal saline before used for implantation.

*Endotoxin* assay

The reagents were brought to room temperature and prepared as per manufacturer protocol. Each scaffold was weighed and cut into 1g portions and incubated in 200μl endotoxin free water to dissolve any endotoxin present in the scaffold (neat sample solution). Standards were prepared as per manufacturer instructions. Serial dilutions of each sample solution were prepared in duplicates. 50μl of each sample solution was added to the endotoxin free 96-well plate. Endotoxin free water was used as a negative control. 50μlof LAL reagent was then added to each sample solution. The plate was incubated at room temperature for 20 minutes. Absorbance values were determined using a UV-Vis plate reader (Spectramax i300, Molecular Devices, CA, USA) at 405nm.

*Animals*

Three healthy Sprague-Dawley rats (2-month old, males, body weight between 500 - 600 grams) were individually housed. All animals had free access to standard nutritionally balanced food and drinking water and were maintained on a 12-hour light/dark cycle. Environmental enrichment was provided to keep the rats active and healthy. The study was carried out at Northwick Park Institute of Medical Research (NPIMR). This study was conducted with ethical approval from the Animal Welfare and Ethical Review Bodies (AWERB) at NPIMR under a project license granted by the Home Office. All surgical procedures were done under isofluorane inhalational anaesthesia and analgesia was provided pre- and post- procedure to minimize animal suffering. All animals were acclimatized for two weeks prior to study and monitored closely for signs of complications post-operatively.

*Scaffold implantation*

Four scaffolds were implanted on the dorsum of each rat. Scaffolds tested were EmDerm-C, EmDerm-CF and EmDerm-F. A total of three rats were used in this study (one scaffold per rat). A diagrammatic representation of the scaffold implantation in each rat is shown in Figure 31. Each rat was implanted with 4 scaffold discs. On one side, two wounds were subcutaneous pockets with scaffold implanted subdermally, while on the other side two wounds were abraded to remove part of the dermis and panniculli carnosus, leaving only the epidermis and part of the dermis overlying the scaffold to simulate a split-thickness skin graft wound model. Biopsies were taken on Day 7 and Day 14. Blood sampling was done on Day 0, Day 7 and Day 14.

*Surgical procedure*

All surgical procedures were aseptically performed using sterile equipment in the animal facility operating rooms. The dorsum of each animal was shaved and cleaned using chlorhexidine. Two vertical incisions measuring 15mm were made and blunt dissection was done to create subcutaneous pockets in either side of the incision. For the abraded wounds, a scalpel was used to remove part of the dermis and paniculli from the incision edge (approximately 225 $mm^2$). Scaffolds were carefully inserted into each subcutaneous pocket. A tunnelling stitch was used to close the subcutaneous pockets, to prevent scaffold migration. The wounds were sutured closed using Vicryl sutures and clips were applied to protect the wound and prevent the animals from biting the suture ends.

*Wound biopsies*

At Day 7, two wound biopsies were performed per rat - one for the non-abraded wound and one for the abraded wound. 5mm punch biopsies were used. As far as possible, biopsies were done in a manner which included the part of the scaffold and part of the surrounding tissues around the scaffold to determine if there was any inflammatory reaction within the scaffold and in the tissue around the scaffold. Biopsy specimens were immediately fixed in neutral buffered formalin for 24 hours before embedding in paraffin blocks. Photographs of the biopsies and biopsy wounds were also taken. Wounds were then sutured closed with Vicryl and clips applied. At Day 14, all animals were euthanized and all four wounds were biopsied/excised.

*Blood sampling*

At Day -7 (baseline), Day 7 and Day 14 tail vein blood sampling was done to assess if there was any systemic inflammatory response. Briefly, each rat was immobilized using a tunnel and a 24G needle was used to aspirate 1.5mL of blood volume from the superficial caudal vein at each time point for downstream haematological and biochemical assessment. 500μl of blood was collected into an EDTA tube and the remaining blood was kept in a heparinised tube for C-reactive protein (CRP) ELISA analysis.

*Haematological and biochemical assessment*

For the haematological assessment, the blood samples were transferred from the EDTA tubes into an automated veterinary haematology analyser (Procyte Dx Analyser) which gave the following readings: erythrocyte count (RBC), total leucocyte count (WBC), differential white cell count (Diff), haemoglobin (Hb), haematocrit (Hct), mean cell volume (MCV), mean cell haemoglobin (MCH), mean cell haemoglobin concentration (MCHC), platelets (PLT), mean platelet volume (MPV). In particular, the WBC and PLT values were compared at each time point to determine if there was any change in these inflammatory markers in each rat.

CRP ELISA analysis was performed using the Abcam Rat CRP ELISA kit, according to the manufacturer's protocol. Serum samples were centrifuged at 3000 g for 10 minutes then diluted at 1 :60,000. Briefly, reagents were brought to room temperature. Serial dilutions of standards and samples were prepared in duplicates. 50μl of each standard/sample was added to the 96-well plate and incubated for 2 hours. The content of each well was then aspirated and washed five times with the 1x wash buffer. Subsequently, 50μl of biotinylated CRP antibody was added and incubated for an hour. The wells were then washed again and 1x SP Conjugate was added to each well and incubated for 30 minutes. Then, the wells were washed again and 50μl of chromogen substrate were added to each well and incubated in the dark for 10 minutes. Finally, 50μl of stop solution was added to each well and absorbance was read at 450 nm immediately (wavelength correction done at 570 nm). OD readings from each sample was compared at different time points to determine if there was any change in CRP levels in each rat.

*Histological assessment of inflammatory response*

Each tissue sample was fixed for 24 hours in neutral buffered formalin (NBF), processed and embedded into paraffin blocks. A microtome was used to section the samples into 10 um thickness, mounted on glass slides, deparaffinized then stained using conventional Haematoxylin-Eosin (H&E) protocol. Briefly, each slide was deparaffinized in Citroclear for 3 minutes (x2), then transferred into 100% ethanol for 3 minutes (x2), 70% ethanol for 3 minutes, 50% ethanol for 3 minutes, and tap water for 3 minutes. The slides were then transferred into filtered Harris' Haematoxylin for 4 minutes then back into running tap water for 3 minutes. Then, slides were dipped ten times in 1% acid alcohol, Scott's tap water and 0.5% lithium carbonate (with tap water washes between each solution). The slides were then dipped in 50% ethanol and 70% ethanol ten times before incubating in Eosin Y for 1 minute. The slides were then washed in 90% ethanol for 1 minute (x4), 100% ethanol for 1 minute (x2) and xylene for 1 minute (x2) before mounting with a coverslip. Each slide was imaged using a Leica Scanscope at 40x magnification for downstream analysis.

*Quantification of scaffold vascularization*

Degree of vascularization of each scaffold were quantified at Day 14. Ten frames per slide for each tissue specimen were taken at 40x magnification using a light microscope. Each frame was taken at random. The number of vascular structures found within the scaffolds were manually counted for quantification. Mean ± SD for each scaffold were calculated and analysed for statistical significance.

*Quantification of local inflammation*

Degree of inflammation surrounding each scaffold was quantified at Day 7. Ten frames per slide for each tissue specimen were taken at 40x magnification using a light microscope. Each frame was taken at random. The degree of inflammatory response found within the scaffolds were manually graded as: 1 - minimal, 2 - mild, 3 - moderate and 4 - severe. This was done using reference frames for neutrophil density. Mean ± SD of total inflammatory grade for each scaffold were calculated and analysed for statistical significance.

*Statistical analysis*

All values are expressed as mean ± SD for each experiment. One-way ANOVA was used to determine significance with post-hoc Tukey test. Repeated measures ANOVA was used for the values which were measured repeatedly at different time points. All statistical analysis was done using GrapPad Prism 4 (Graph Pad, La Jolla, CA) and a p-value of $<0.05$ was considered statistically significant.

***Results and Discussion***

*Endotoxin Assay*

The endotoxin assay (LAL assay) is one of the recognized assays for quantifying amount of endotoxin in materials under the Food and Drug Administration (FDA) Regulations. The accepted endotoxin limit is 5 EU/kg. In all the scaffolds tested, the endotoxin levels were below this detection limit. The endotoxin level for EmDerm-C was 0.08 EU/g, while EmDerm-CF was 0.10 EU/g and EmDerm-F was 0.12 EU/g respectively.

*Haematological analysis of systemic inflammatory response*

There was no significant change in total white blood cell count (WBC), neutrophil count or lymphocyte count in all three animals during the entire period of the study (14 days). There was also no significant change in platelet count, which is a known marker of systemic inflammation. There was also no gross evidence of localized inflammation such as erythema, swelling, or increased warmth in the area of scaffold implantation which is consistent with the blood results.

*Biochemical analysis of acute phase reactant*

There was no significant change in concentration of C-reactive protein (CRP) as measured using the ELISA assay. This is consistent with the haematological analysis as above, indicating that the EmDerm scaffolds were not pro-inflammatory at a systemic level.

*Histological assessment of inflammatory response*

*Week 1*

*(1) EmDerm-C*

There was no evidence of significant localized inflammation within and around the collagen scaffold. Scaffolds were partially degraded and were loosely attached to the connective tissue surrounding the scaffold. There was no evidence of revascularisation at Day 7. See Figure 32. The left image (abraded) and right image (non-abraded) both show minimal local inflammatory changes and partly degraded scaffolds.

*(2) EmDerm-CF*

A moderate degree of inflammation was present within the scaffold and around the EmDerm-CF scaffolds. There was a lesser degree of scaffold degradation despite the influx of inflammatory lymphocytes around the scaffold. There were also no nascent vascular structures present at this stage. See Figure 33. The abraded wound showed minimal inflammatory reaction compared to the non-abraded wound where there is a mild to moderate inflammatory reaction around the scaffold. In both cases, scaffolds were partly degraded.

*(3) EmDerm-F*

A moderate degree of inflammation was present within or around the EmDerm-F scaffolds, which is similar to the EmDerm-CF scaffolds. However, there was minimal scaffold degradation despite the inflammatory response around the scaffold. There was also no vascularization of the scaffold present at this stage. See Figure 34. In both scaffolds, there was mild to moderate inflammatory reaction within and around the partially degraded scaffolds.

*Week 2*

*Abraded Wounds*

*(1) EmDerm-C*

There was no evidence of inflammation within the scaffold or around the scaffold. There were many vascular structures present containing red blood cells within the lumen, suggesting active blood flow within the scaffold and integration with surrounding tissue. There was also significant regeneration of the dermal layer with deposition of collagen and part regeneration of the paniculli carnosus. See Figure 35. There was increased vascularization of the wound site

as shown by the arrows and scaffolds have been remodelled/integrated into the wounds.

*(2) EmDerm-CF*

There was evidence of residual inflammation in the tissue around the scaffold. There is evidence of vascularization of the scaffold, with some collagen matrix deposition and remodelling. There was also regeneration of the dermal layer but no evidence of regeneration of the paniculli carnosus layer. See Figure 36. There was increased vascularization of the wound site as shown by the arrows and scaffolds have been remodelled/integrated into the wounds. However, there was some residual inflammatory response and increased deposition of ECM in the dermal layer.

*(3) EmDerm-F*

There was minimal inflammation in the tissue surrounding the scaffold. There was evidence of vascularization of the scaffold with increased collagen matrix deposition and scaffold remodelling. The dermal layer has almost completely healed, however there was no regeneration of the paniculli carnosus, which is similar to the EmDerm-CF scaffold. See Figure 37. There was increased vascularization of the wound (as shown by arrows) and significant deposition of ECM material within the wound site and dermal layer.

*Non-abraded Wounds*

*(1) EmDerm-C*

At Week 2, the non-abraded wounds with EmDerm-C implanted showed minimal inflammatory response within and surrounding the scaffold. There was excellent vascularization of the scaffold and matrix remodelling as well. See Figure 38. There was minimal inflammatory response and most of the scaffold has been remodelled with ingrowth of vascular structure (see arrows).

*(2) EmDerm-CF*

EmDerm-CF scaffolds in the non-abraded wounds also showed minimal inflammatory response around and within the scaffolds. There was formation of vasculature within and around the scaffolds and much of the original scaffold had been degraded and remodelled into connective tissue. See Figure 39. There was minimal inflammatory response and most of the scaffold had been remodelled with ingrowth of vascular structure (see arrows).

*(3) EmDerm-F*

EmDerm-F scaffolds demonstrated the least degree of vascularization and some residual inflammatory response. The scaffold itself was mostly entirely degraded. However, there was also greater deposition of extracellular matrix compared to the other two scaffolds, indicating some degree of fibrosis present. The new matrix laid down appeared to be disorganized fibrils with no obvious form or structure within it. See Figure 40. There was minimal inflammatory response and most of the scaffold has been remodelled with ingrowth of vascular structure (see arrows). There was significant deposition of ECM as well.

*Quantification of vascularization*

The mean number of vessels in EmDerm-C scaffolds (mean 6.5 $\pm$ 2.3) was significantly higher compared to the EmDerm-F scaffolds in the abraded and non-abraded wound model (mean 3.7 $\pm$ 1.6 and 1.9 $\pm$ 1.6 respectively). EmDerm-CF scaffolds resulted in intermediate degree of vascularization compared to EmDerm-C and EmDerm-F in both the abraded and non-abraded wounds (mean 4.7 $\pm$ 2.0 and 3.2 $\pm$ 1.0 respectively). However, this result is not statistically significant. See Figure 41. In both the abraded and non-abraded wounds, there was a higher mean number of vascular structures in wounds treated with EmDerm-C.

*Quantification of local inflammatory response*

The inflammatory grade was lowest in EmDerm-C (mean 1.5 $\pm$ 0.48) scaffolds compared to EmDerm-CF (mean 3.1 $\pm$ 0.81) and EmDerm-F (mean 3.3 $\pm$ 0.67) scaffolds in the abraded wound model. The mean inflammatory grade was also lowest in EmDerm-C (mean 1.5 $\pm$ 0.52) compared to EmDerm-CF (mean 3.1 $\pm$ 0.74) and EmDerm-F (mean 3.4 $\pm$ 0.7). There was no significant difference in inflammatory response between EmDerm-CF and EmDerm-F scaffolds.

See Figure 42. In both the abraded and non-abraded wounds, there was a higher degree of inflammation in the EmDerm-F scaffolds, followed by EmDerm-CF, and then EmDerm-C.

The non-abraded wound model was designed to test for the presence of any localized reaction to scaffold implantation itself. The abraded wound model simulates a partial thickness skin graft where part of the lower dermis was removed, and the scaffold was implanted to test for wound healing and revascularization of the wound.

A time point of two weeks was selected to examine the effect of the scaffold on early revascularization. This duration of time would allow for the inflammatory response from the wound creation and initial injury to settle down and formation of a neodermis. Ideally, wound beds should be ready for epidermal engraftment by Day 14, which was another reason for selecting this time point. On Day 7, all three EmDerm scaffolds were noted to have minimal angiogenesis and vasculogenesis and had varying inflammatory responses. Therefore, a longer time-point was needed to assess vascular ingrowth and resolution of inflammation.

A differential effect was noted across all three EmDerm scaffolds over the two-week period as well. EmDerm-C scaffolds had the highest number of vascular structures on Day 14 compared to other EmDerm scaffolds. This is comparable to Integra.

Overall, EmDerm-C scaffolds appear to be the most biocompatible with the least inflammatory response and highest rate of vascularization of the scaffold. This may be due to the fact that collagen is an abundantly found extracellular matrix which allows for good cellular attachment and migration. This could also be due to the added nanofibrillar surface of EmDerm-C scaffolds, resulting in greater surface area for cell adhesion and proliferation.

EmDerm-F scaffolds appear to shower lower biocompatibility, with the greatest inflammatory response within 7 days and lowest rate of vascularization at 14 days.

Although there was no obvious systemic inflammatory response in the animals to the scaffolds, there was a notable local inflammatory effect at Day 7 post-implantation in both the abraded and non-abraded wound models. This is rather unsurprising as inflammation is part of the wound healing response. Nevertheless, the inflammatory response appears to resolve by Day 14. This is in contrast to Matriderm and Integra where the inflammatory response persists beyond Day 14 in a full-thickness defect rat wound healing model. The inflammatory phase is followed by a rather rapid vascularization and remodelling of the neodermis by Day 14. Also, extracellular matrix deposition was more pronounced in EmDerm-F compared to the other two scaffolds.

The degree of local inflammatory response and vascularization was not significantly affected by type of wound (abraded vs non-abraded) but rather, the type of material the scaffolds were made of. The release of fibrin degradation products in EmDerm-CF and EmDerm-F may have resulted in a greater inflammatory response compared to EmDerm-C scaffolds.

Overall, the degree of inflammation noted for EmDerm scaffolds was minimal and transient. Most of the inflammatory cells (e.g. polymorphonuclear cells) were seen in Day 7 biopsies but were resolved by Day 14. The degree of inflammatory response correlated somewhat to the endotoxin levels and enzymatic stability of the EmDerm scaffolds with EmDerm-C having the lowest endotoxin levels and slowest degradation rate, while EmDerm-F has the highest endotoxin levels and fastest degradation rate.

There were notable differences in extracellular matrix remodelling between the various EmDerm scaffolds when implanted *in vivo,* despite using the same emulsion-templating manufacturing methods. This suggests that the type of material used to create the scaffolds influences the cell behaviour in the acute wound environment.

In conclusion, these *in vivo* results indicate that EmDerm scaffolds are biocompatible and promote wound healing and vascularization over a two-week period.

Features, integers, characteristics, compounds, chemical moieties or groups described in conjunction with a particular aspect, embodiment or example of the invention are to be understood to be applicable to any other aspect, embodiment or example described herein unless incompatible therewith. The invention is not restricted to the details of any foregoing embodiments.

## Claims

**1.** A method of preparing a porous protein scaffold for supporting the growth of biological tissue, said method comprising:

providing an oil-in water emulsion comprising oil droplets dispersed in a continuous phase comprising a pH-buffered aqueous protein solution, wherein the oil-in-water emulsion comprises a non-ionic surfactant or a mixture of non-ionic surfactants, wherein the non-ionic surfactant is in an amount of 0.01 to 10 volume % of the total volume of the oil phase in the oil-in-water emulsion, wherein a HLB of the non-ionic surfactant or the surfactant mixture is 10 to 16;
gelling the protein around the oil droplets;
removing the oil droplets from the continuous phase after formation of a scaffold; and

incubating the scaffold with an excipient solution.

2. The method of claim 1, wherein the protein is a gellable protein selected from at least one of collagen, fibrinogen, fibronectin, laminin and elastin.

3. The method of claim 1 or claim 2, wherein the aqueous protein solution comprises a native protein.

4. The method of any preceding claim, wherein the non-ionic surfactant or a mixture of non-ionic surfactants has an HLB of between 11 and 14.

5. The method of any preceding claim, wherein non-ionic surfactant is present in an amount of 0.05 to 1 volume % of the total volume of oil in the oil-in-water emulsion.

6. The method of any preceding claim, wherein the excipient solution comprises a watersoluble organic hydrophilic excipient, which is a polyol or a polymer selected from polyvinyl alcohol (PVA), polyethylene glycol (PEG), polyoxyethylene-polyoxypropylene glycol block co-polymer, polyvinylpyrrolidone (PVP) and polyethylene glycol-polyvinyl alcohol copolymer.

7. The method of any preceding claim, wherein the non-ionic surfactant comprises an ester of a polyol.

8. The method of any preceding claim, wherein the oil-in-water emulsion comprises an oil selected from a light mineral oil, decane and a short chain hydrocarbon oil having a hydrocarbon chain length in the range of C8-C18.

9. The method of any preceding claim, wherein the emulsion comprises oil droplets having a diameter in the range of between 10 to 500 microns.

10. The method of any preceding claim, wherein the continuous aqueous phase additionally comprises a biological material.

11. A porous protein scaffold obtainable by a method as claimed in any one of the preceding claims using a gellable protein selected from at least one of collagen, fibrinogen, fibronectin, laminin and elastin, wherein the porous protein scaffold comprises an interconnected fibrous structure of proteins and has an average pore size in the range of 80 to 200 microns.

12. A porous protein scaffold for use in repairing tissue or for tissue regeneration in a human or an animal, wherein the porous protein scaffold is as defined in claim 11.

13. An *in vitro* method of manufacturing or engineering a tissue, wherein the method comprises applying cells to the porous protein scaffold according to claim 11.

14. A tissue-engineered construct obtainable from the method of claim 13 and/or which comprises cells or a tissue supported on the porous protein scaffold as defined in claim 11.

15. A tissue-engineered construct for use in the treatment of the human or animal body, wherein the tissue-engineered construct is as defined in claim 14.

**Patentansprüche**

1. Verfahren zum Herstellen eines porösen Proteingerüsts zum Unterstützen des Wachstums von biologischem Gewebe, das Verfahren umfassend:

Bereitstellen einer Öl-in-Wasser-Emulsion, umfassend Öltröpfchen, die in einer kontinuierlichen Phase dispergiert sind, umfassend eine pH-gepufferte wässrige Proteinlösung, wobei die Öl-in-Wasser-Emulsion ein nichtionisches Tensid oder ein Gemisch aus nicht-ionischen Tensiden umfasst, wobei das nicht-ionische Tensid in einer Menge von 0,01 bis 10 Volumen-% des Gesamtvolumens der Ölphase in der Öl-in-Wasser-Emulsion ist, wobei ein HLB-Wert des nicht-ionischen Tensids oder des Tensidgemischs 10 bis 16 ist;
Gelieren des Proteins um die Öltröpfchen,

Entfernen der Öltröpfchen aus der kontinuierlichen Phase nach Bildung eines Gerüsts; und Inkubieren des Gerüsts mit einer Hilfsstofflösung.

**2.** Verfahren nach Anspruch 1, wobei das Protein ein gelierbares Protein ist, das ausgewählt ist aus mindestens einem von Kollagen, Fibrinogen, Fibronektin, Laminin und Elastin.

**3.** Verfahren nach Anspruch 1 oder Anspruch 2, wobei die wässrige Proteinlösung ein natives Protein umfasst.

**4.** Verfahren nach einem der vorherigen Ansprüche, wobei das nicht-ionische Tensid oder ein Gemisch nicht-ionischer Tenside einen HLB-Wert zwischen 11 und 14 aufweist.

**5.** Verfahren nach einem der vorherigen Ansprüche, wobei das nicht-ionische Tensid in einer Menge von 0,05 bis 1 Volumen-% des Gesamtvolumens des Öls in der Öl-in-Wasser-Emulsion vorhanden ist.

**6.** Verfahren nach einem der vorherigen Ansprüche, wobei die Hilfsstofflösung einen wasserlöslichen organischen hydrophilen Hilfsstoff umfasst, der ein Polyol oder ein Polymer ist, das ausgewählt ist aus Polyvinylalkohol (PVA), Polyethylenglykol (PEG), Polyoxyethylen-Polyoxypropylenglykol-Blockcopolymer, Polyvinylpyrrolidon (PVP) und Polyethylenglykol-Polyvinylalkohol-Copolymer.

**7.** Verfahren nach einem der vorherigen Ansprüche, wobei das nicht-ionische Tensid einen Ester eines Polyols umfasst.

**8.** Verfahren nach einem der vorherigen Ansprüche, wobei die Öl-in-Wasser-Emulsion ein Öl umfasst, das ausgewählt ist aus einem leichten Mineralöl, Decan und einem kurzkettigen Kohlenwasserstofföl mit einer Kohlenwasserstoffkettenlänge in dem Bereich von C8-C18.

**9.** Verfahren nach einem der vorherigen Ansprüche, wobei die Emulsion Öltröpfchen umfasst, die einen Durchmesser in dem Bereich zwischen 10 und 500 Mikrometer aufweisen.

**10.** Verfahren nach einem der vorherigen Ansprüche, wobei die kontinuierliche wässrige Phase zusätzlich ein biologisches Material umfasst.

**11.** Poröses Proteingerüst, das durch ein Verfahren nach einem der vorherigen Ansprüche unter Verwendung eines gelierbaren Proteins, ausgewählt aus mindestens einem von Kollagen, Fibrinogen, Fibronektin, Laminin und Elastin, erlangt werden kann, wobei das poröse Proteingerüst eine miteinander verbundene faserige Struktur von Proteinen umfasst und eine durchschnittliche Porengröße in dem Bereich von 80 bis 200 Mikrometer aufweist.

**12.** Poröses Proteingerüst zur Verwendung beim Reparieren von Gewebe oder zur Geweberegeneration bei einem Menschen oder einem Tier, wobei das poröse Proteingerüst wie definiert in Anspruch 11 ist.

**13.** *In vitro*-Verfahren zum Herstellen oder Modifizieren eines Gewebes, wobei das Verfahren ein Anwenden von Zellen auf das poröse Proteingerüst nach Anspruch 11 umfasst.

**14.** Gewebemodifiziertes Konstrukt, das von dem Verfahren von Anspruchs 13 erlangt werden kann und/oder das Zellen oder ein Gewebe umfasst, das auf dem porösen Proteingerüst wie definiert in Anspruch 11 getragen wird.

**15.** Gewebemodifiziertes Konstrukt zur Verwendung bei der Behandlung des menschlichen oder tierischen Körpers, wobei das gewebemodifizierte Konstrukt wie definiert in Anspruch 14 ist.

## Revendications

**1.** Procédé de préparation d’un échafaudage protéique poreux destiné à favoriser la croissance d’un tissu biologique, ledit procédé comprenant :

la production d’une émulsion d’huile dans l’eau comprenant des gouttelettes d’huile dispersées dans une phase continue comprenant une solution protéique aqueuse à pH tamponné, ladite émulsion d’huile dans l’eau comprenant un agent tensioactif non ionique ou un mélange d’agents tensioactifs non ioniques, ledit agent tensioactif non ionique étant présent en une quantité de 0,01 à 10 % en volume du volume total de la phase huileuse dans

l'émulsion d'huile dans l'eau, le HLB de l'agent tensioactif non ionique ou du mélange d'agents tensioactifs valant de 10 à 16 ;
la gélification de la protéine autour des gouttelettes d'huile ;
l'élimination des gouttelettes d'huile de la phase continue après formation d'un échafaudage ; et
l'incubation de l'échafaudage avec une solution d'excipient.

**2.** Procédé selon la revendication 1, ladite protéine étant une protéine gélifiable choisie parmi au moins une protéine parmi le collagène, le fibrinogène, la fibronectine, la laminine et l'élastine.

**3.** Procédé selon la revendication 1 ou la revendication 2, ladite solution protéique aqueuse comprenant une protéine native.

**4.** Procédé selon l'une quelconque des revendications précédentes, ledit agent tensioactif non ionique ou un mélange d'agents tensioactifs non ioniques comportant un HLB compris entre 11 et 14.

**5.** Procédé selon l'une quelconque des revendications précédentes, ledit agent tensioactif non ionique étant présent en une quantité de 0,05 à 1 % en volume du volume total de l'huile dans l'émulsion d'huile dans l'eau.

**6.** Procédé selon l'une quelconque des revendications précédentes, ladite solution d'excipient comprenant un excipient hydrophile organique soluble dans l'eau, qui est un polyol ou un polymère choisi parmi l'alcool polyvinylique (PVA), le polyéthylène glycol (PEG), un copolymère séquencé polyoxyéthylène-polyoxypropylène glycol, la polyvinylpyrrolidone (PVP) et un copolymère de polyéthylène glycol et d'alcool polyvinylique.

**7.** Procédé selon l'une quelconque des revendications précédentes, ledit agent tensioactif non ionique comprenant un ester d'un polyol.

**8.** Procédé selon l'une quelconque des revendications précédentes, ladite émulsion d'huile dans l'eau comprenant une huile choisie parmi une huile minérale légère, le décane et une huile hydrocarbonée à chaîne courte comportant une longueur de chaîne hydrocarbonée dans la plage de C8 à C18.

**9.** Procédé selon l'une quelconque des revendications précédentes, ladite émulsion comprenant des gouttelettes d'huile comportant un diamètre compris dans la plage entre 10 à 500 microns.

**10.** Procédé selon l'une quelconque des revendications précédentes, ladite phase aqueuse continue comprenant de plus un matériau biologique.

**11.** Echafaudage protéique poreux pouvant être obtenu par un procédé selon l'une quelconque des revendications précédentes utilisant une protéine gélifiable choisie parmi au moins une protéine parmi le collagène, le fibrinogène, la fibronectine, la laminine et l'élastine, ledit échafaudage protéique poreux comprenant une structure fibreuse interconnectée de protéines et comportant une taille moyenne de pores comprise dans la plage de 80 à 200 microns.

**12.** Echafaudage protéique poreux destiné à être utilisé dans la réparation d'un tissu ou destiné à la régénération de tissus chez un humain ou un animal, ledit échafaudage protéique poreux étant tel que défini dans la revendication 11.

**13.** Procédé *in vitro* de fabrication ou d'ingénierie d'un tissu, ledit procédé comprenant l'application de cellules sur l'échafaudage protéique poreux selon la revendication 11.

**14.** Construction produite par ingénierie tissulaire pouvant être obtenue à partir du procédé selon la revendication 13 et/ou qui comprend des cellules ou un tissu soutenu sur l'échafaudage protéique poreux tel que défini dans la revendication 11.

**15.** Construction produite par ingénierie tissulaire destinée à être utilisée dans le traitement du corps humain ou animal, ladite construction produite par ingénierie tissulaire étant telle que définie dans la revendication 14.

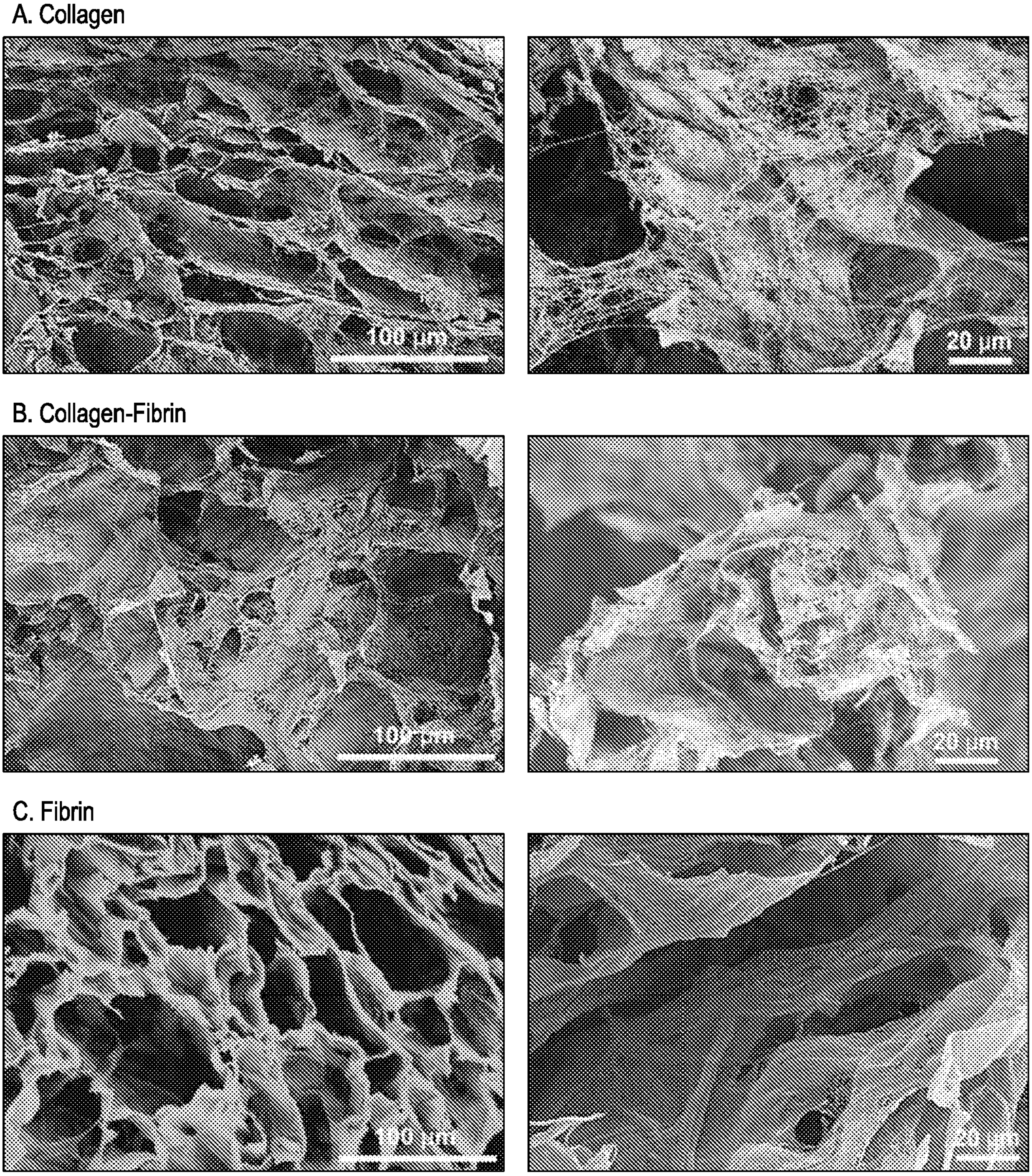
A. Collagen
100 µm
20 µm
B. Collagen-Fibrin
100 µm
20 µm
C. Fibrin
100 µm
20 µm

FIG. 1

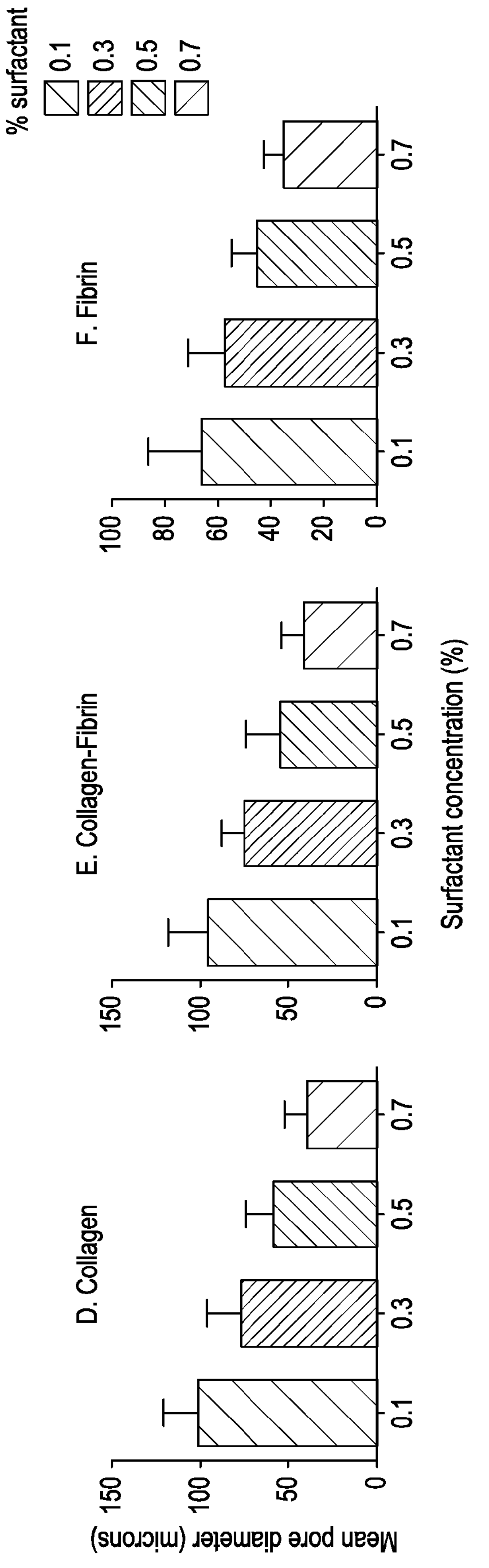
% surfactant
0.1
0.3
0.5
0.7
D. Collagen
E. Collagen-Fibrin
F. Fibrin
Mean pore diameter (microns)
150
100
50
0
150
100
50
0
100
80
60
40
20
0
0.1
0.3
0.5
0.7
0.1
0.3
0.5
0.7
0.1
0.3
0.5
0.7
Surfactant concentration (%)

*FIG. 1 Cont'd*

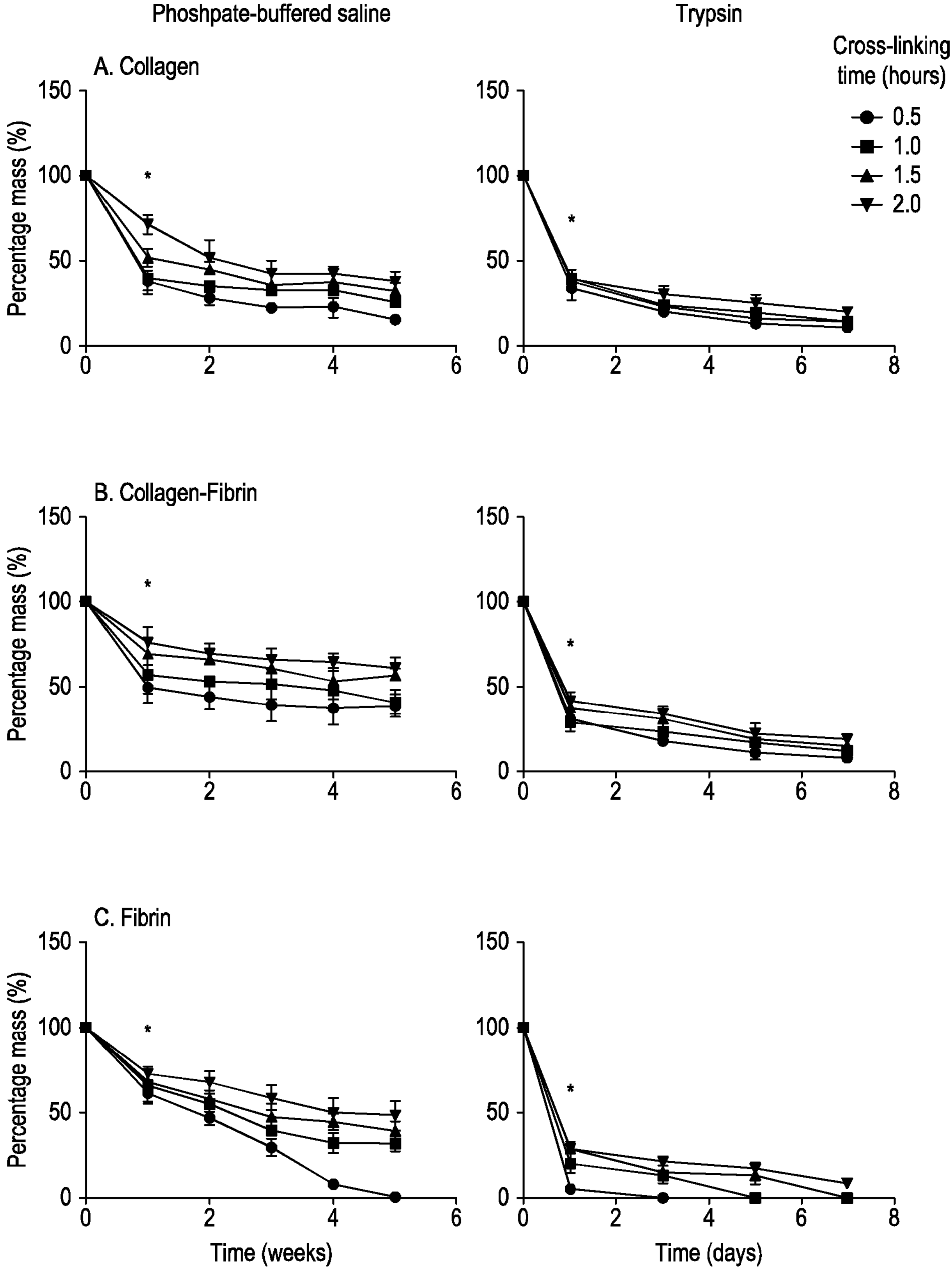
Phoshpate-buffered saline
Trypsin
Cross-linking time (hours)
0.5
1.0
1.5
2.0
A. Collagen
B. Collagen-Fibrin
C. Fibrin
Percentage mass (%)
150
100
50
0
0
2
4
6
8
*
Time (weeks)
Time (days)

*FIG. 2*

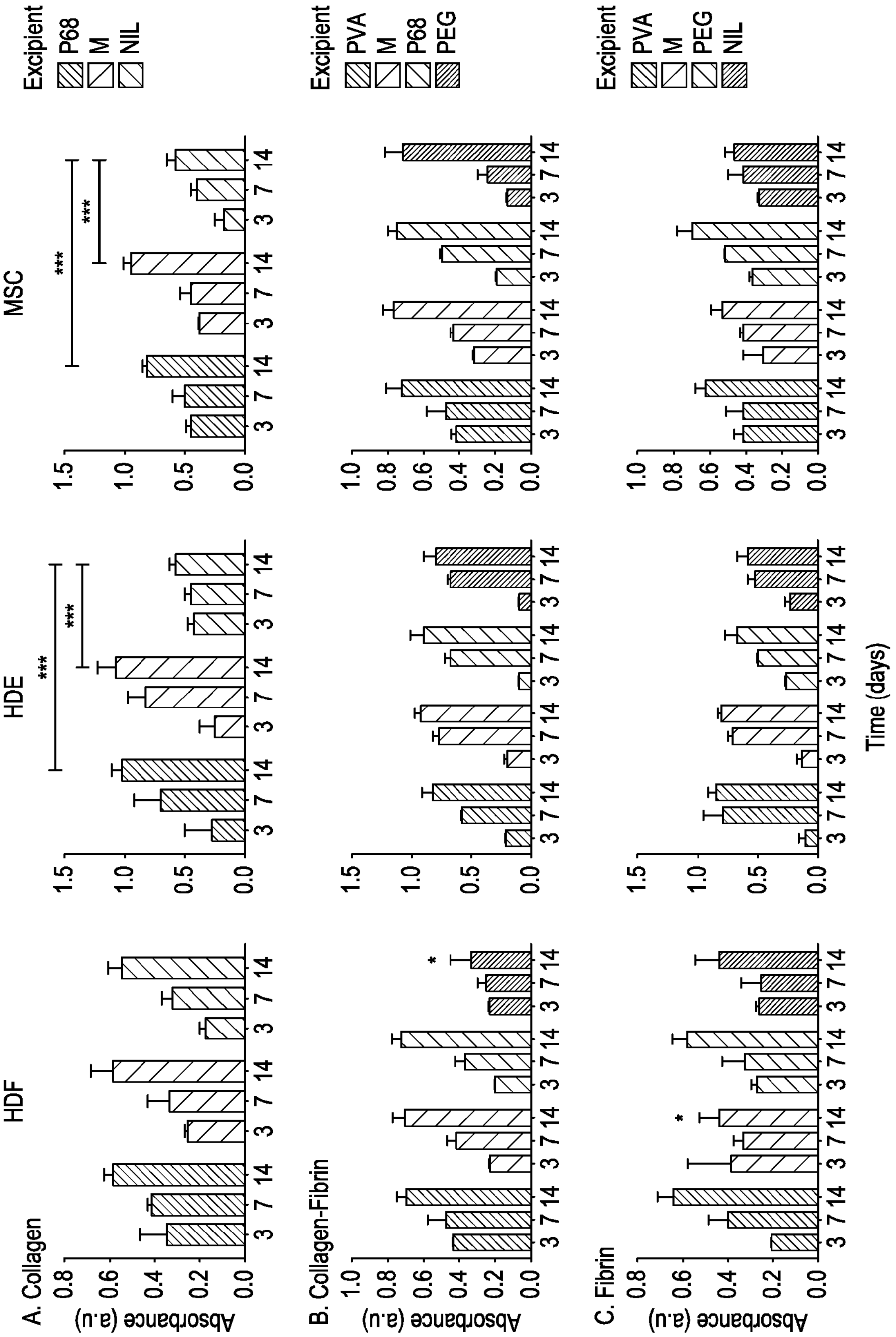
HDF
HDE
MSC
A. Collagen
B. Collagen-Fibrin
C. Fibrin
Absorbance (a.u)
Excipient
P68
M
NIL
PVA
PEG
***
*
Time (days)

*FIG. 3*

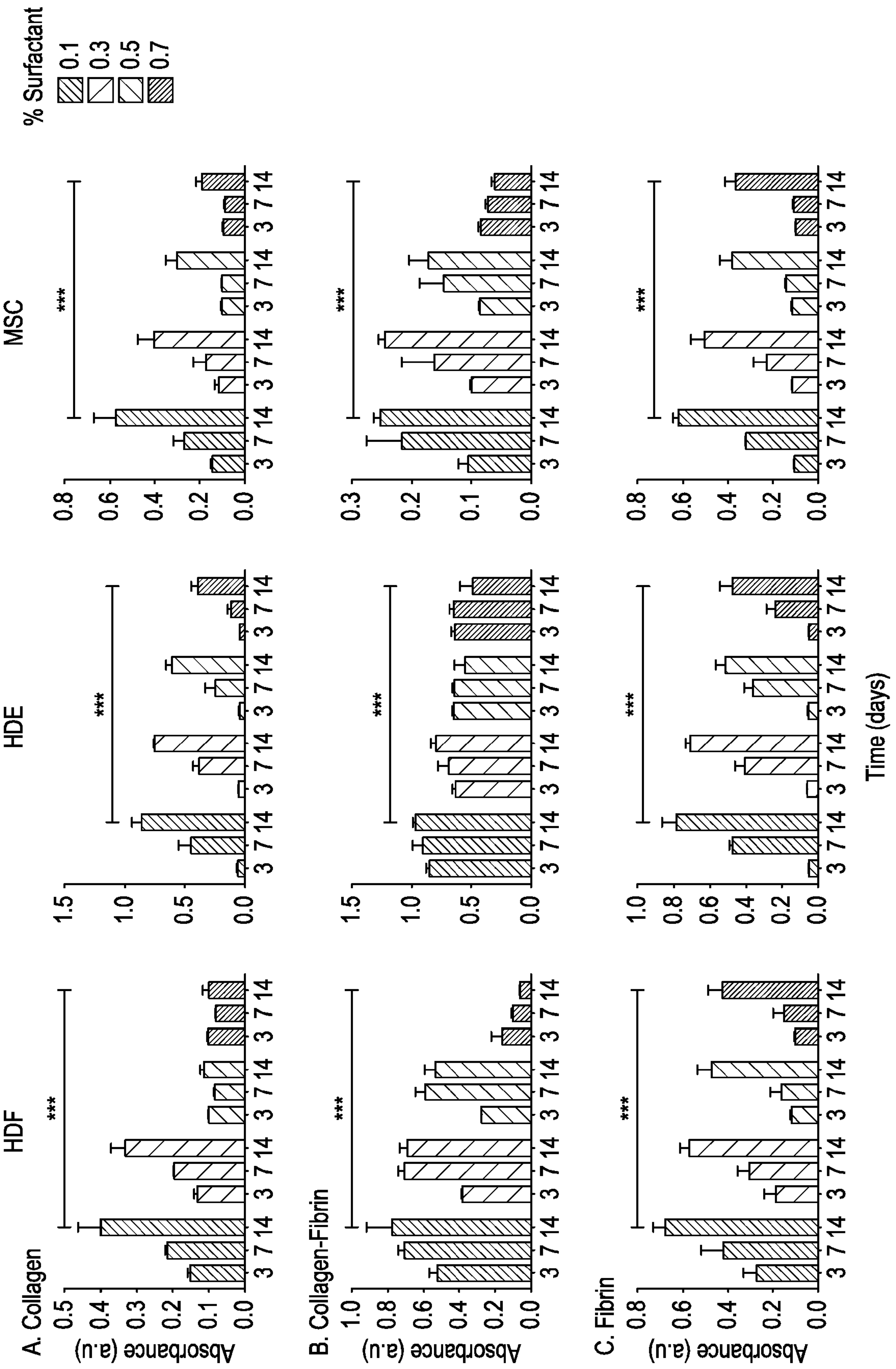
A. Collagen
B. Collagen-Fibrin
C. Fibrin
HDF
HDE
MSC
Absorbance (a.u)
Time (days)
% Surfactant
0.1
0.3
0.5
0.7
***

*FIG. 4*

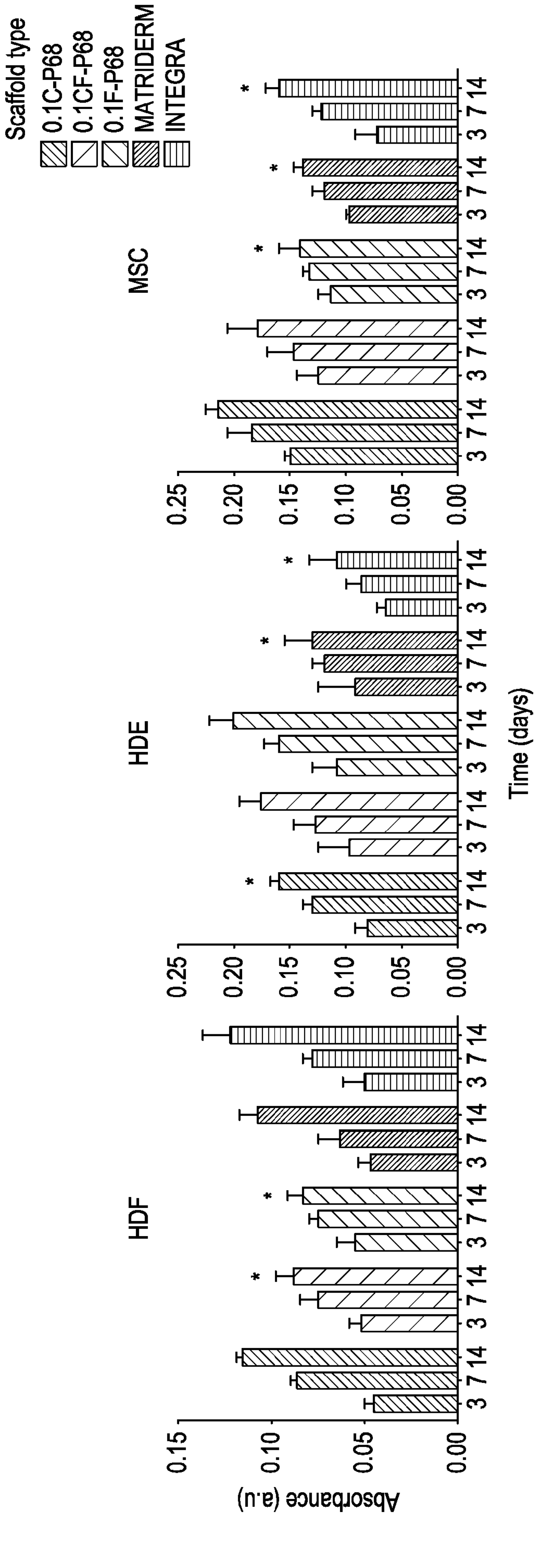

Scaffold type
0.1C-P68
0.1CF-P68
0.1F-P68
MATRIDERM
INTEGRA
HDF
HDE
MSC
Absorbance (a.u)
Time (days)
0.00
0.05
0.10
0.15
0.20
0.25
3 7 14
*


FIG. 5

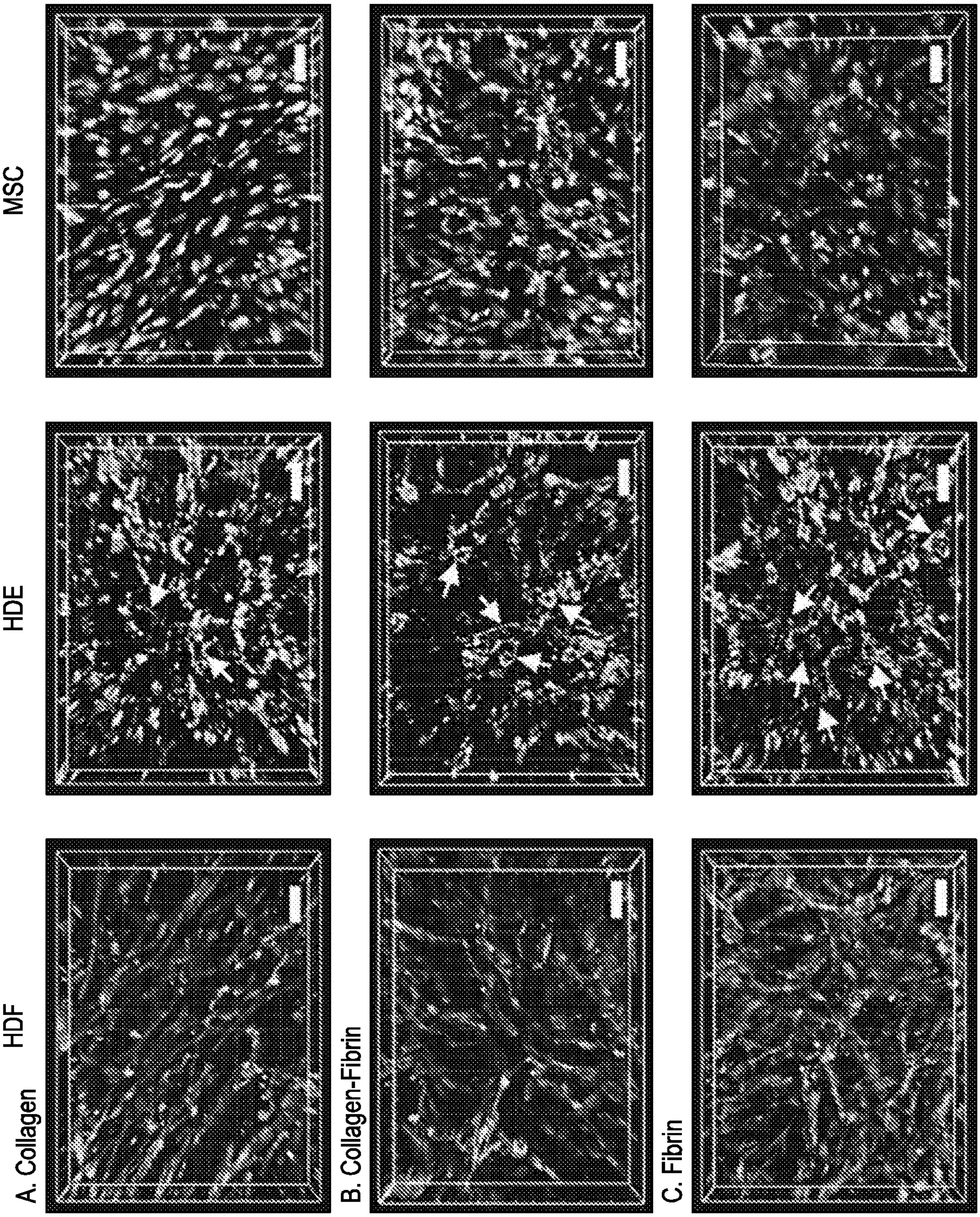


FIG. 6

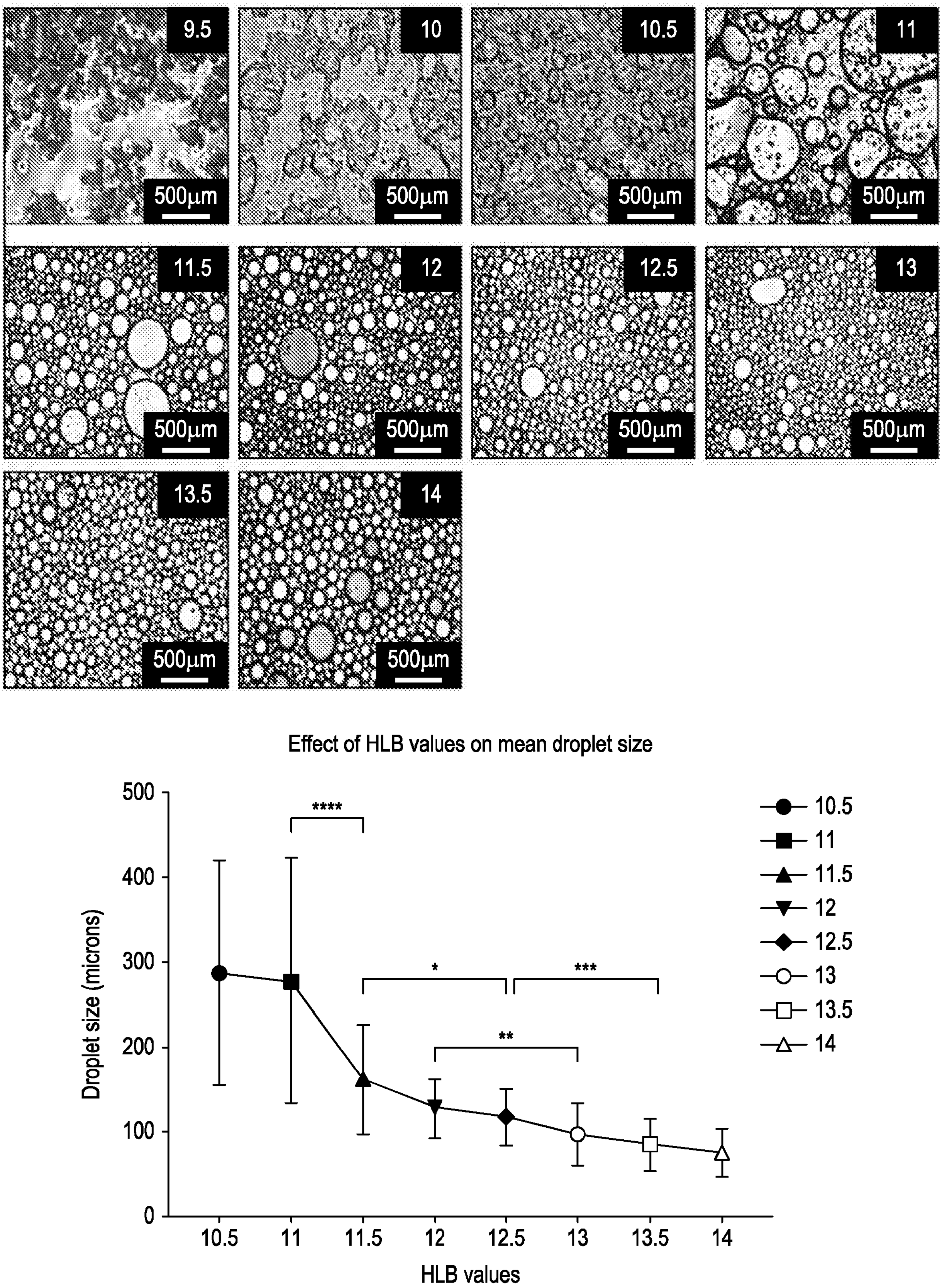
9.5
10
10.5
11
11.5
12
12.5
13
13.5
14
500μm
Effect of HLB values on mean droplet size
Droplet size (microns)
500
400
300
200
100
0
10.5
11
11.5
12
12.5
13
13.5
14
HLB values
****
*
***
**

FIG. 7

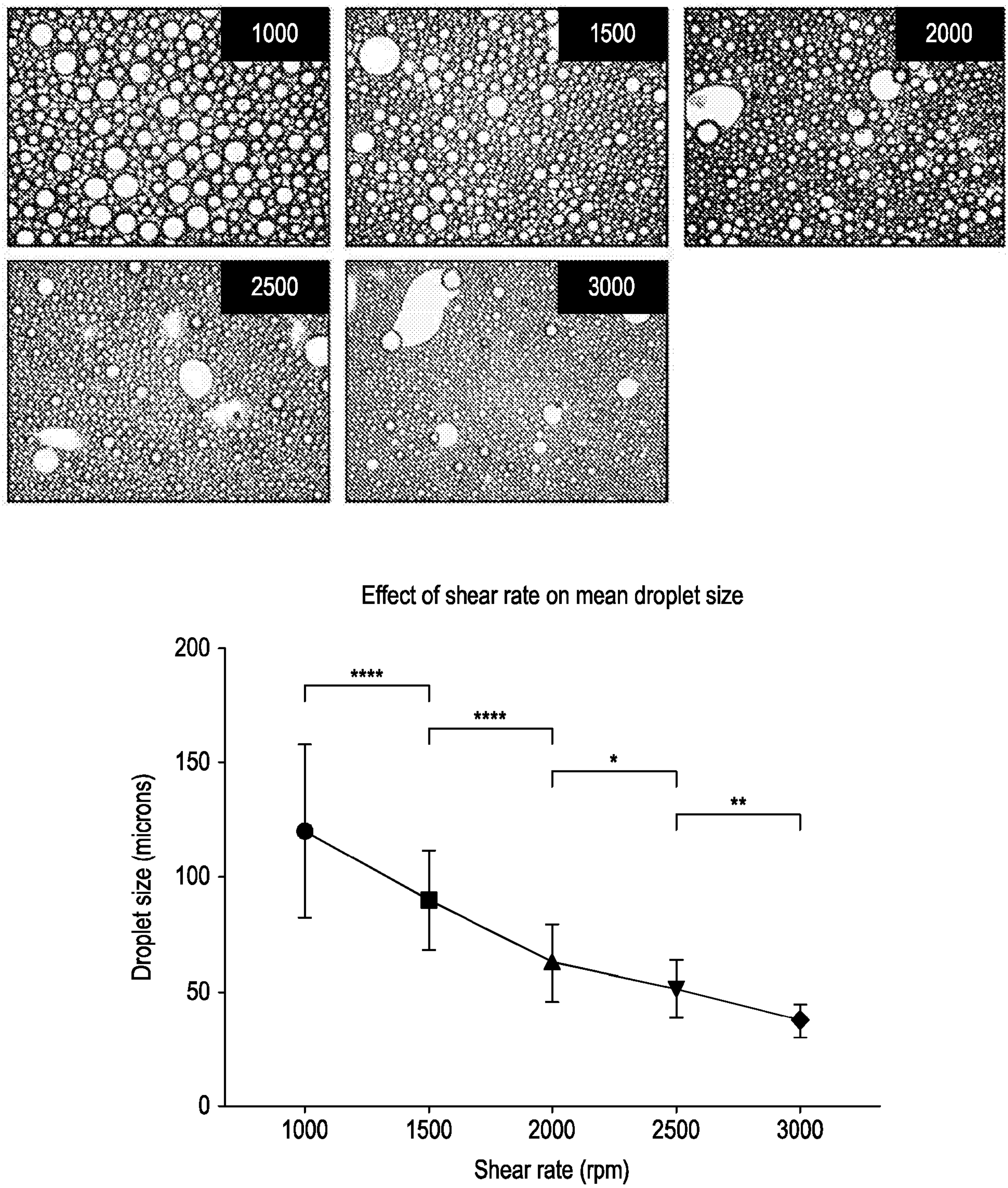
1000
1500
2000
2500
3000
Effect of shear rate on mean droplet size
200
150
100
50
0
****
****
*
**
Droplet size (microns)
1000
1500
2000
2500
3000
Shear rate (rpm)

FIG. 8A

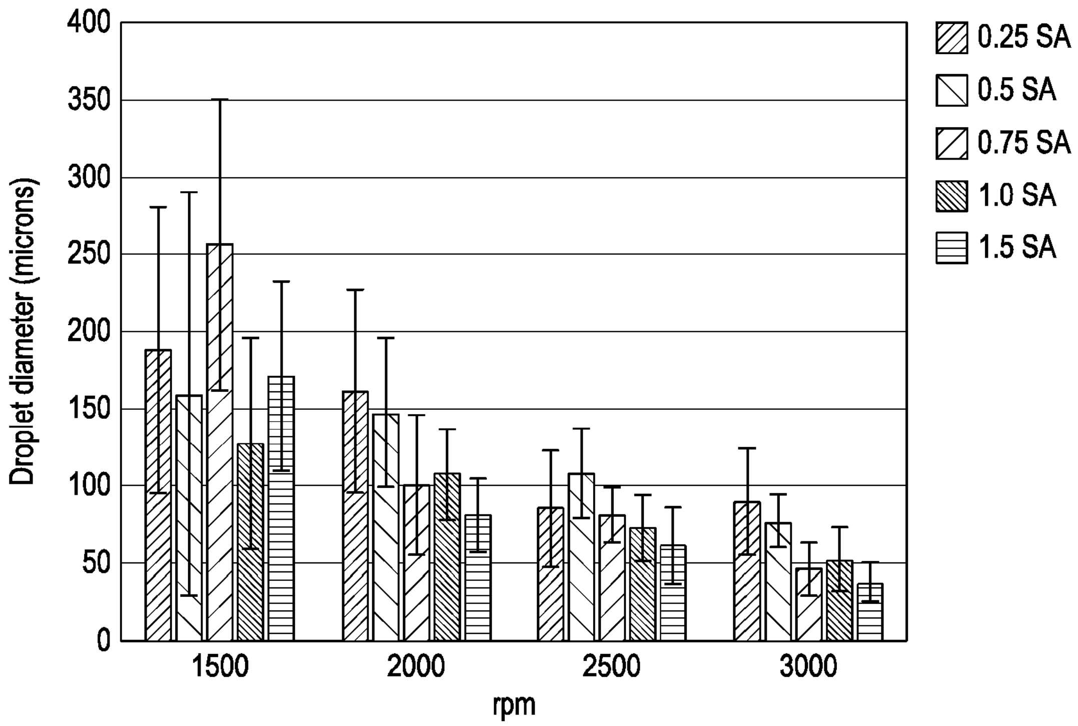
400
350
300
250
200
150
100
50
0
Droplet diameter (microns)
1500
2000
2500
3000
rpm
0.25 SA
0.5 SA
0.75 SA
1.0 SA
1.5 SA

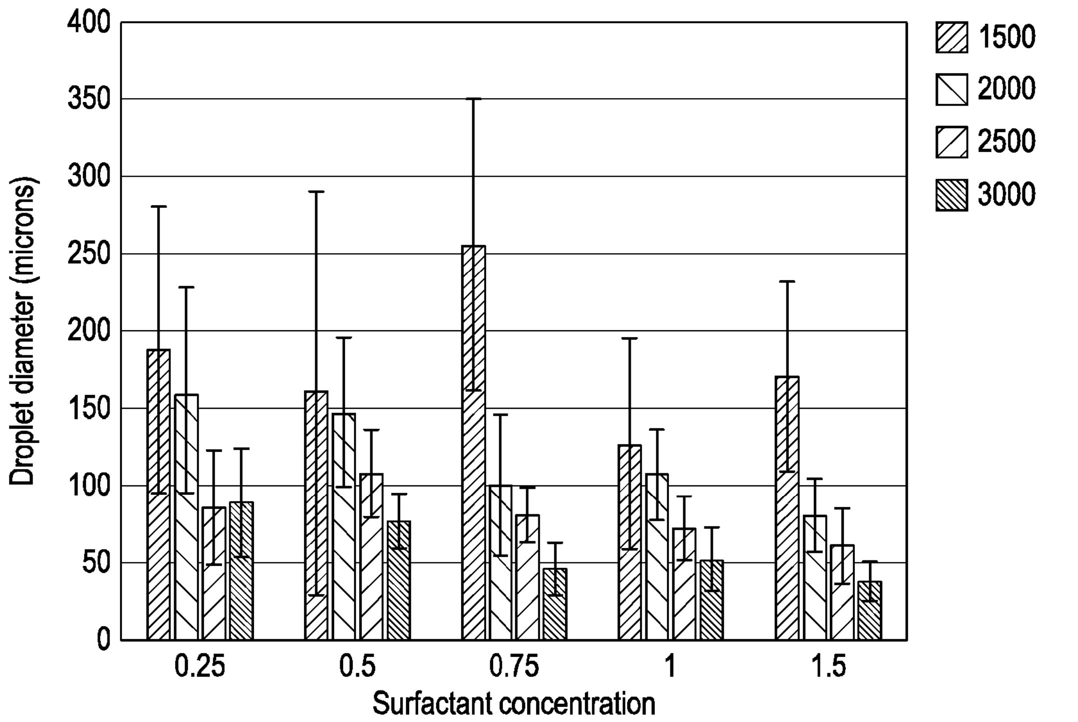
400
350
300
250
200
150
100
50
0
Droplet diameter (microns)
0.25
0.5
0.75
1
1.5
Surfactant concentration
1500
2000
2500
3000

FIG. 8B

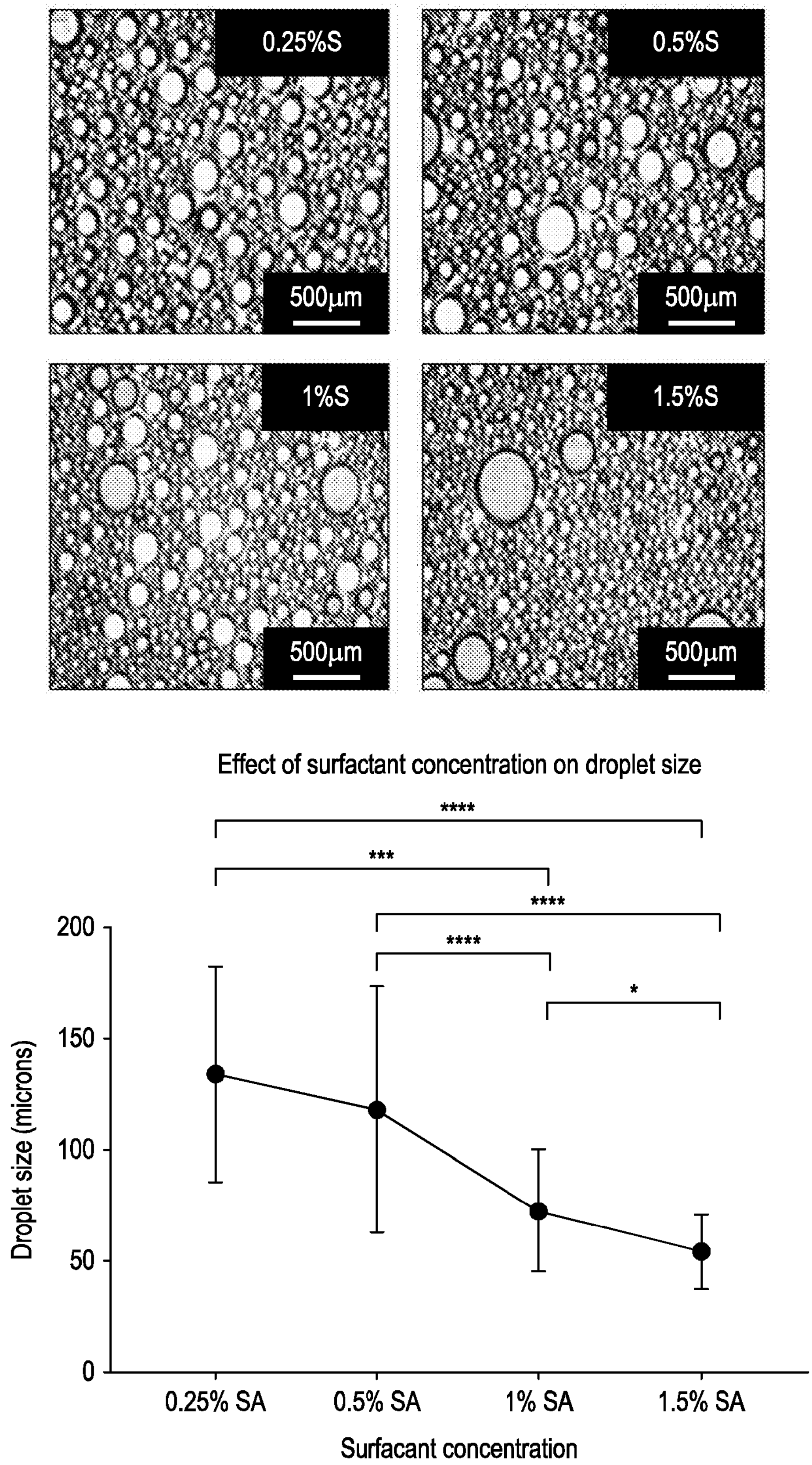
0.25%S
500μm
0.5%S
500μm
1%S
500μm
1.5%S
500μm
Effect of surfactant concentration on droplet size
****
***
****
****
*
200
150
100
50
0
Droplet size (microns)
0.25% SA
0.5% SA
1% SA
1.5% SA
Surfactant concentration

FIG. 9

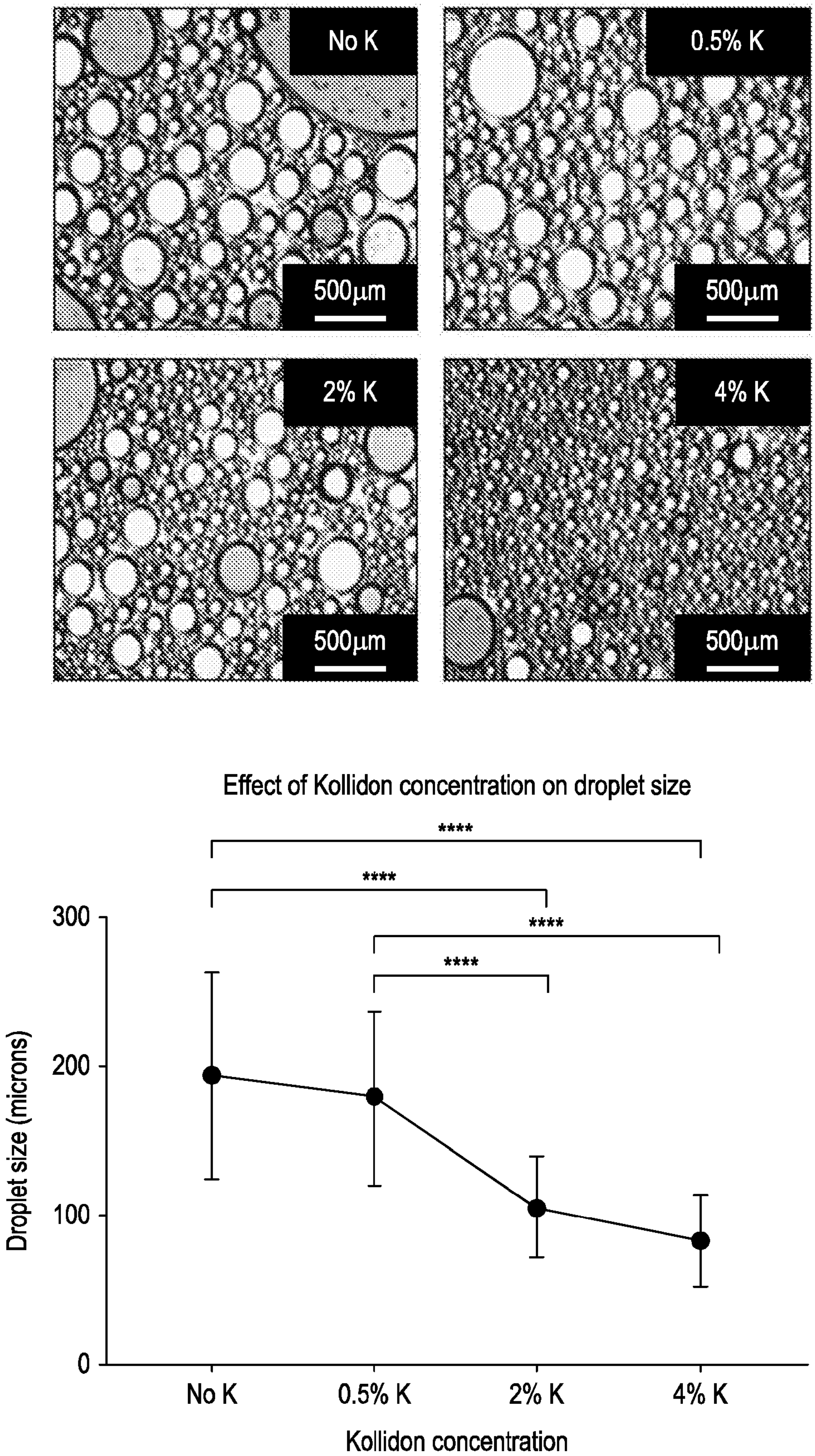
No K
500μm
0.5% K
500μm
2% K
500μm
4% K
500μm
Effect of Kollidon concentration on droplet size
****
****
****
****
300
200
100
0
Droplet size (microns)
No K
0.5% K
2% K
4% K
Kollidon concentration

*FIG. 10*

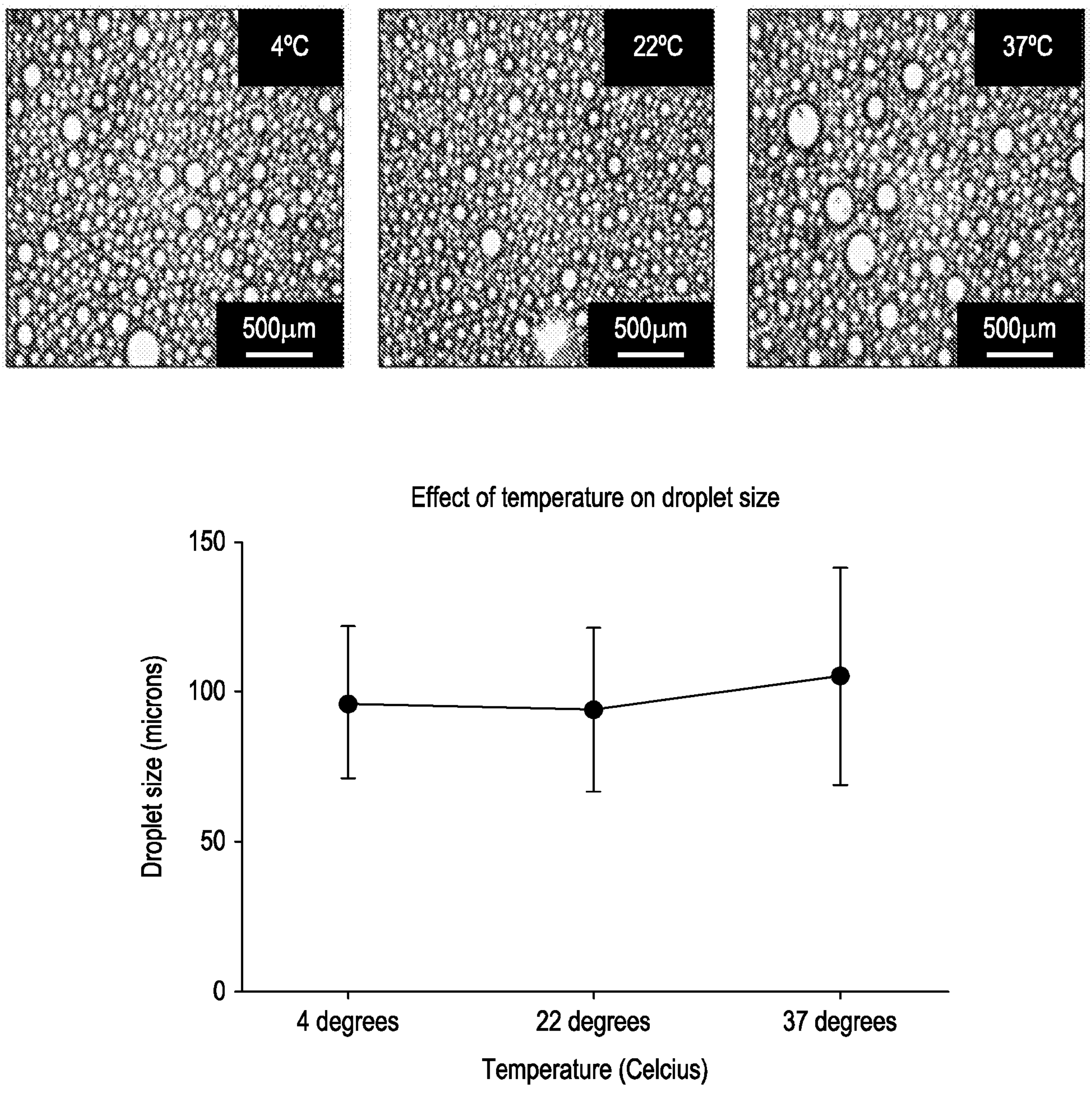
4°C
22°C
37°C
500µm
500µm
500µm
Effect of temperature on droplet size
Droplet size (microns)
150
100
50
0
4 degrees
22 degrees
37 degrees
Temperature (Celcius)

FIG. 11

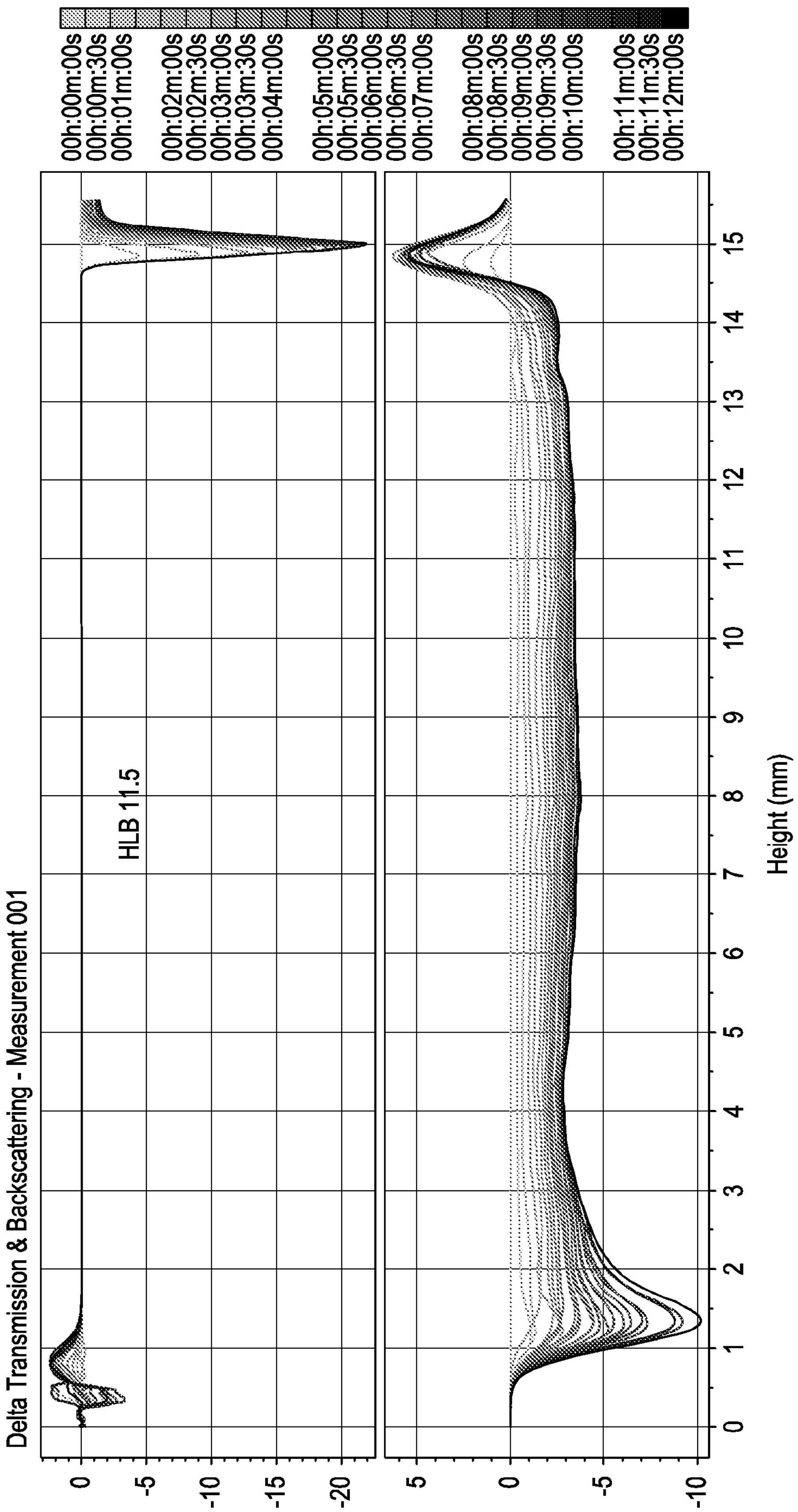
Delta Transmission & Backscattering - Measurement 001
HLB 11.5
ΔT(%)
ΔBS(%)
Height (mm)
0
-5
-10
-15
-20
5
0
-5
-10
0 1 2 3 4 5 6 7 8 9 10 11 12 13 14 15
00h:00m:00s
00h:00m:30s
00h:01m:00s
00h:02m:00s
00h:02m:30s
00h:03m:00s
00h:03m:30s
00h:04m:00s
00h:05m:00s
00h:05m:30s
00h:06m:00s
00h:06m:30s
00h:07m:00s
00h:08m:00s
00h:08m:30s
00h:09m:00s
00h:09m:30s
00h:10m:00s
00h:11m:00s
00h:11m:30s
00h:12m:00s

FIG. 12A

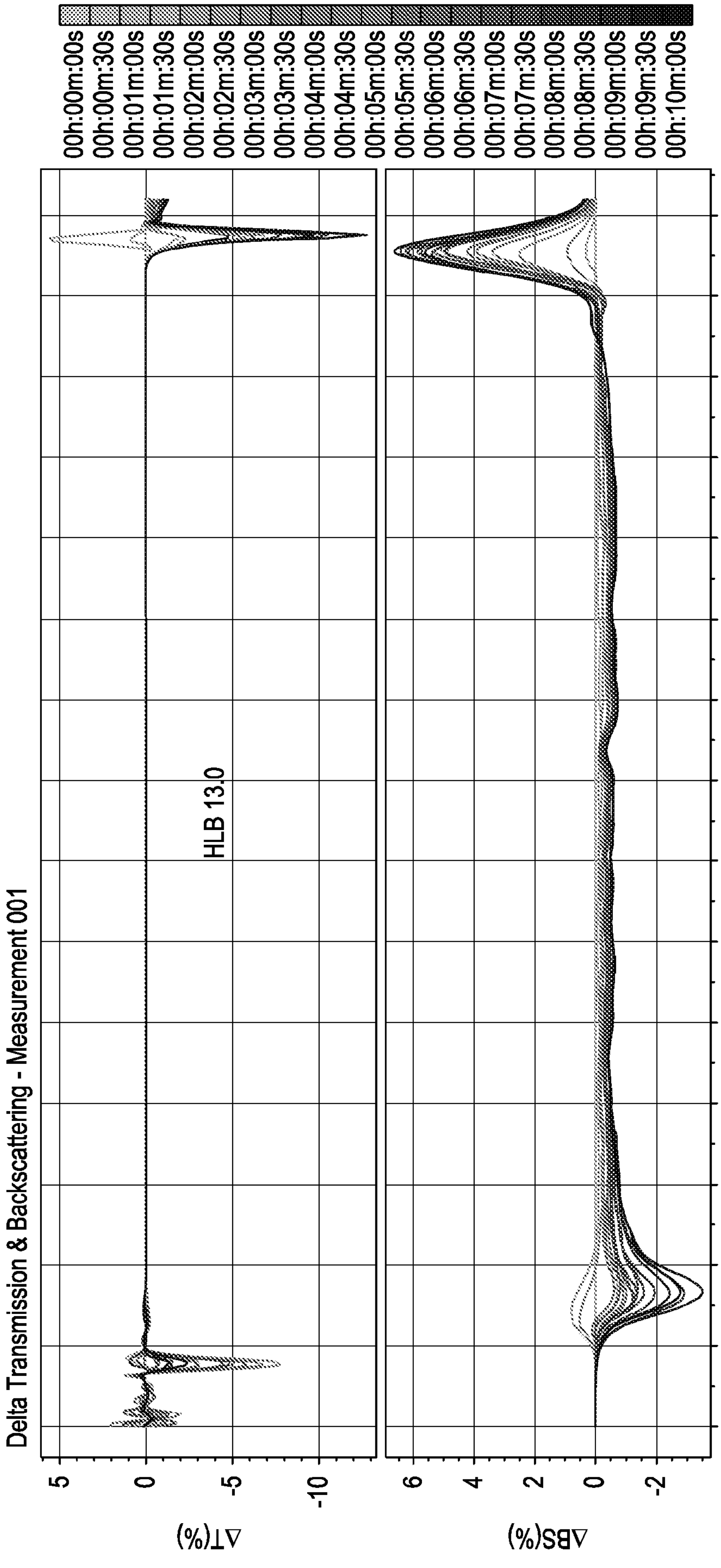
Delta Transmission & Backscattering - Measurement 001
HLB 13.0
ΔT(%)
ΔBS(%)
Height (mm)
5
0
-5
-10
6
4
2
0
-2
0
1
2
3
4
5
6
7
8
9
10
11
12
13
14
15
00h:00m:00s
00h:00m:30s
00h:01m:00s
00h:01m:30s
00h:02m:00s
00h:02m:30s
00h:03m:00s
00h:03m:30s
00h:04m:00s
00h:04m:30s
00h:05m:00s
00h:05m:30s
00h:06m:00s
00h:06m:30s
00h:07m:00s
00h:07m:30s
00h:08m:00s
00h:08m:30s
00h:09m:00s
00h:09m:30s
00h:10m:00s

FIG. 12B

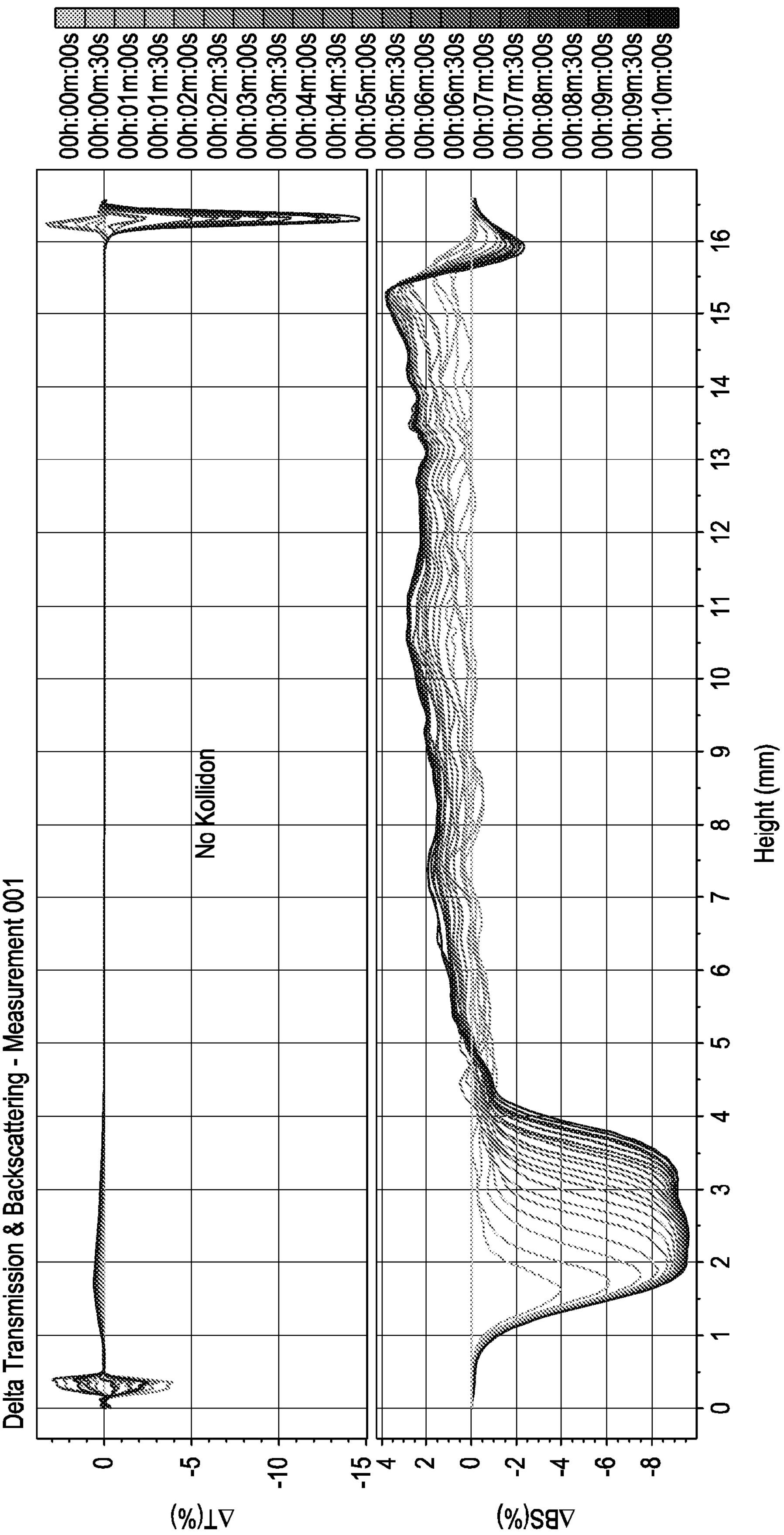
Delta Transmission & Backscattering - Measurement 001
No Kollidon
ΔT(%)
0
-5
-10
-15
ΔBS(%)
4
2
0
-2
-4
-6
-8
0
1
2
3
4
5
6
7
8
9
10
11
12
13
14
15
16
Height (mm)
00h:00m:00s
00h:00m:30s
00h:01m:00s
00h:01m:30s
00h:02m:00s
00h:02m:30s
00h:03m:00s
00h:03m:30s
00h:04m:00s
00h:04m:30s
00h:05m:00s
00h:05m:30s
00h:06m:00s
00h:06m:30s
00h:07m:00s
00h:07m:30s
00h:08m:00s
00h:08m:30s
00h:09m:00s
00h:09m:30s
00h:10m:00s

FIG. 12C

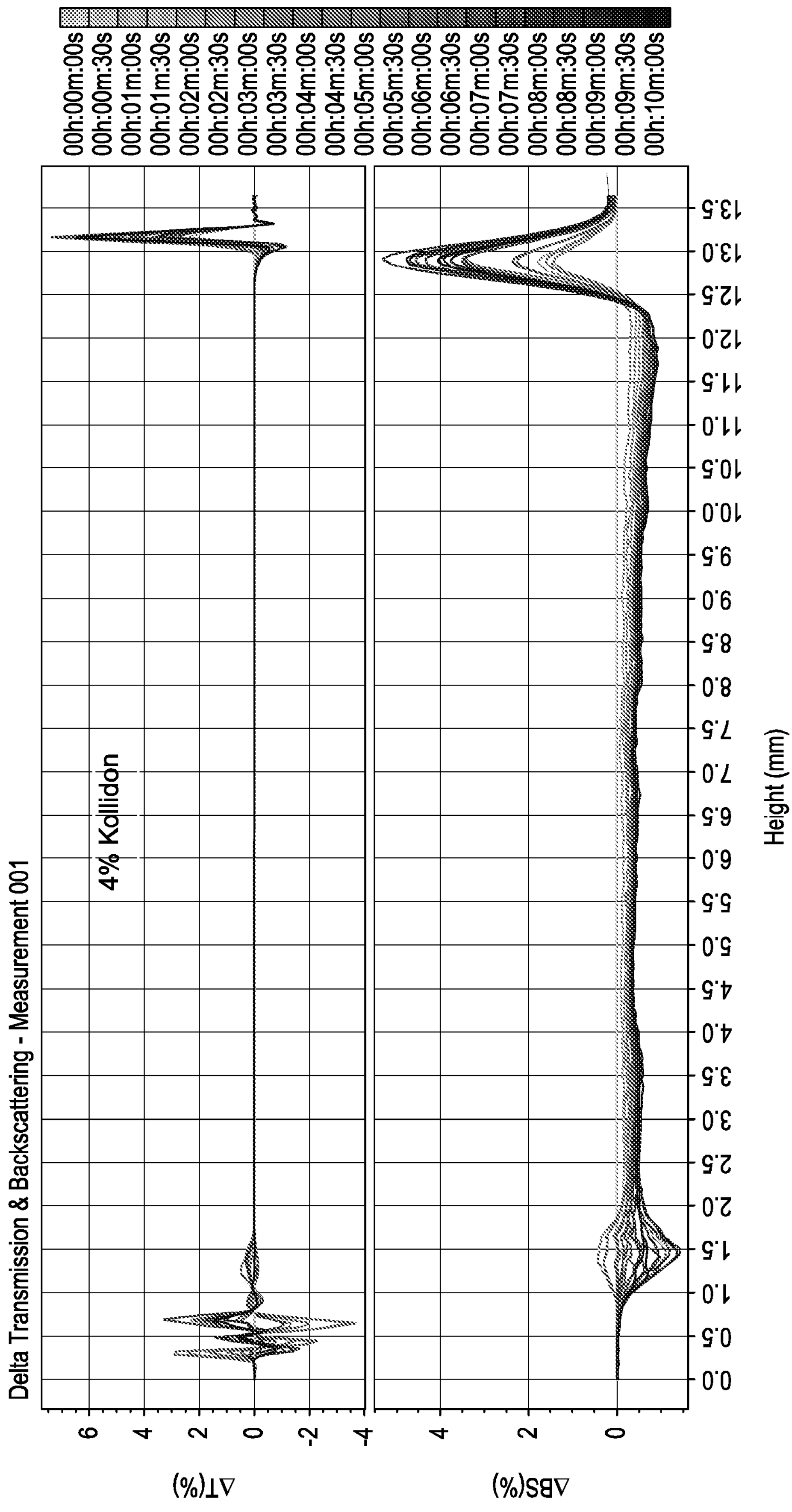

Delta Transmission & Backscattering - Measurement 001
4% Kollidon
ΔT(%)
ΔBS(%)
Height (mm)
00h:00m:00s
00h:00m:30s
00h:01m:00s
00h:01m:30s
00h:02m:00s
00h:02m:30s
00h:03m:00s
00h:03m:30s
00h:04m:00s
00h:04m:30s
00h:05m:00s
00h:05m:30s
00h:06m:00s
00h:06m:30s
00h:07m:00s
00h:07m:30s
00h:08m:00s
00h:08m:30s
00h:09m:00s
00h:09m:30s
00h:10m:00s


FIG. 12D

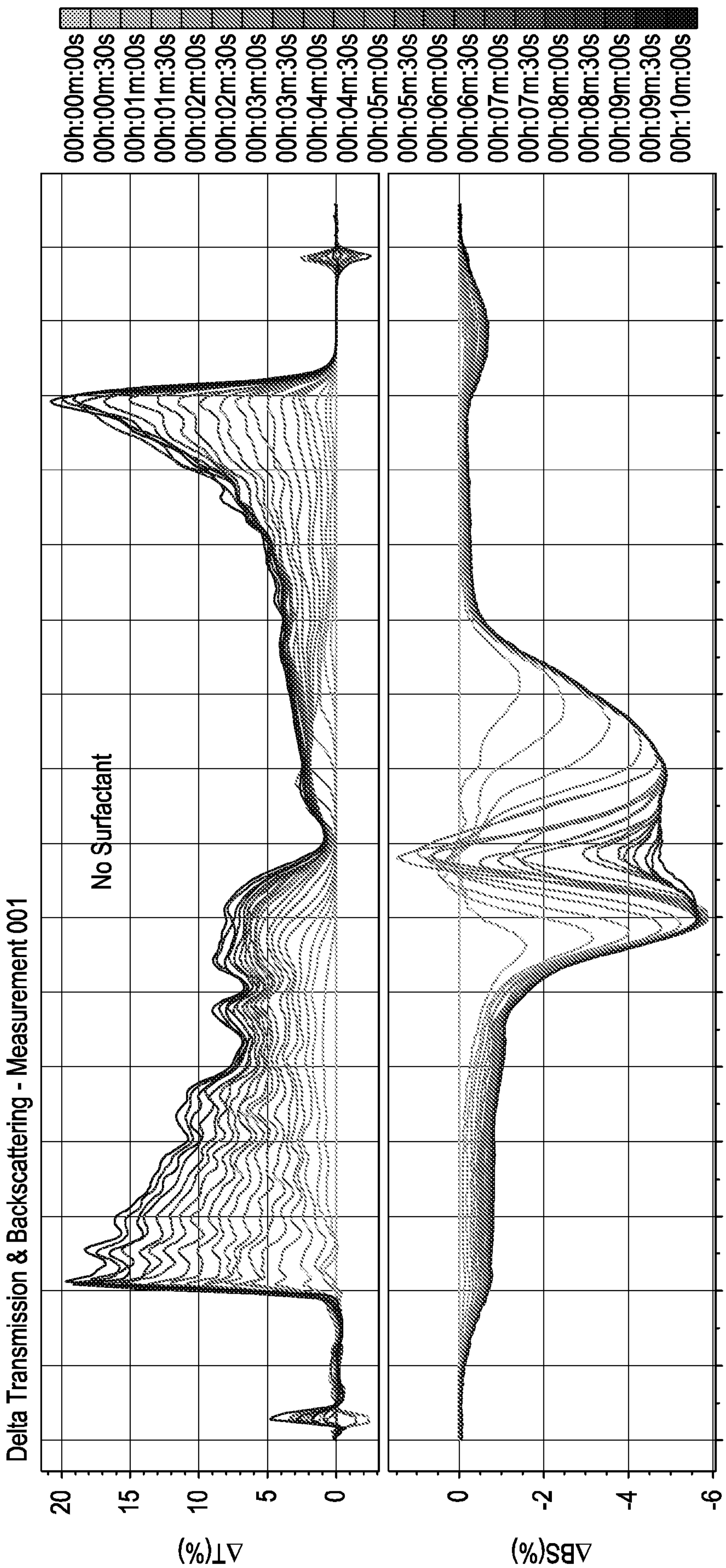
Delta Transmission & Backscattering - Measurement 001
No Surfactant
ΔT(%)
ΔBS(%)
Height (mm)
20
15
10
5
0
-2
-4
-6
0
1
2
3
4
5
6
7
8
9
10
11
12
13
14
15
16
00h:00m:00s
00h:00m:30s
00h:01m:00s
00h:01m:30s
00h:02m:00s
00h:02m:30s
00h:03m:00s
00h:03m:30s
00h:04m:00s
00h:04m:30s
00h:05m:00s
00h:05m:30s
00h:06m:00s
00h:06m:30s
00h:07m:00s
00h:07m:30s
00h:08m:00s
00h:08m:30s
00h:09m:00s
00h:09m:30s
00h:10m:00s

FIG. 12E

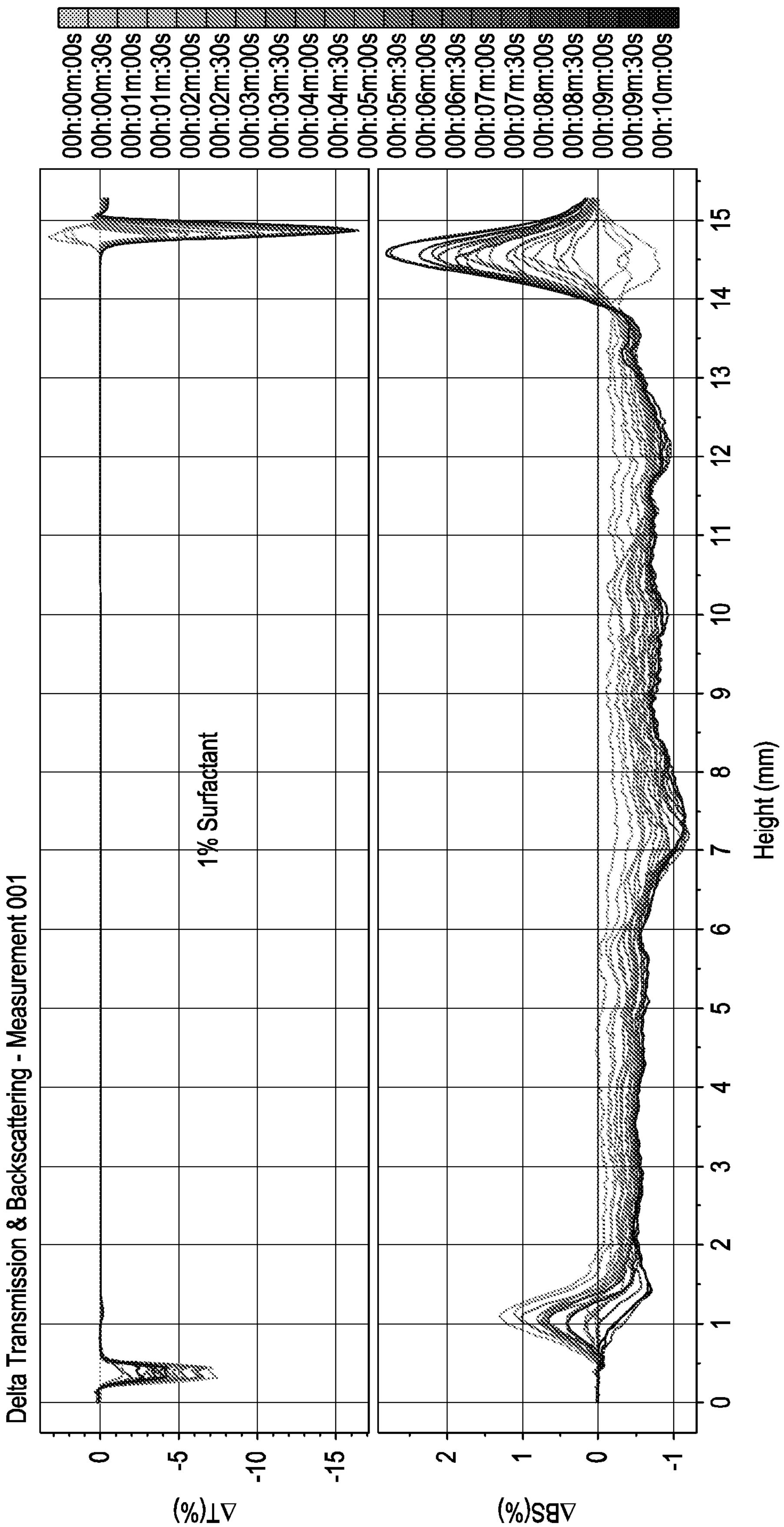
Delta Transmission & Backscattering - Measurement 001
1% Surfactant
ΔT(%)
ΔBS(%)
Height (mm)
0
-5
-10
-15
2
1
0
-1
0 1 2 3 4 5 6 7 8 9 10 11 12 13 14 15
00h:00m:00s
00h:00m:30s
00h:01m:00s
00h:01m:30s
00h:02m:00s
00h:02m:30s
00h:03m:00s
00h:03m:30s
00h:04m:00s
00h:04m:30s
00h:05m:00s
00h:05m:30s
00h:06m:00s
00h:06m:30s
00h:07m:00s
00h:07m:30s
00h:08m:00s
00h:08m:30s
00h:09m:00s
00h:09m:30s
00h:10m:00s

FIG. 12F

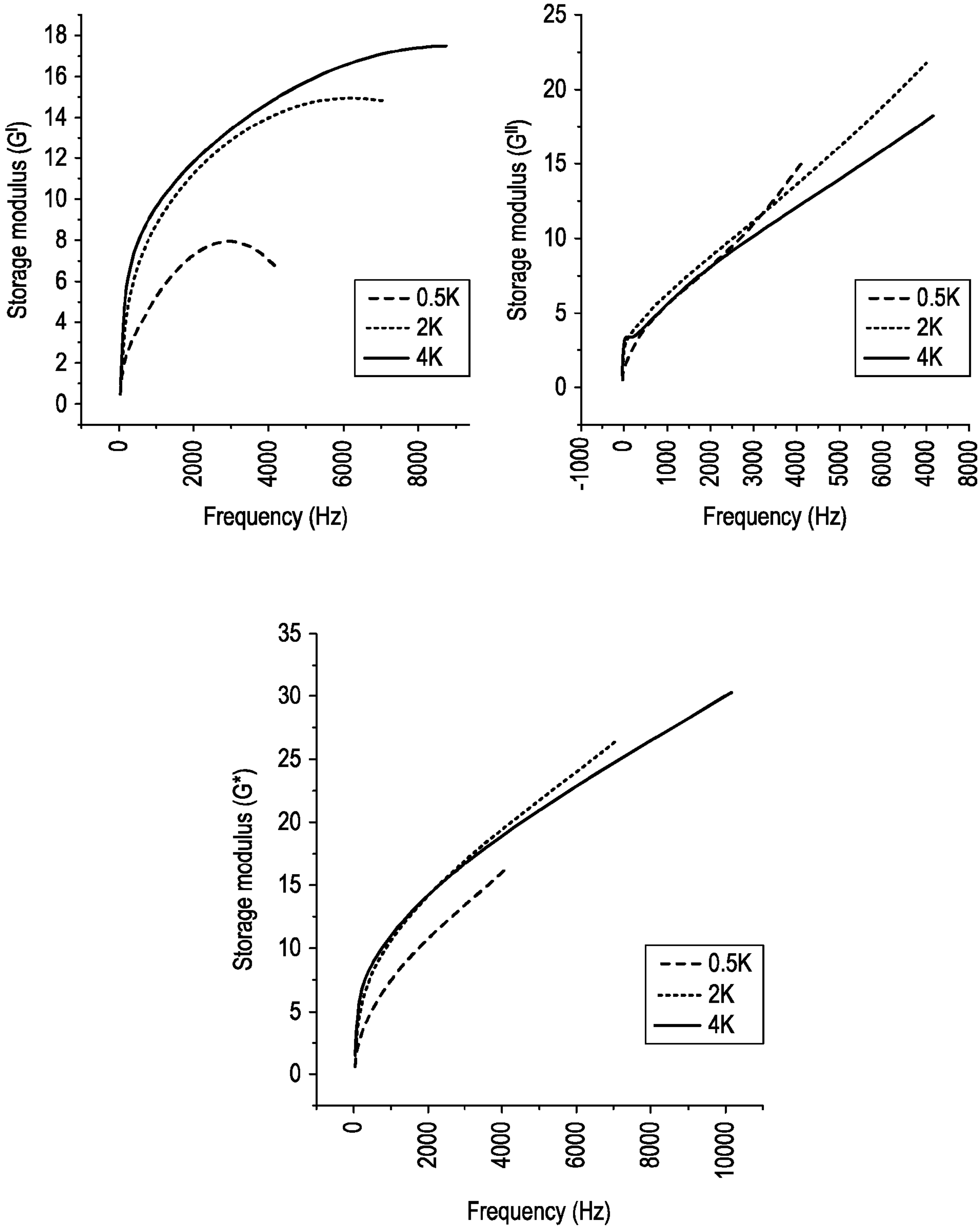

Storage modulus (G')
Frequency (Hz)
0.5K
2K
4K
0
2
4
6
8
10
12
14
16
18
0
2000
4000
6000
8000
Storage modulus (G'')
Frequency (Hz)
0.5K
2K
4K
0
5
10
15
20
25
-1000
0
1000
2000
3000
4000
5000
6000
4000
8000
Storage modulus (G*)
Frequency (Hz)
0.5K
2K
4K
0
5
10
15
20
25
30
35
0
2000
4000
6000
8000
10000


FIG. 13

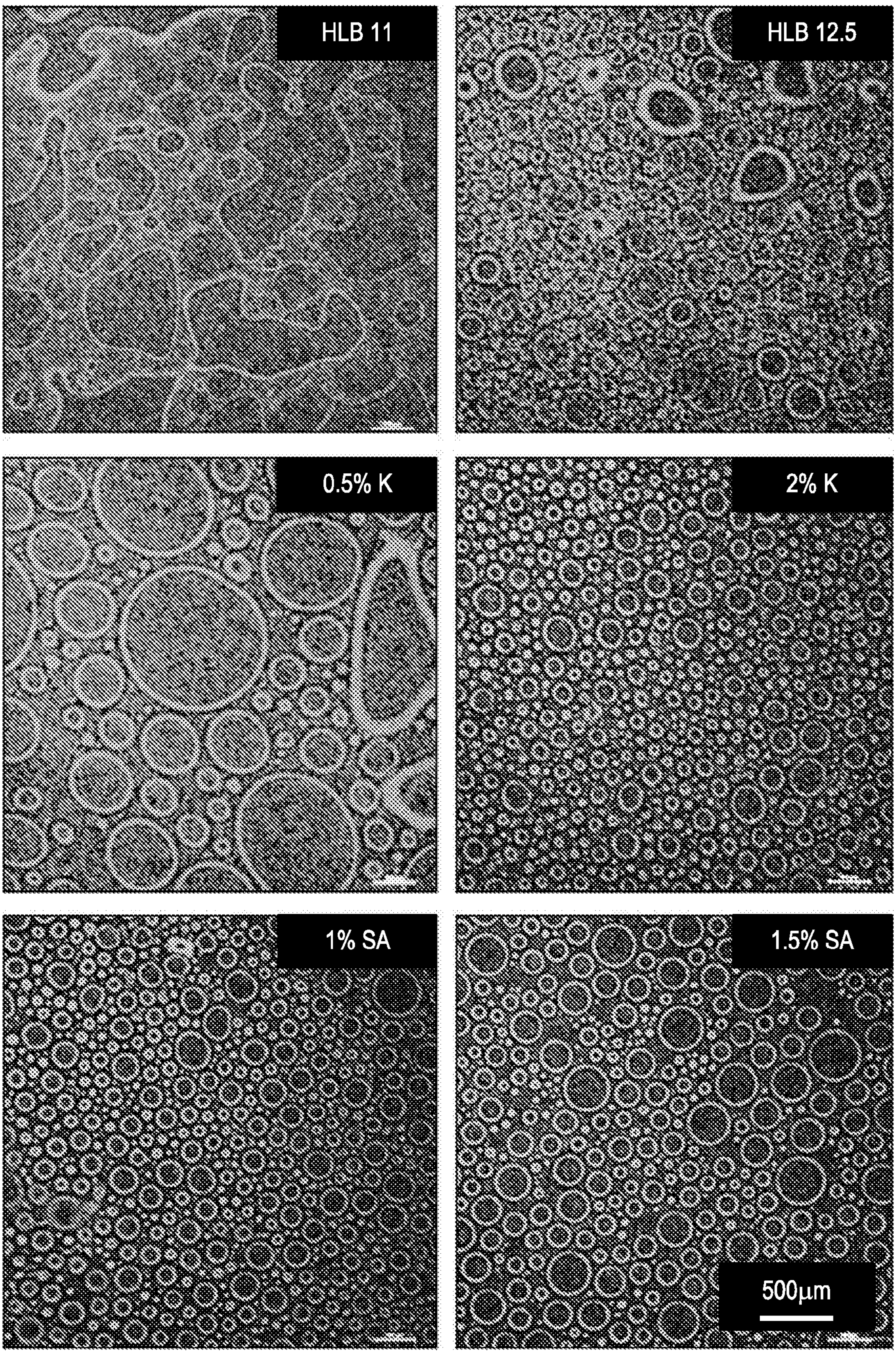


FIG. 14

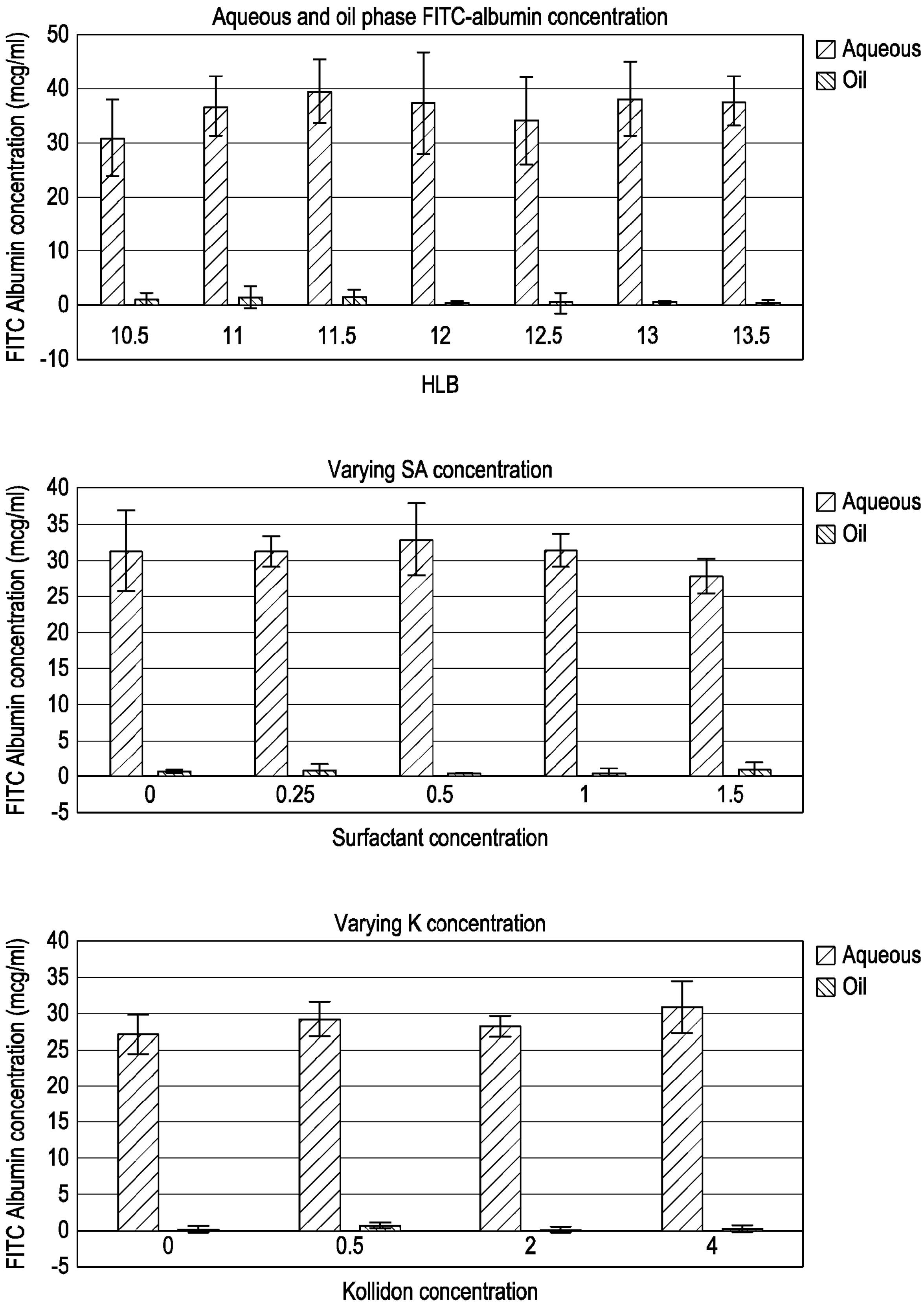
Aqueous and oil phase FITC-albumin concentration
FITC Albumin concentration (mcg/ml)
50
40
30
20
10
0
-10
10.5
11
11.5
12
12.5
13
13.5
HLB
Aqueous
Oil
Varying SA concentration
FITC Albumin concentration (mcg/ml)
40
35
30
25
20
15
10
5
0
-5
0
0.25
0.5
1
1.5
Surfactant concentration
Aqueous
Oil
Varying K concentration
FITC Albumin concentration (mcg/ml)
40
35
30
25
20
15
10
5
0
-5
0
0.5
2
4
Kollidon concentration
Aqueous
Oil

*FIG. 15*

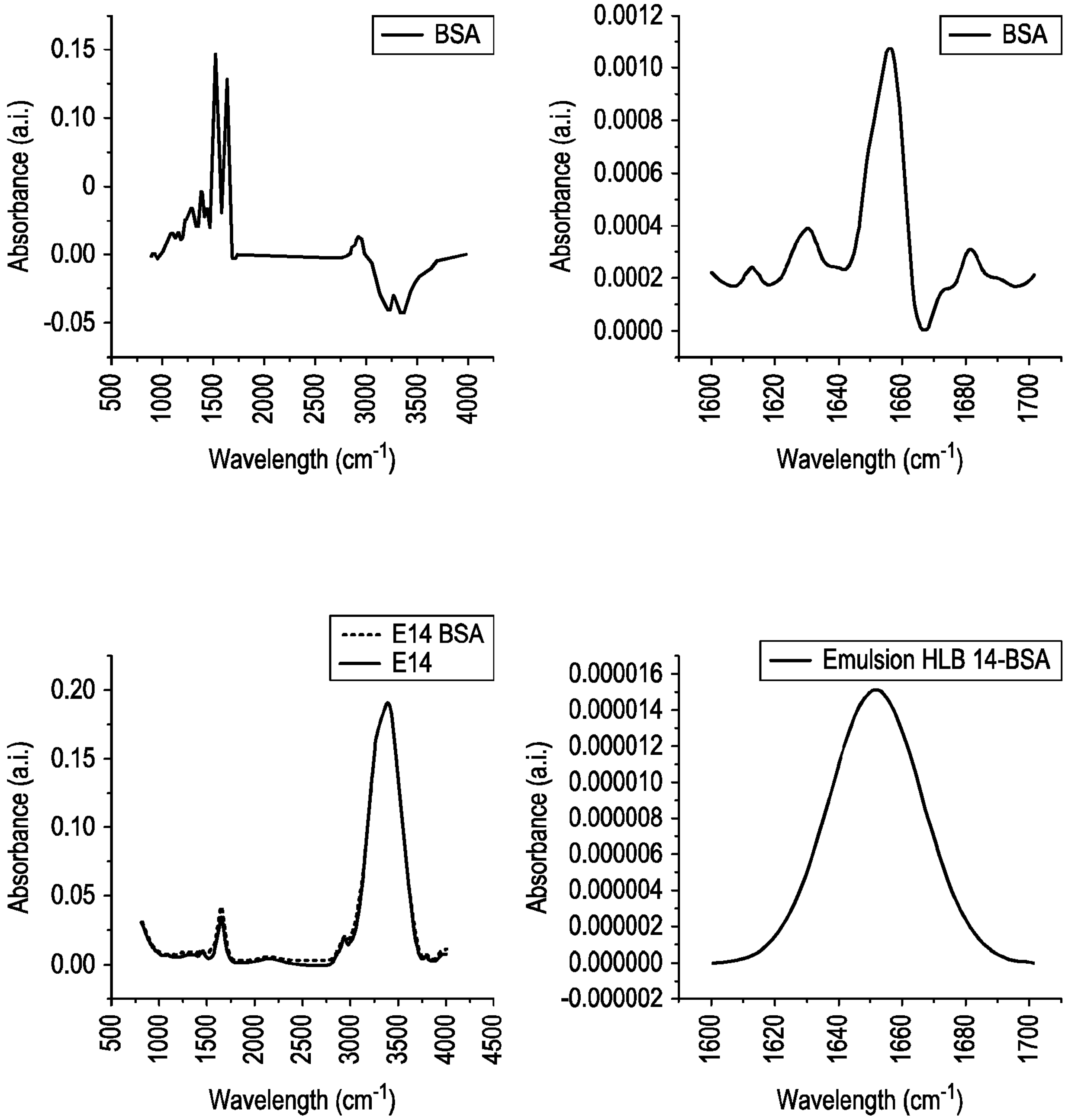
BSA
Absorbance (a.i.)
Wavelength (cm-1)
BSA
Absorbance (a.i.)
Wavelength (cm-1)
E14 BSA
E14
Absorbance (a.i.)
Wavelength (cm-1)
Emulsion HLB 14-BSA
Absorbance (a.i.)
Wavelength (cm-1)

*FIG. 16*

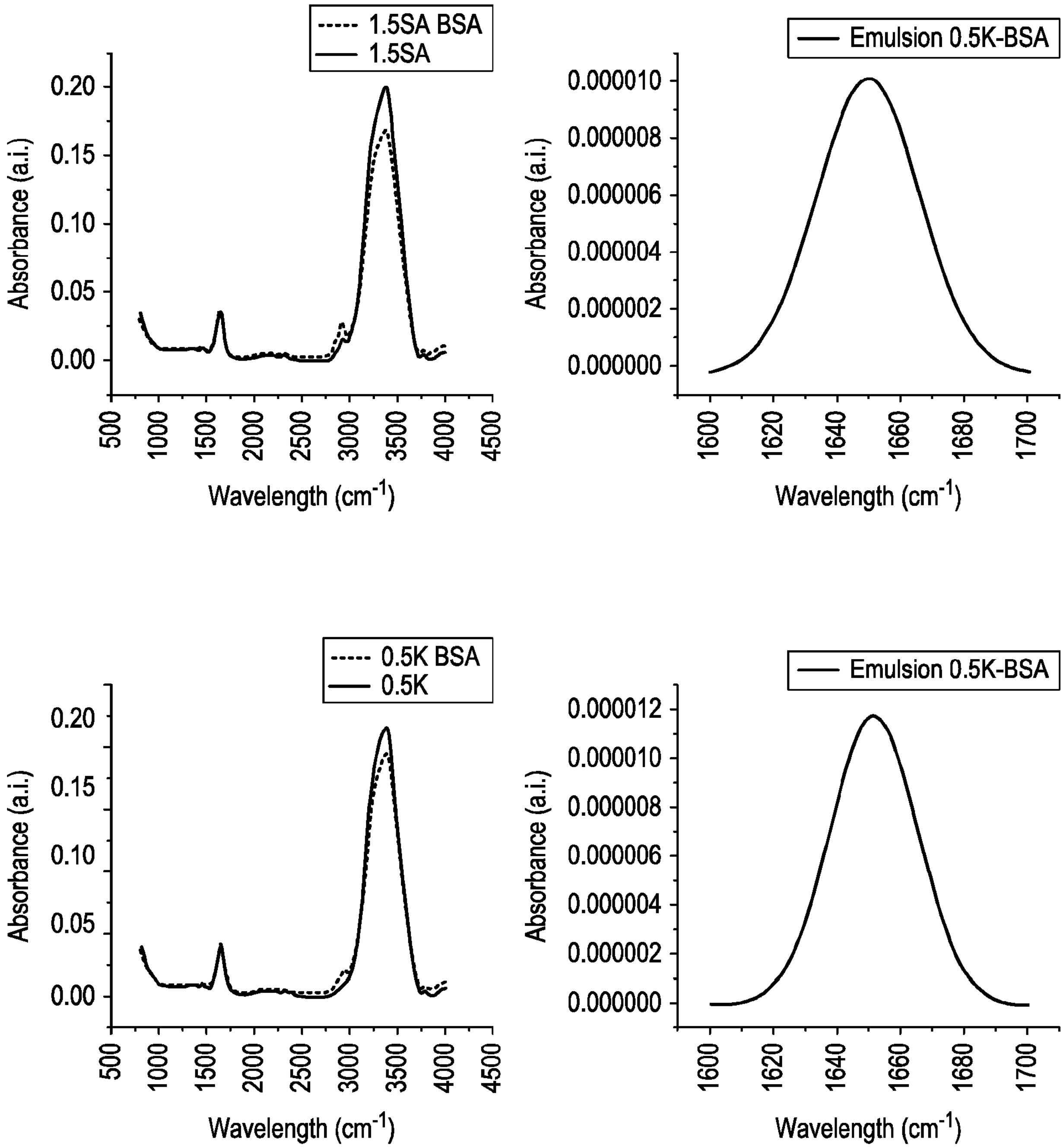
1.5SA BSA
1.5SA
Absorbance (a.i.)
0.20
0.15
0.10
0.05
0.00
500
1000
1500
2000
2500
3000
3500
4000
4500
Wavelength (cm-1)
Emulsion 0.5K-BSA
0.000010
0.000008
0.000006
0.000004
0.000002
0.000000
1600
1620
1640
1660
1680
1700
0.5K BSA
0.5K
0.000012

*FIG. 16 Cont'd*

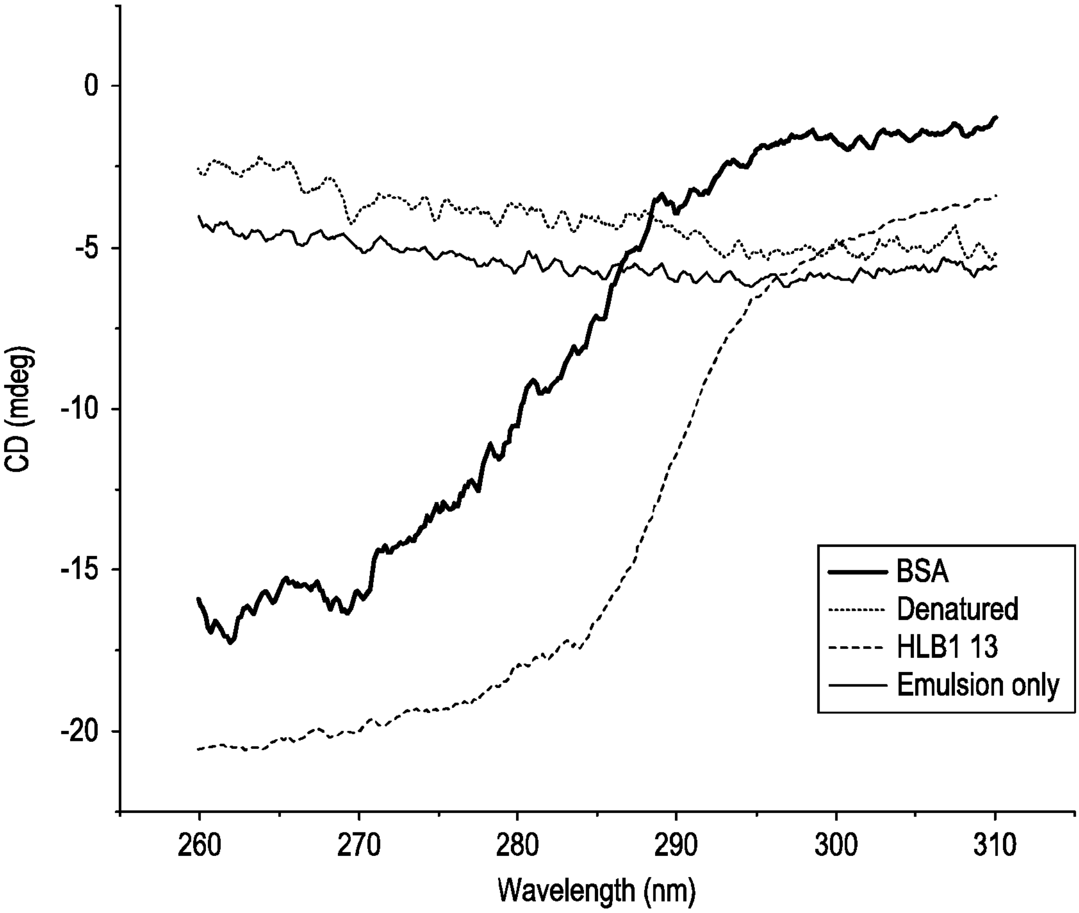
0
-5
-10
-15
-20
CD (mdeg)
260
270
280
290
300
310
Wavelength (nm)
BSA
Denatured
HLB1 13
Emulsion only

*FIG. 17*

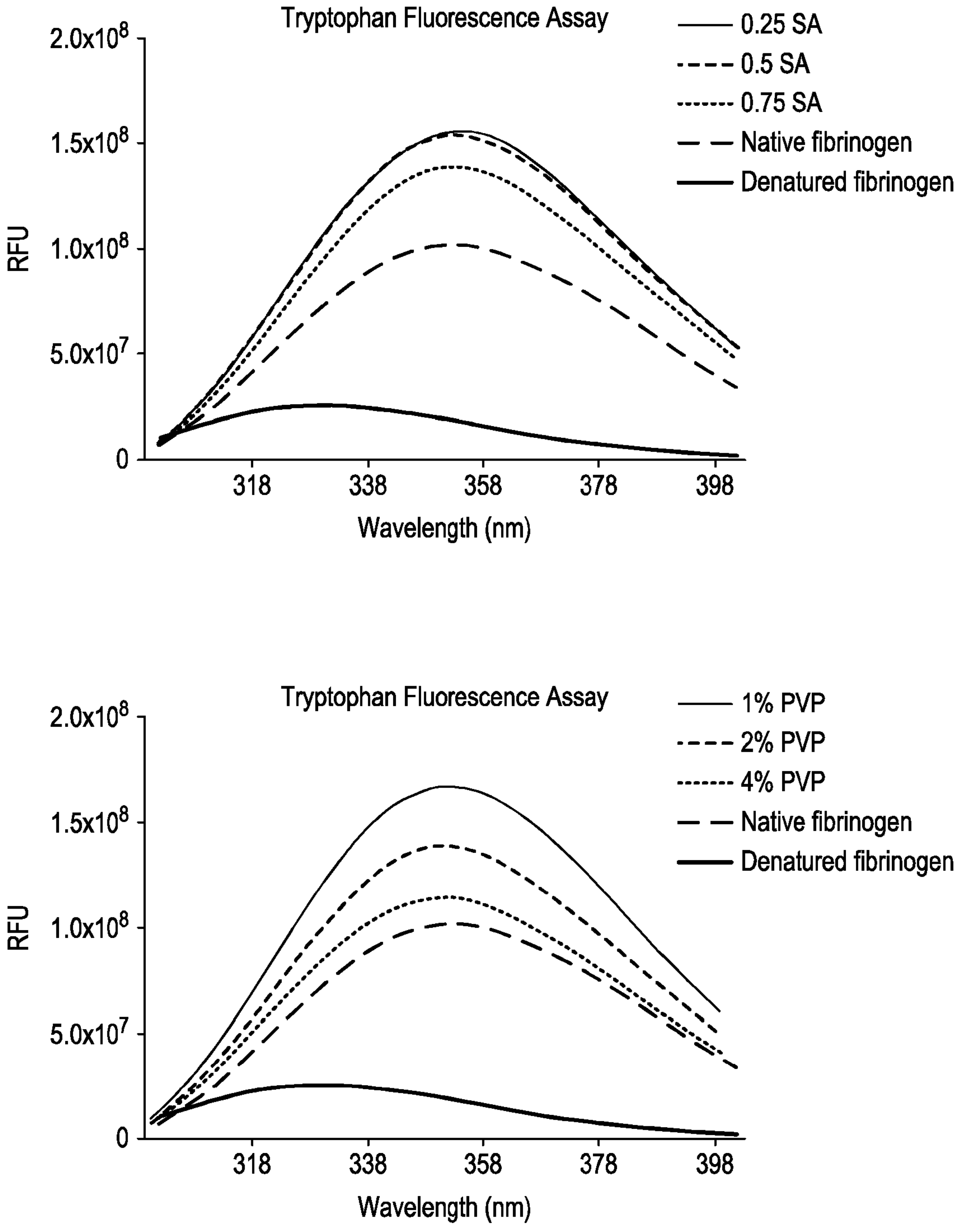
Tryptophan Fluorescence Assay
0.25 SA
0.5 SA
0.75 SA
Native fibrinogen
Denatured fibrinogen
RFU
2.0x10^8
1.5x10^8
1.0x10^8
5.0x10^7
0
318
338
358
378
398
Wavelength (nm)
Tryptophan Fluorescence Assay
1% PVP
2% PVP
4% PVP
Native fibrinogen
Denatured fibrinogen
RFU
2.0x10^8
1.5x10^8
1.0x10^8
5.0x10^7
0
318
338
358
378
398
Wavelength (nm)

FIG. 18

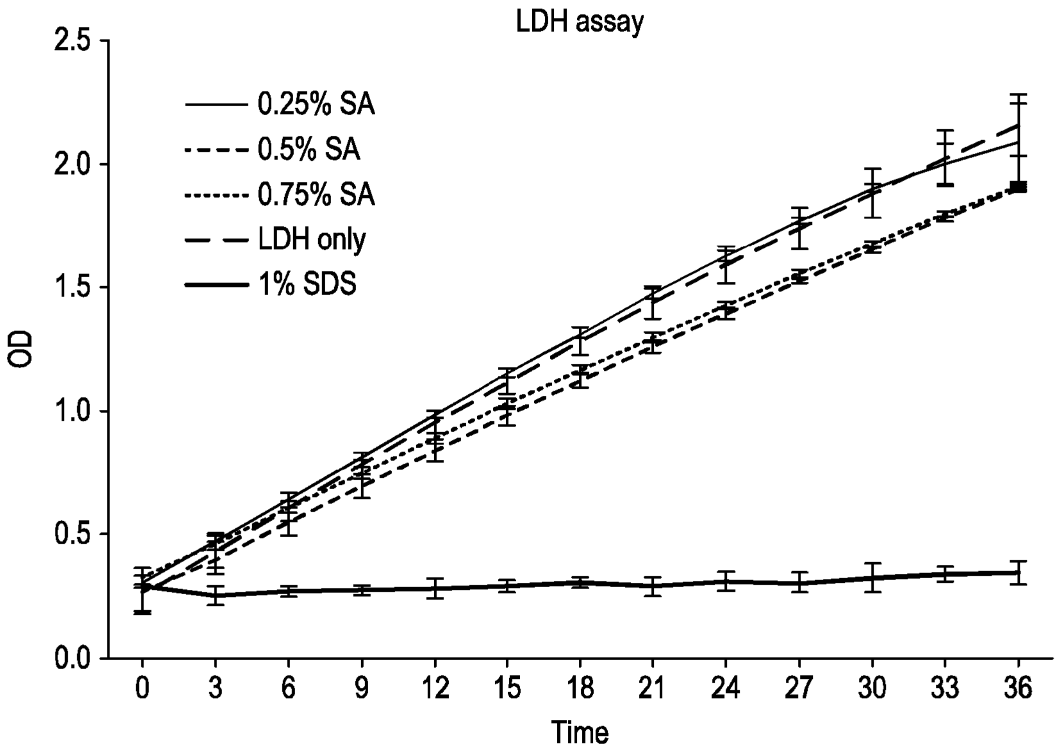
LDH assay
0.25% SA
0.5% SA
0.75% SA
LDH only
1% SDS
OD
2.5
2.0
1.5
1.0
0.5
0.0
0
3
6
9
12
15
18
21
24
27
30
33
36
Time

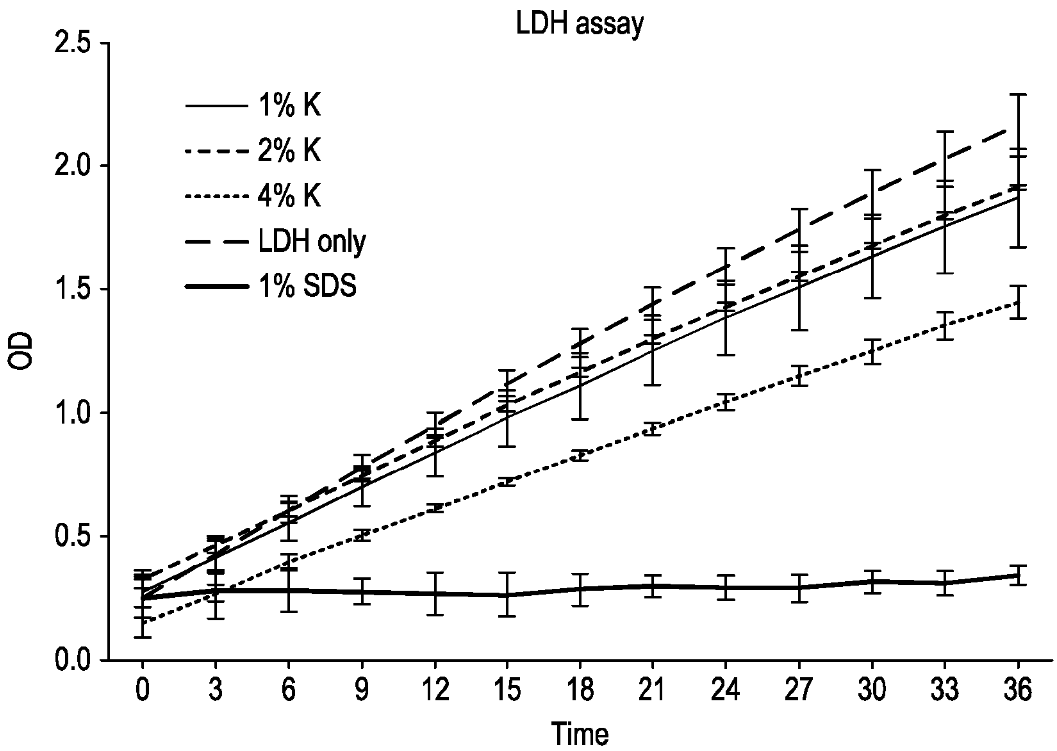
LDH assay
1% K
2% K
4% K
LDH only
1% SDS
OD
2.5
2.0
1.5
1.0
0.5
0.0
0
3
6
9
12
15
18
21
24
27
30
33
36
Time

*FIG. 19*

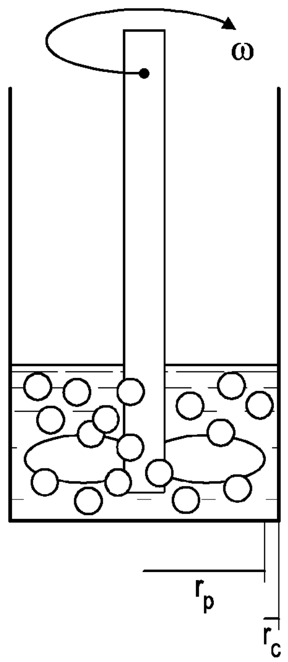
ω
$r_p$
$r_c$

FIG. 20

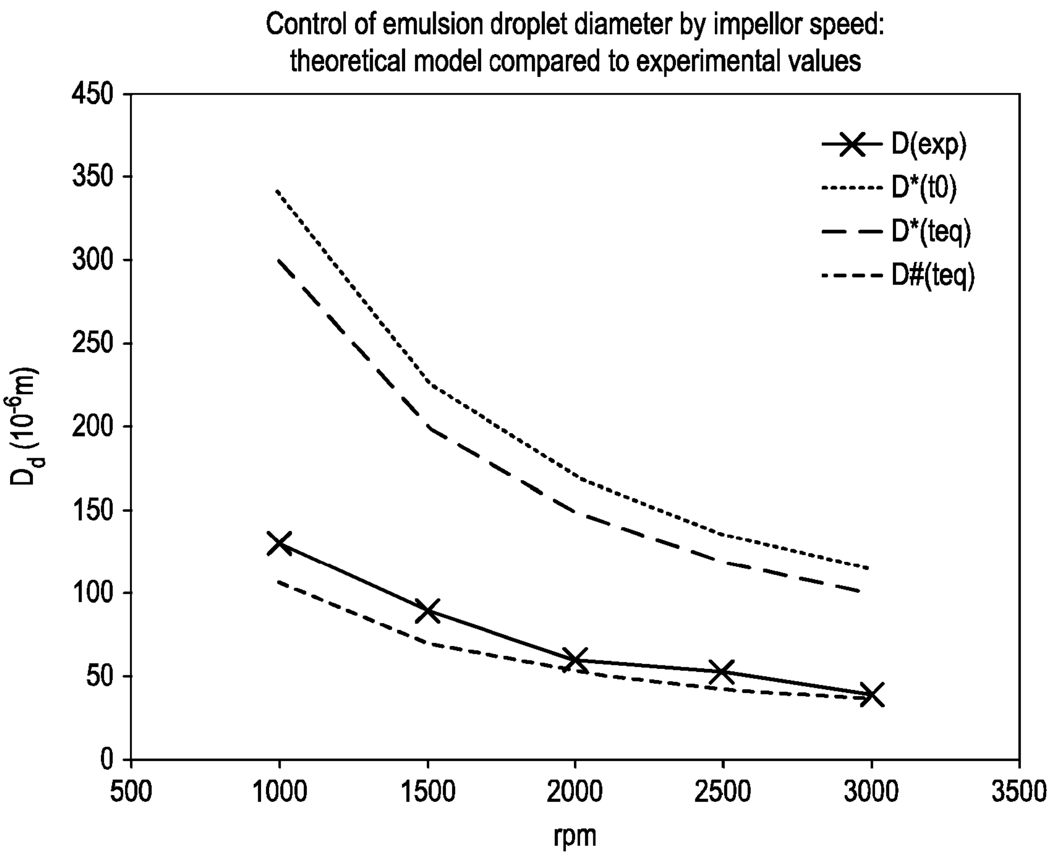
Control of emulsion droplet diameter by impellor speed:
theoretical model compared to experimental values
D(exp)
D*(t0)
D*(teq)
D#(teq)
$D_d$ ($10^{-6}$m)
rpm
0
50
100
150
200
250
300
350
450
500
1000
1500
2000
2500
3000
3500

FIG. 21

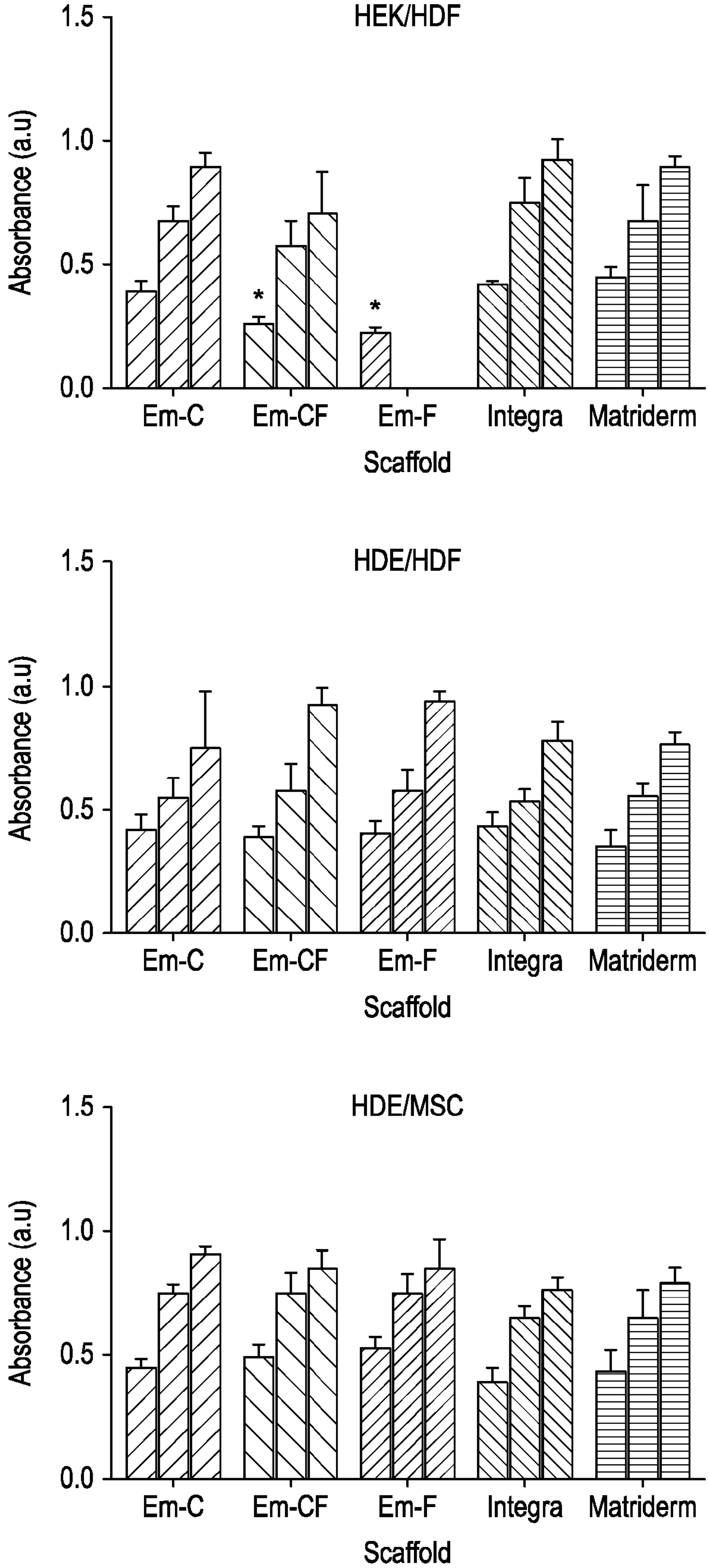
HEK/HDF
Absorbance (a.u)
1.5
1.0
0.5
0.0
*
*
Em-C
Em-CF
Em-F
Integra
Matriderm
Scaffold
HDE/HDF
Absorbance (a.u)
1.5
1.0
0.5
0.0
Em-C
Em-CF
Em-F
Integra
Matriderm
Scaffold
HDE/MSC
Absorbance (a.u)
1.5
1.0
0.5
0.0
Em-C
Em-CF
Em-F
Integra
Matriderm
Scaffold

*FIG. 22*

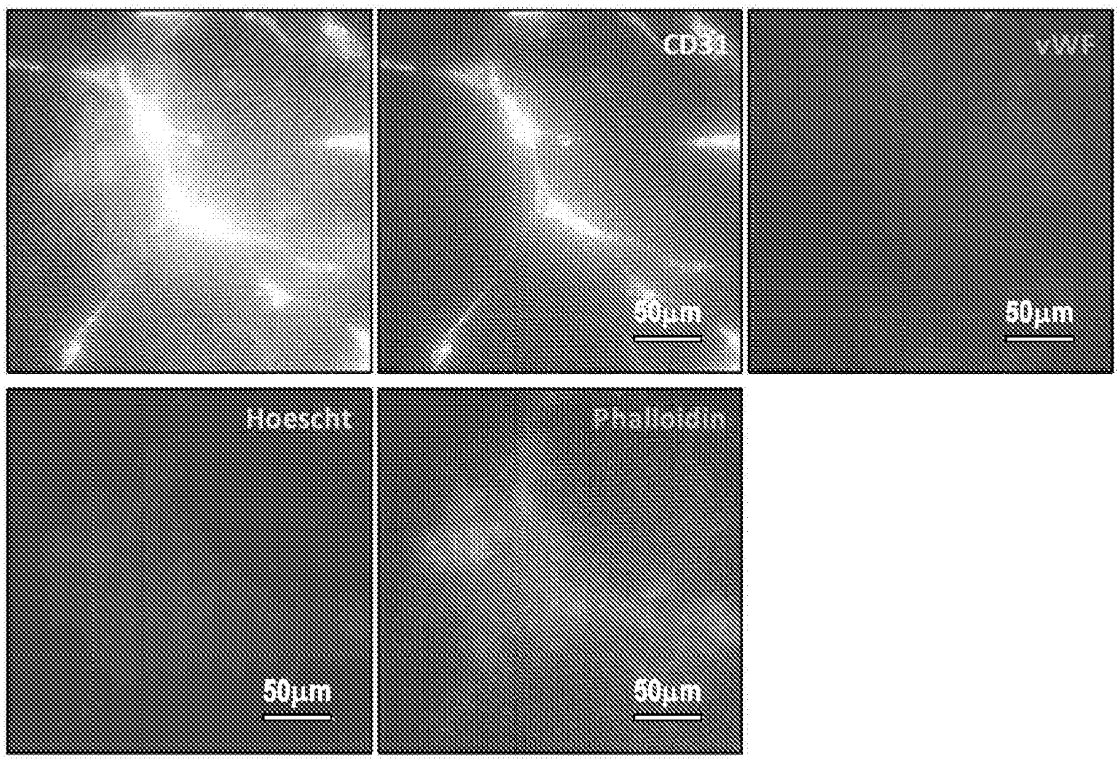


FIG. 23A

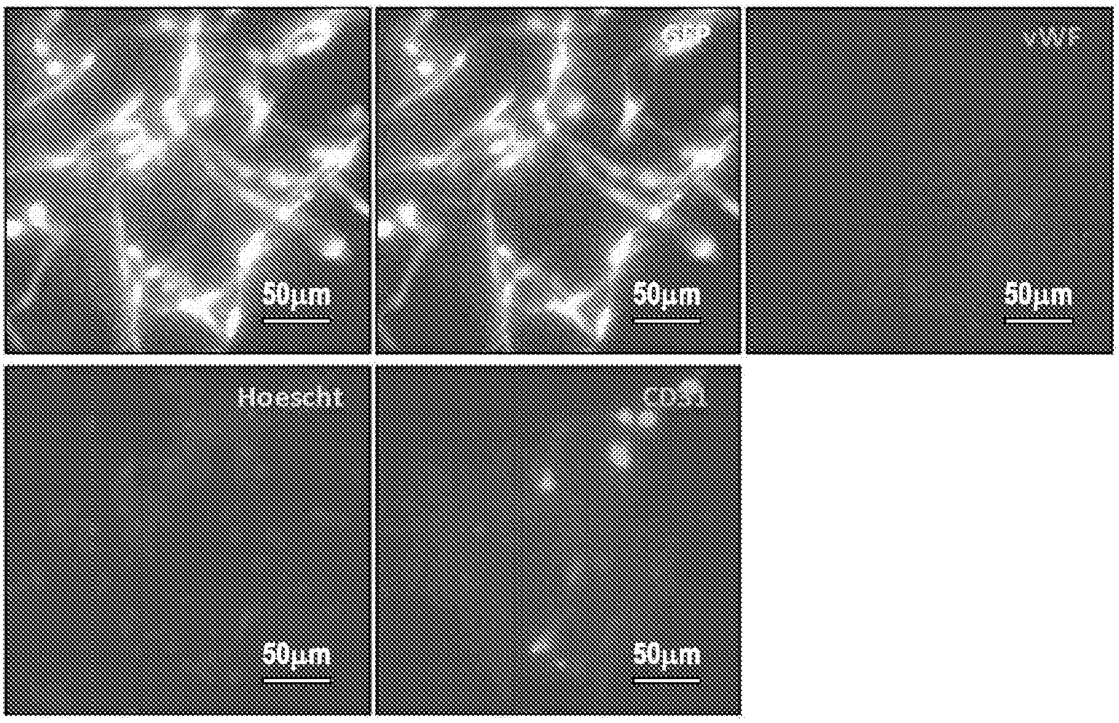


FIG. 23B

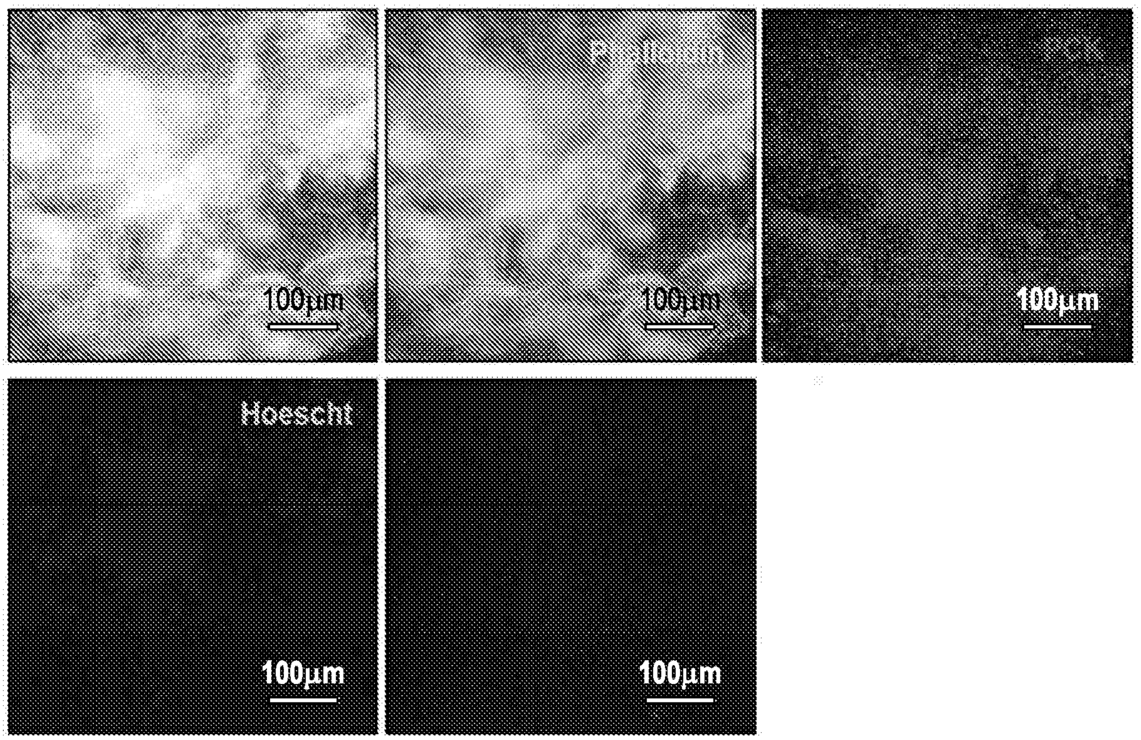
Phalloidin
100μm
100μm
100μm
Hoescht
100μm
100μm

(C)

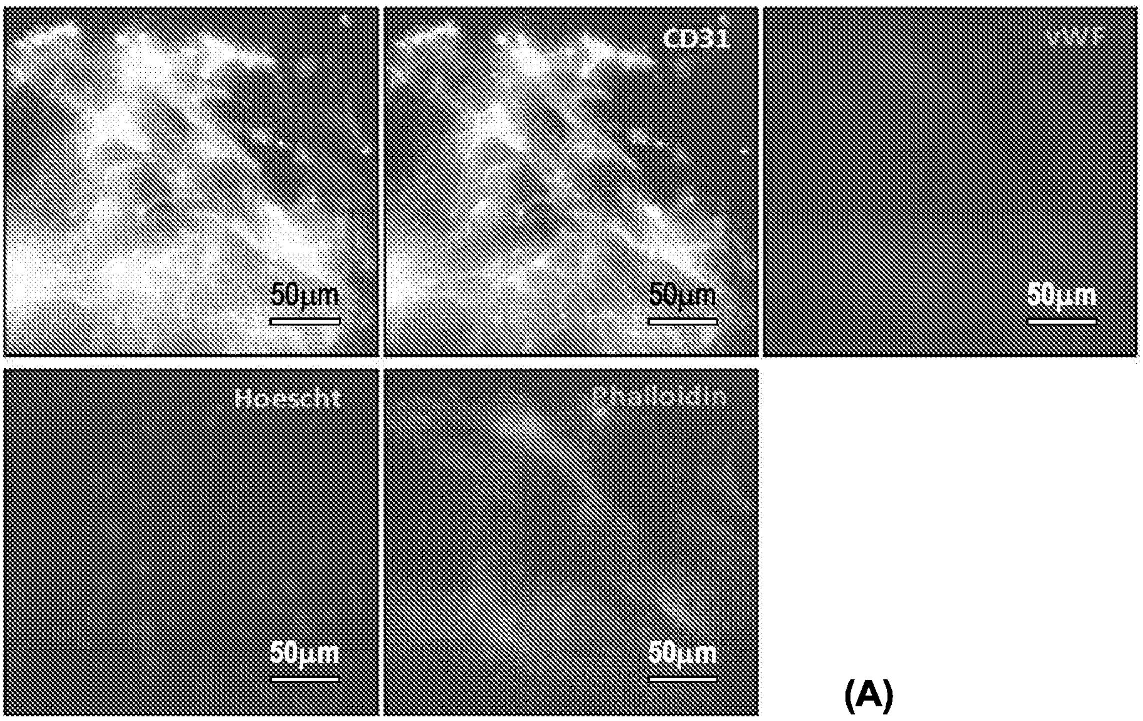
CD31
vWF
50μm
50μm
50μm
Hoescht
Phalloidin
50μm
50μm

(A)

*FIG. 24*

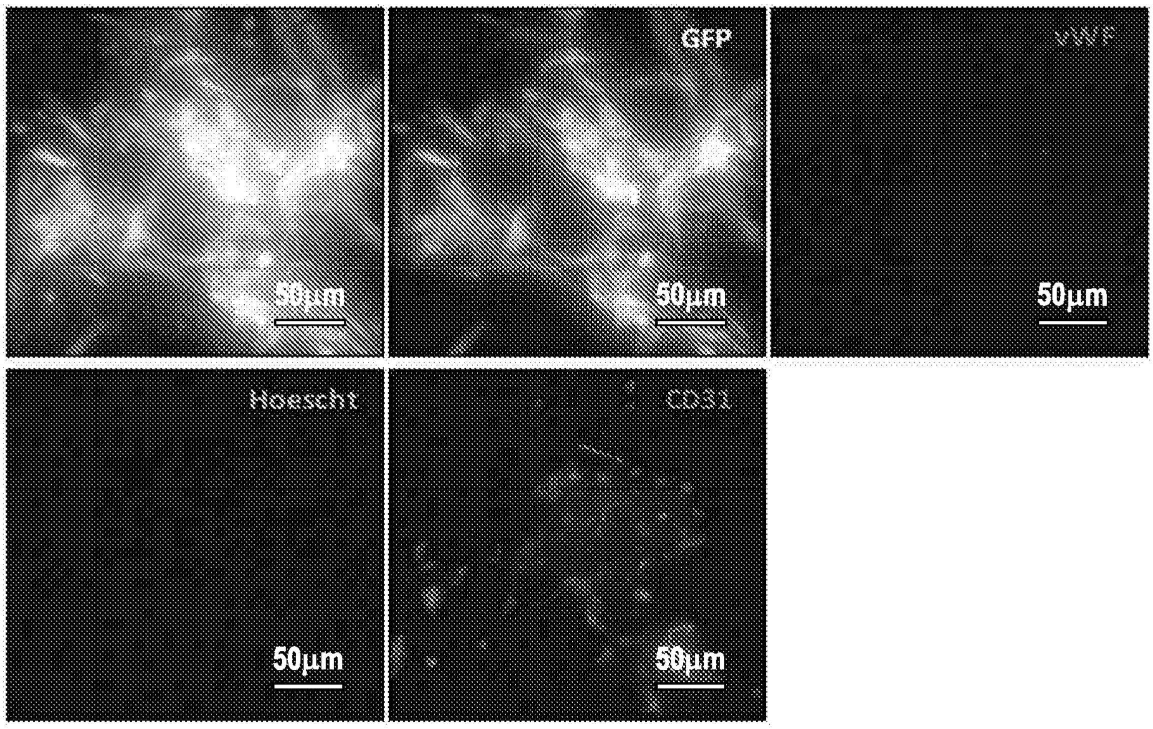
GFP
vWF
50μm
50μm
50μm
Hoescht
CD31
50μm
50μm

(B)

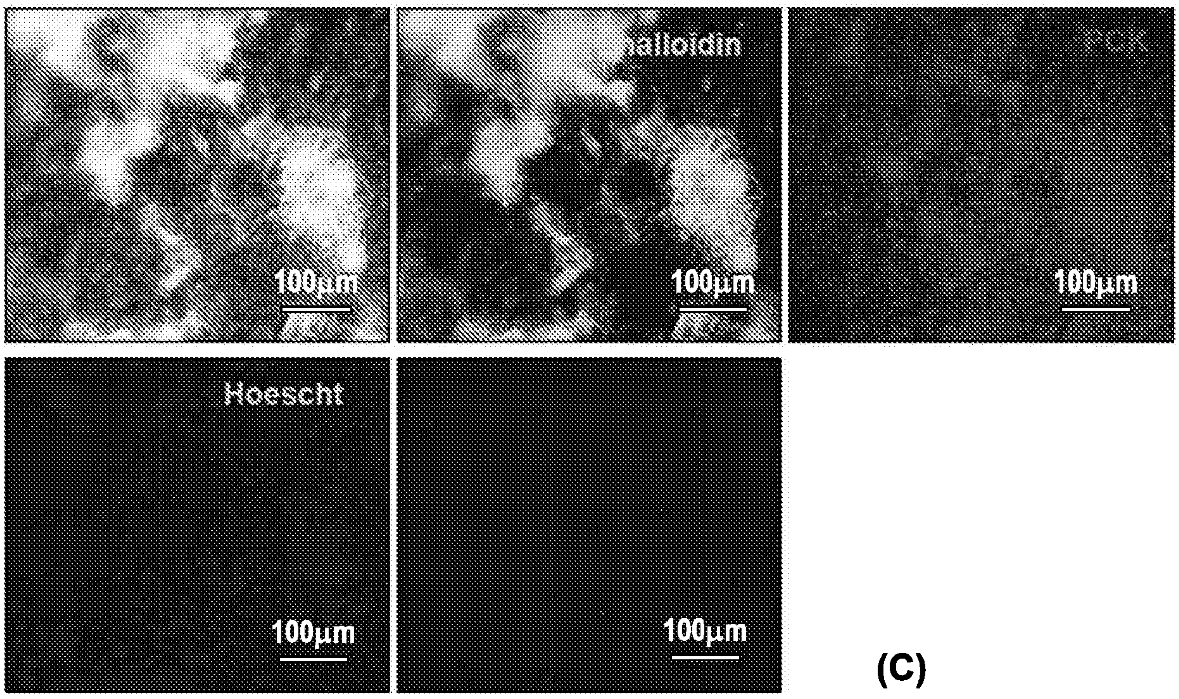
halloidin
100μm
100μm
100μm
Hoescht
100μm
100μm

(C)

*FIG. 25*

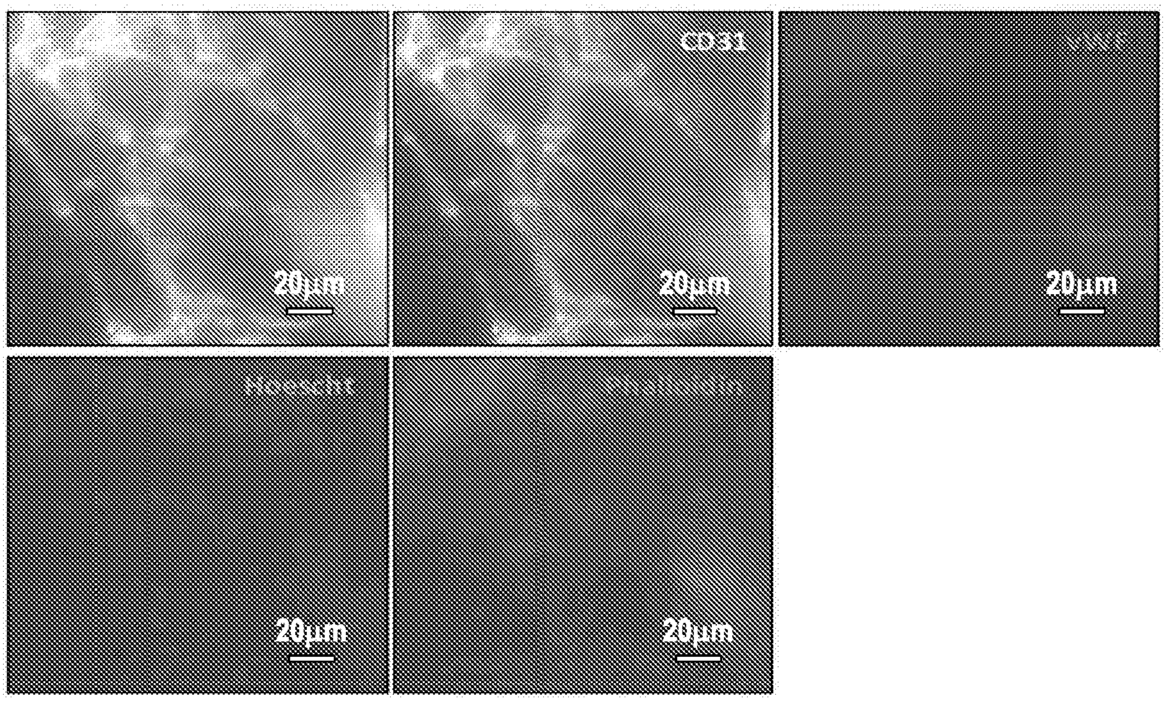
CD31
20µm
20µm
20µm
Hoescht
20µm
20µm

(A)

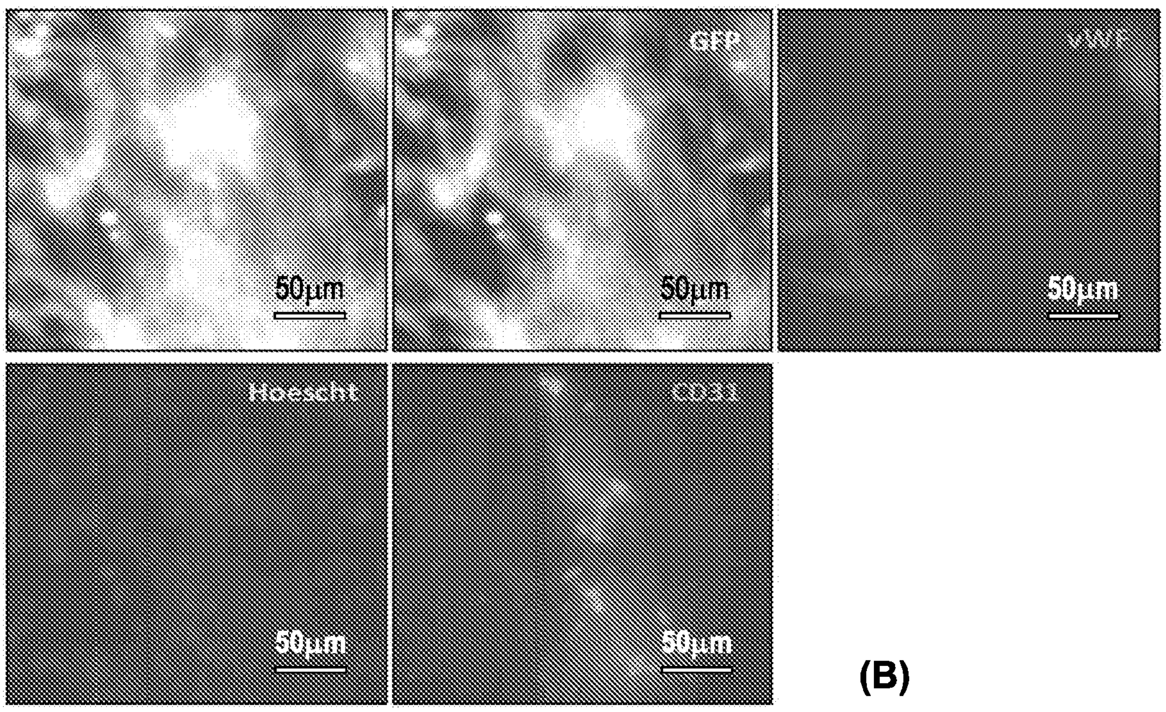
GFP
vWF
50µm
50µm
50µm
Hoescht
CD31
50µm
50µm

(B)

FIG. 26

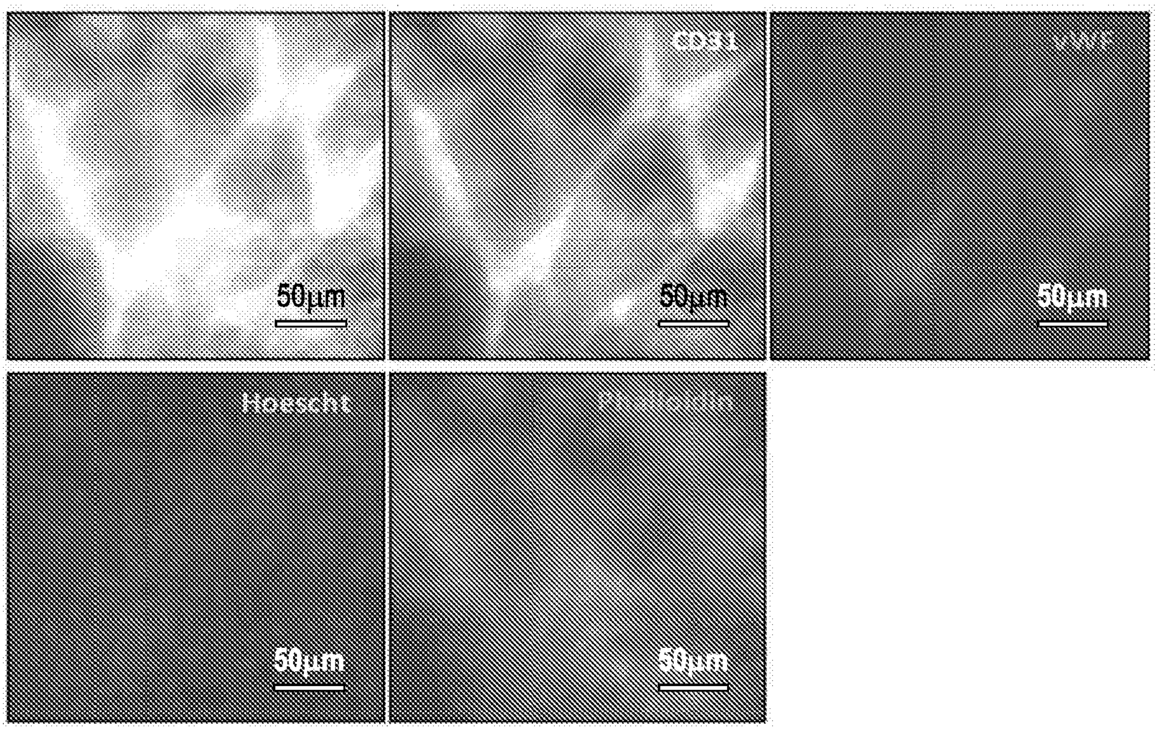


FIG. 27A

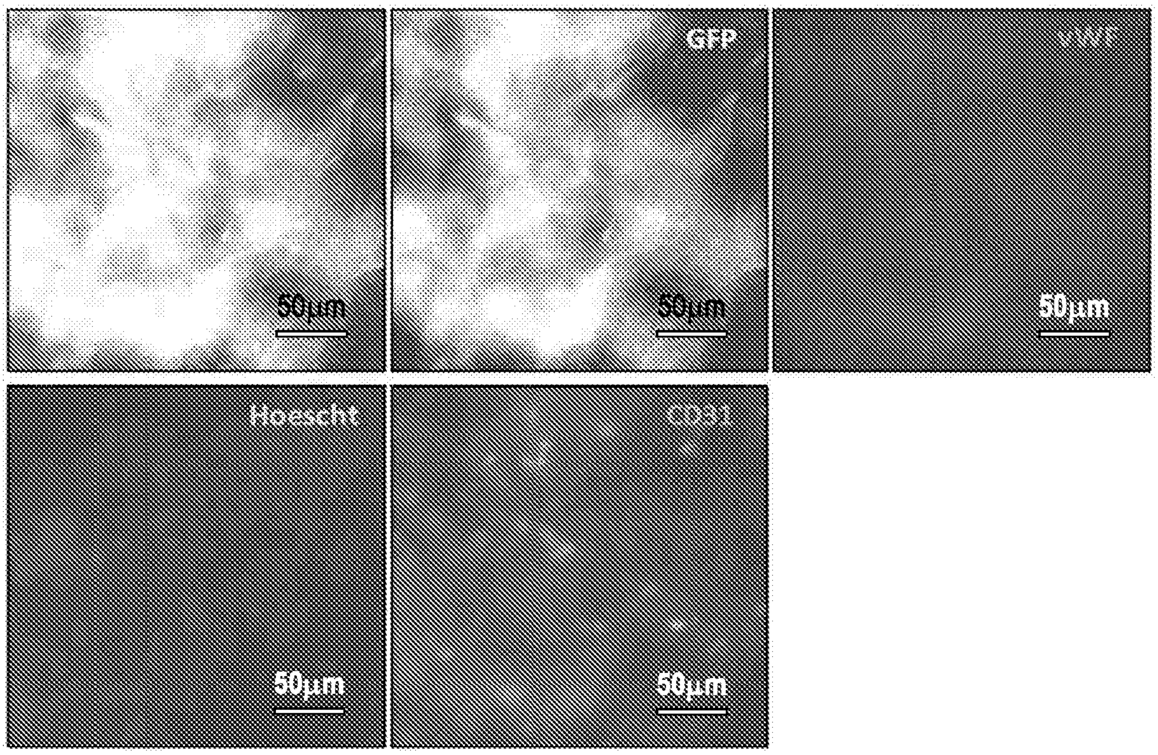


FIG. 27B

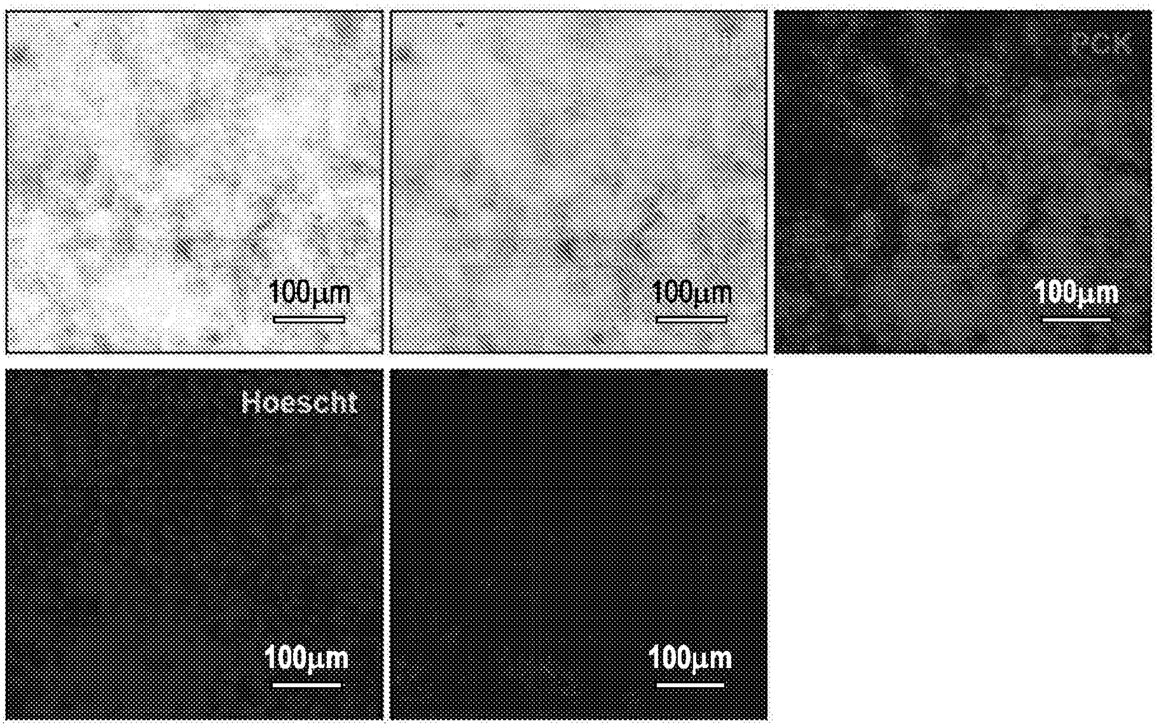
PCK
100μm
100μm
100μm
Hoescht
100μm
100μm

(C)

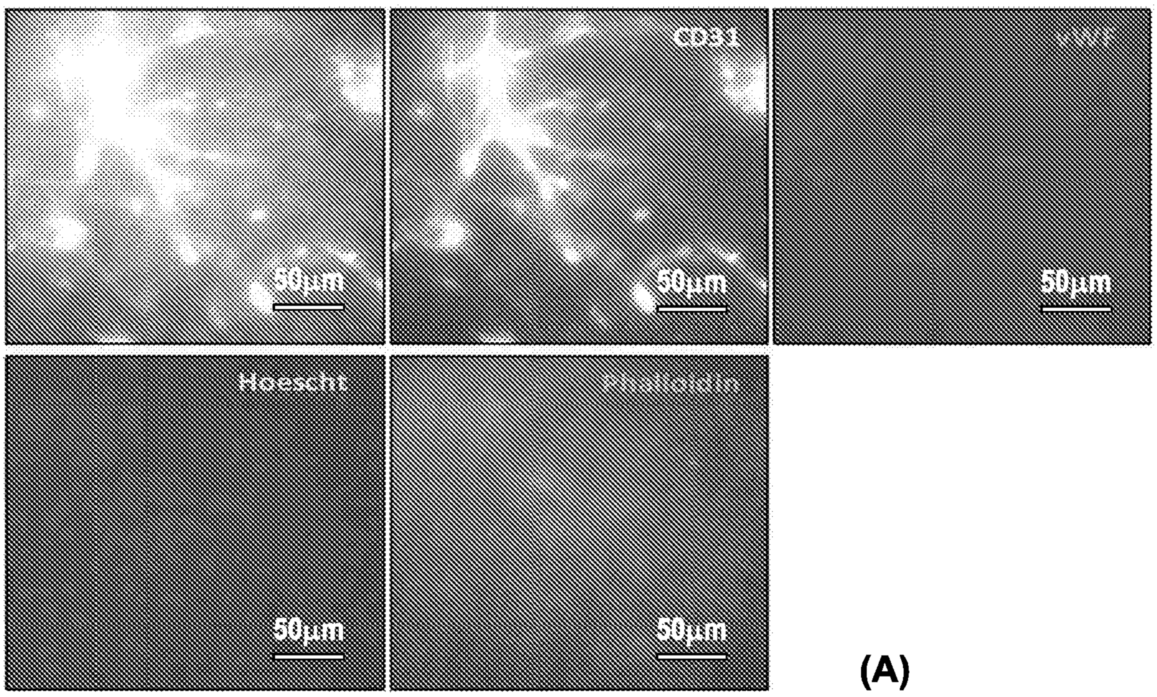
CD31
50μm
50μm
50μm
Hoescht
Phalloidin
50μm
50μm

(A)

*FIG. 28*

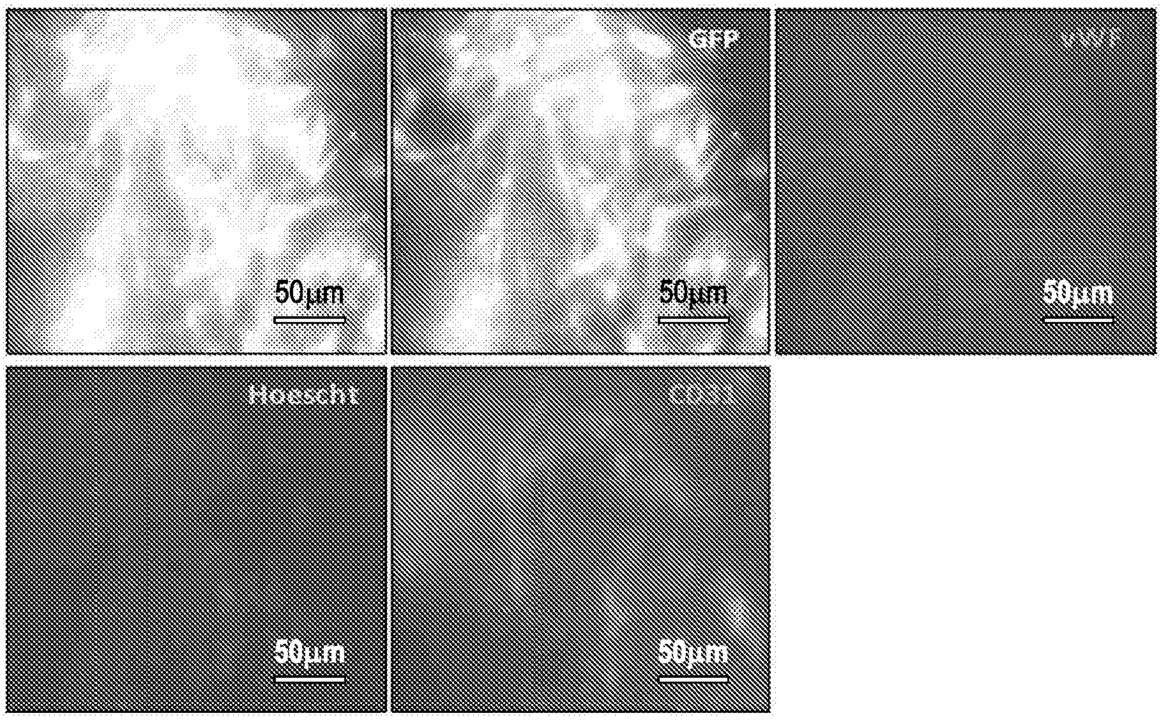


(B)

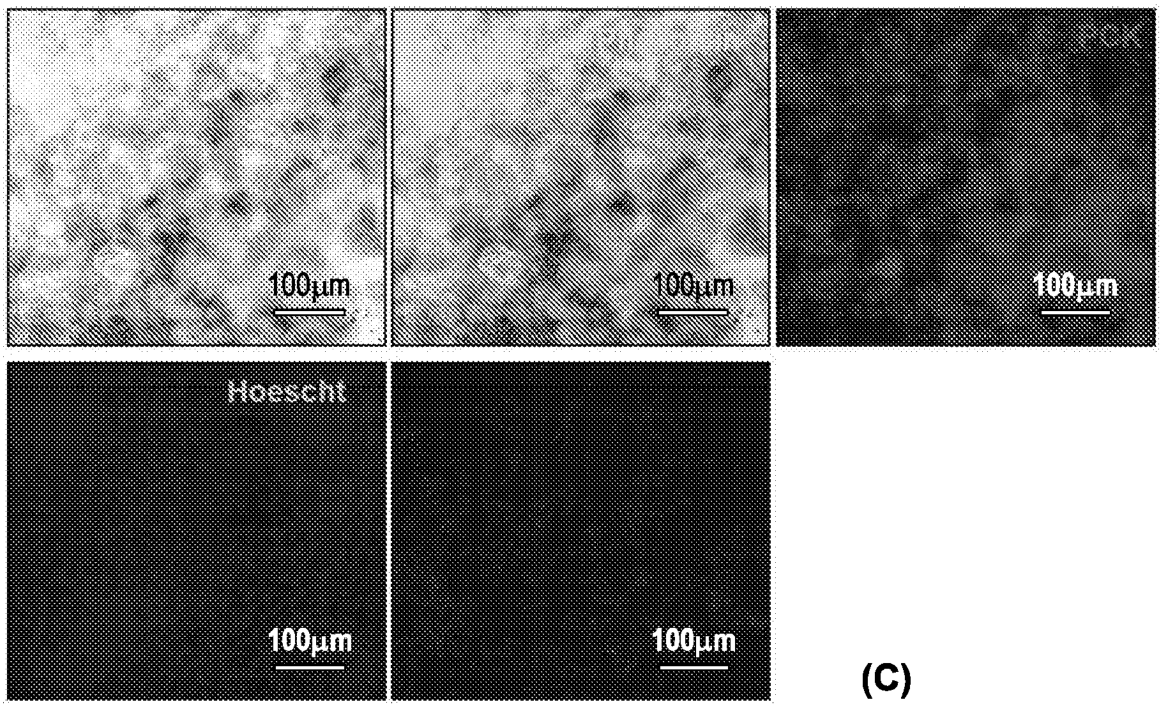


(C)

*FIG. 29*

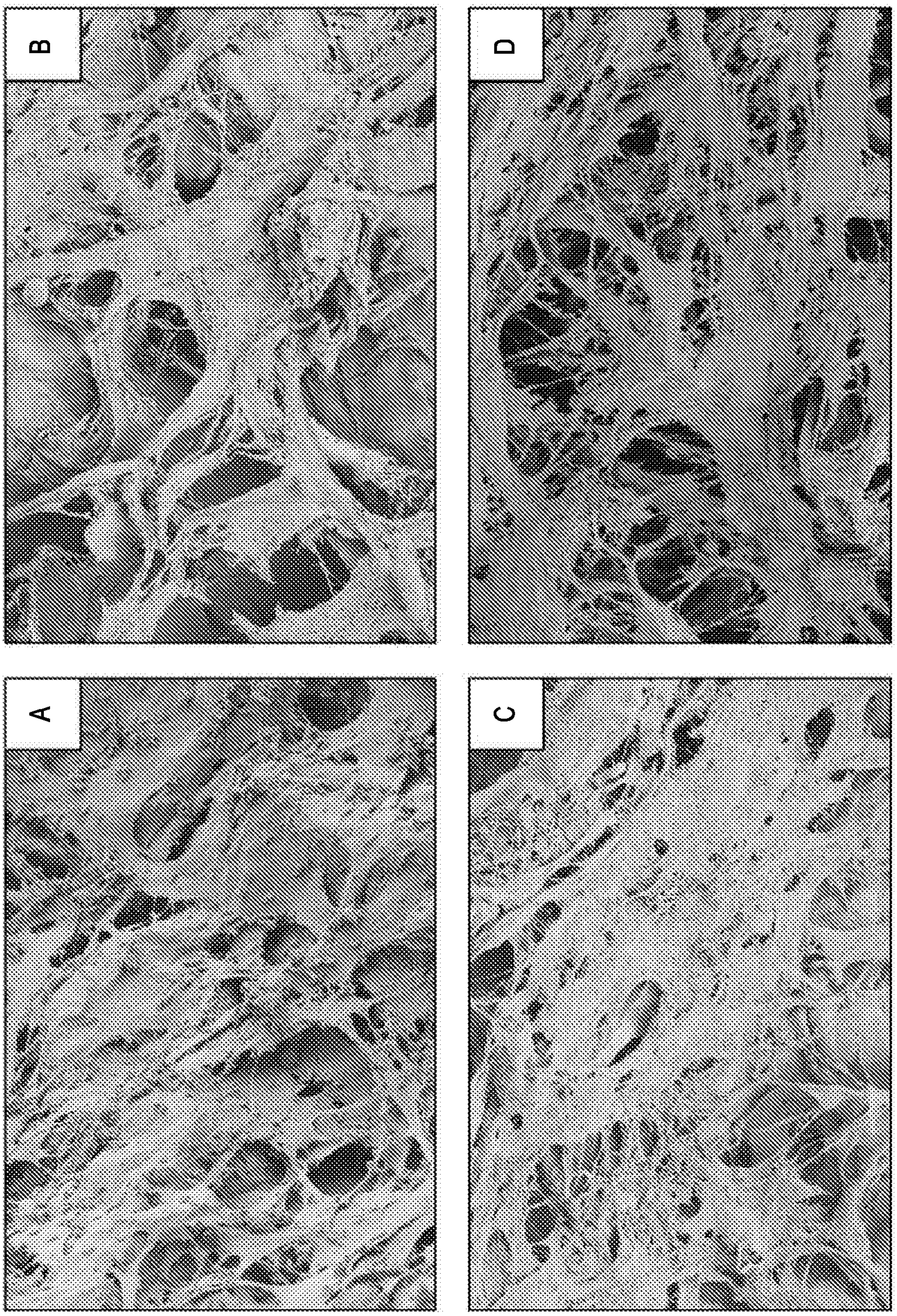


FIG. 30

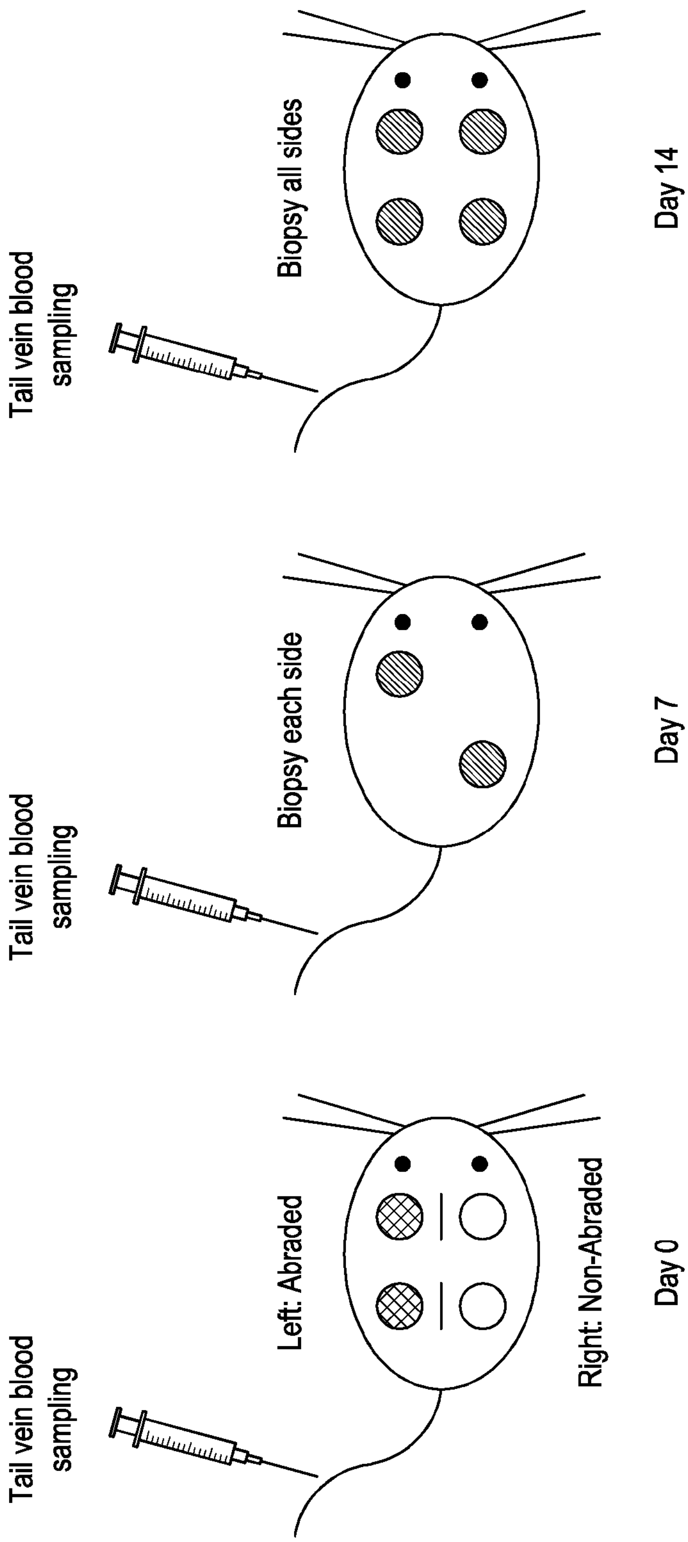
Tail vein blood sampling
Left: Abraded
Right: Non-Abraded
Day 0
Tail vein blood sampling
Biopsy each side
Day 7
Tail vein blood sampling
Biopsy all sides
Day 14

FIG. 31

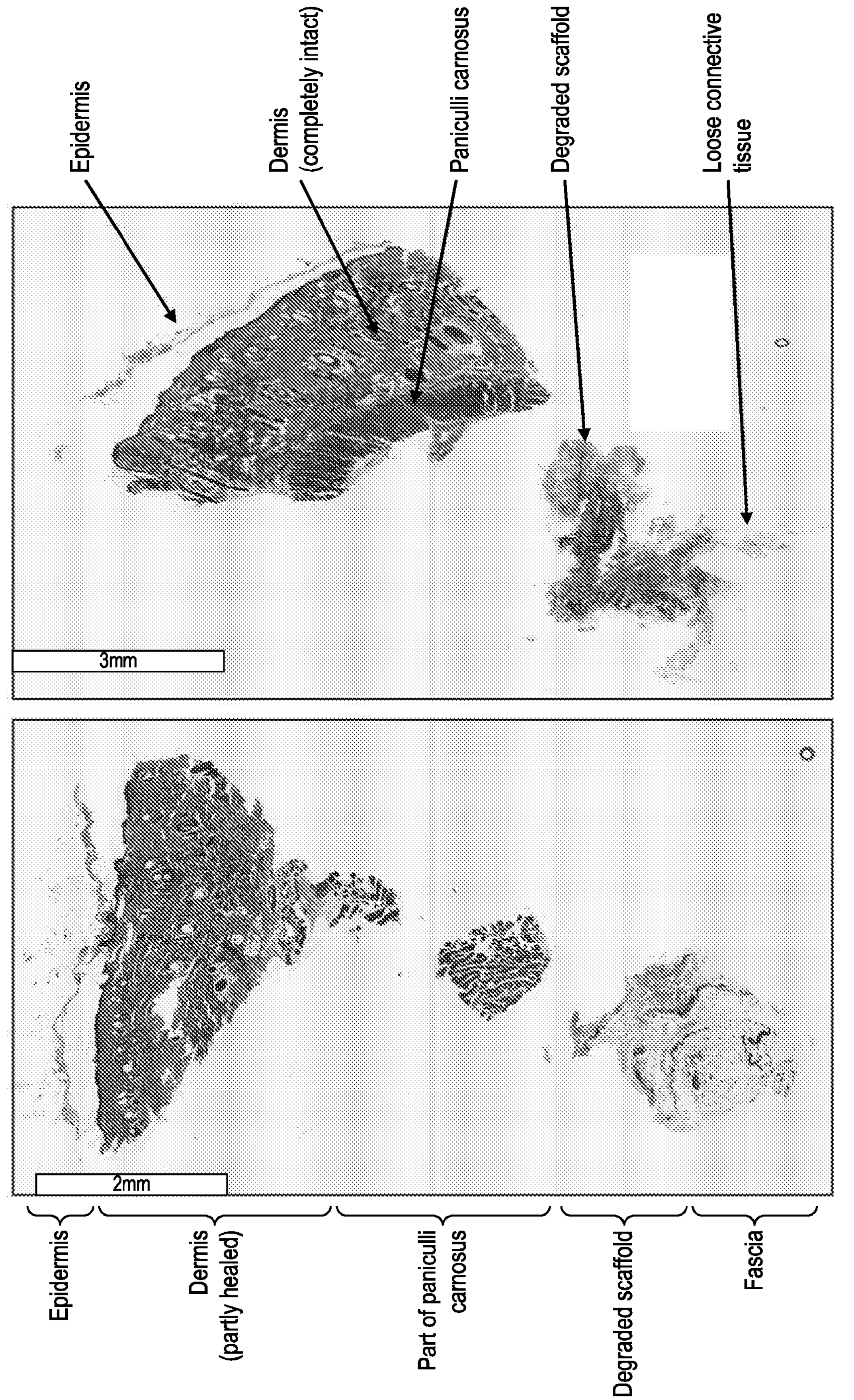
Epidermis
Dermis
(completely intact)
Paniculli carnosus
Degraded scaffold
Loose connective
tissue
3mm
2mm
Epidermis
Dermis
(partly healed)
Part of paniculli
carnosus
Degraded scaffold
Fascia

FIG. 32

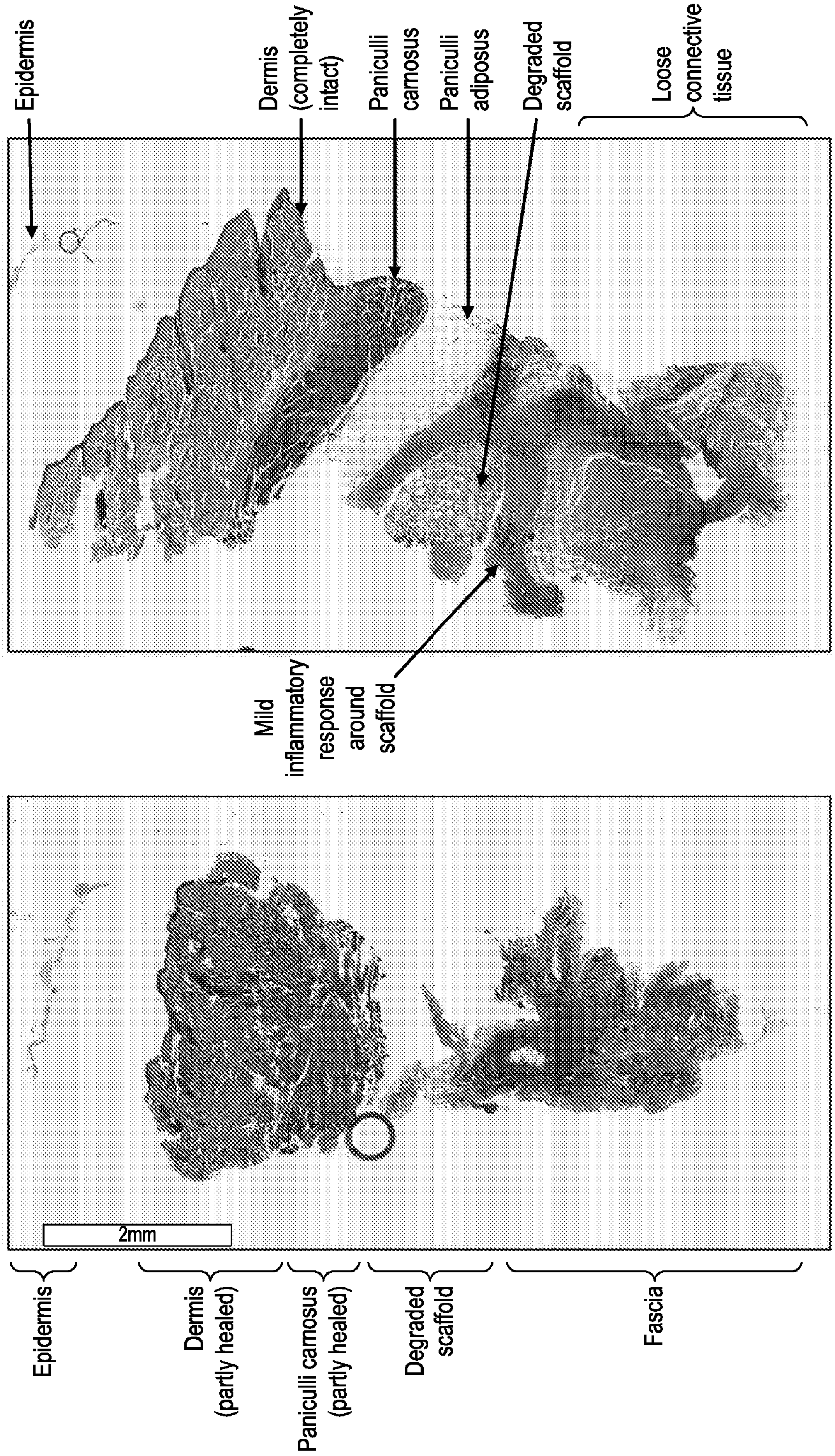
Epidermis
Dermis (completely intact)
Paniculli carnosus
Paniculli adiposus
Degraded scaffold
Loose connective tissue
Mild inflammatory response around scaffold
2mm
Epidermis
Dermis (partly healed)
Paniculli carnosus (partly healed)
Degraded scaffold
Fascia

FIG. 33

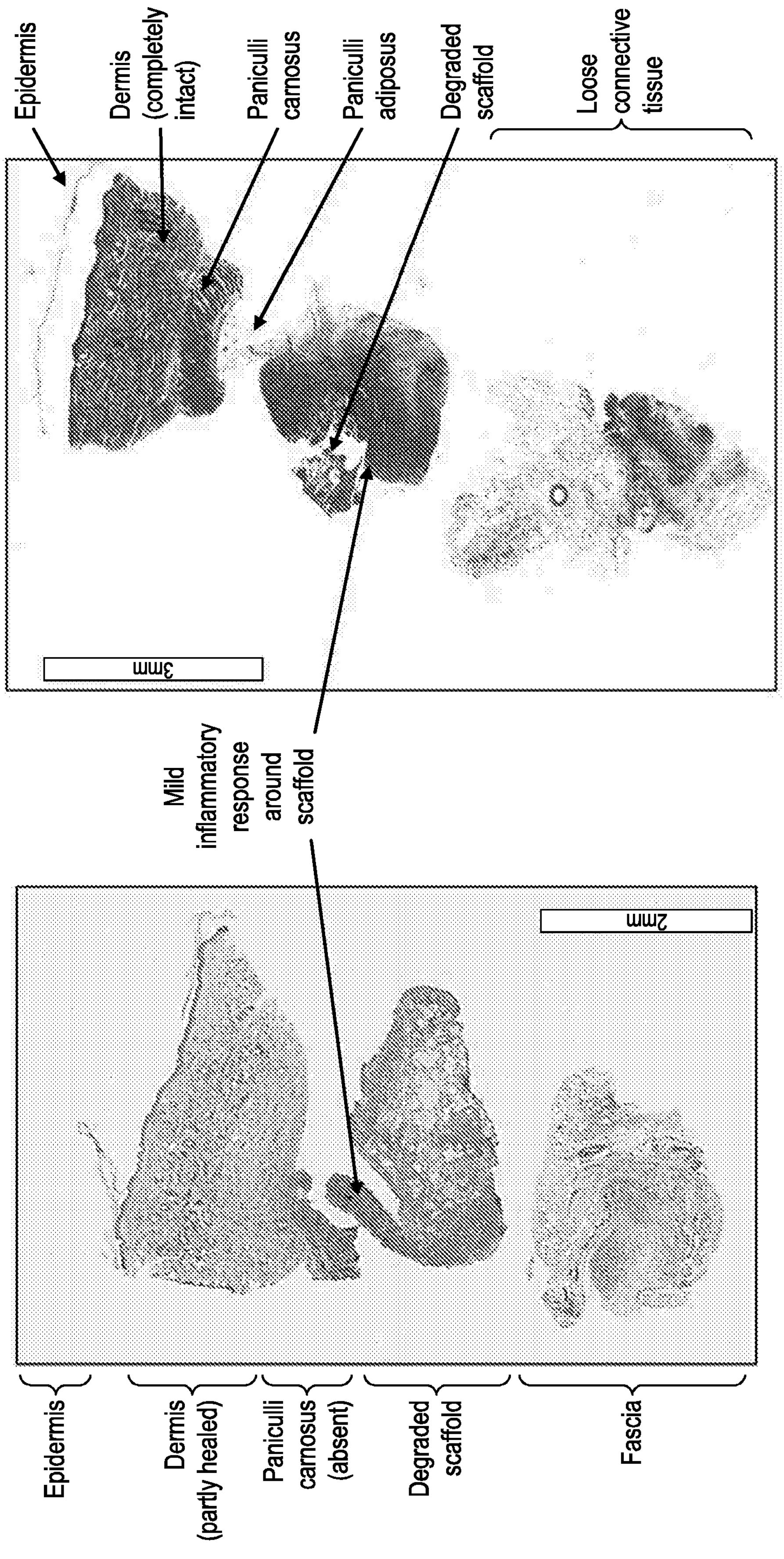
Epidermis
Dermis (partly healed)
Paniculli carnosus (absent)
Degraded scaffold
Fascia
2mm
Mild inflammatory response around scaffold
3mm
Epidermis
Dermis (completely intact)
Paniculli carnosus
Paniculli adiposus
Degraded scaffold
Loose connective tissue

*FIG. 34*

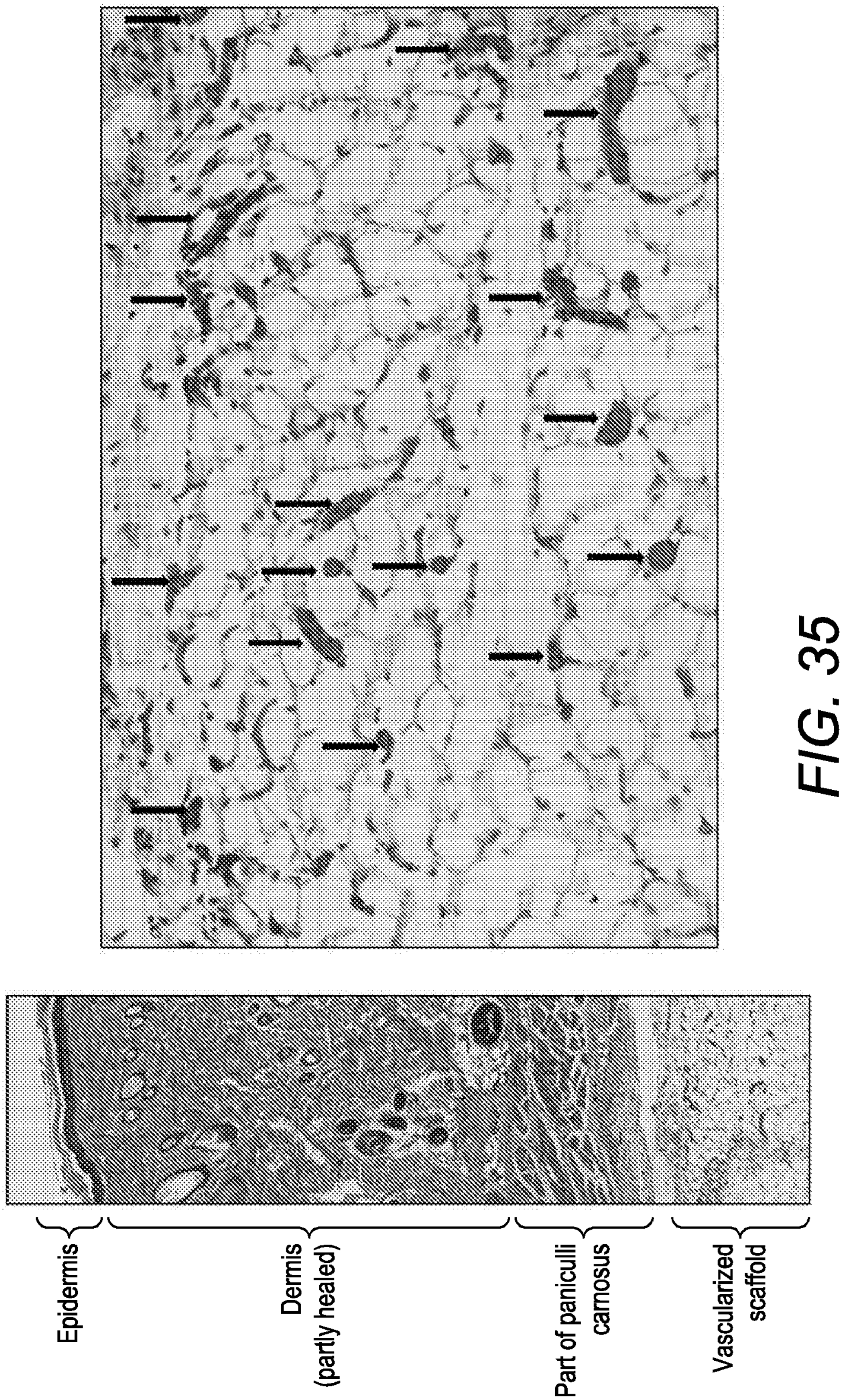
Epidermis
Dermis
(partly healed)
Part of paniculli
carnosus
Vascularized
scaffold

*FIG. 35*

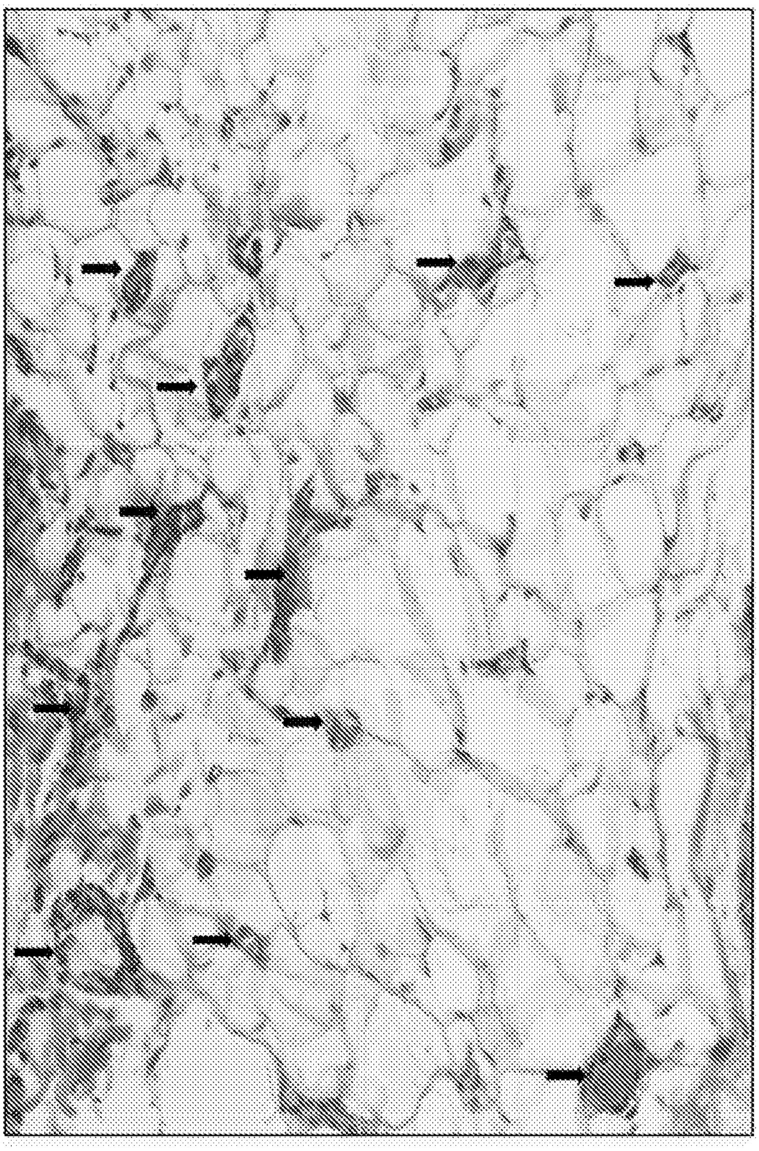

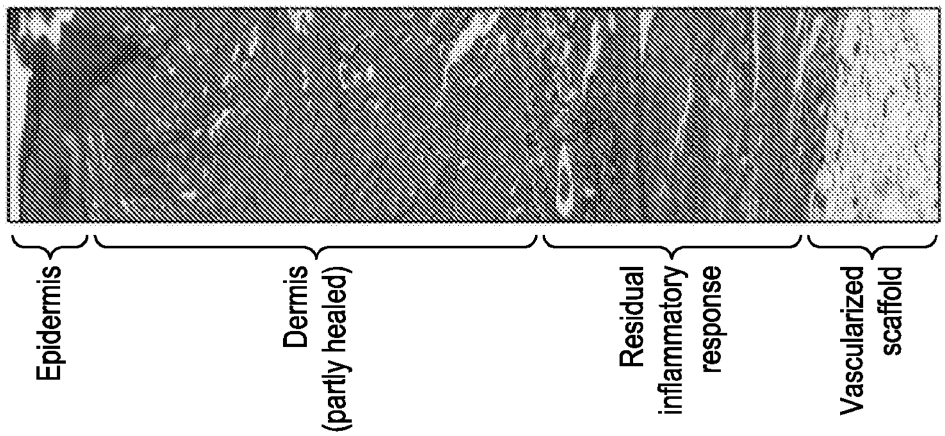
Epidermis
Dermis
(partly healed)
Residual
inflammatory
response
Vascularized
scaffold

FIG. 36

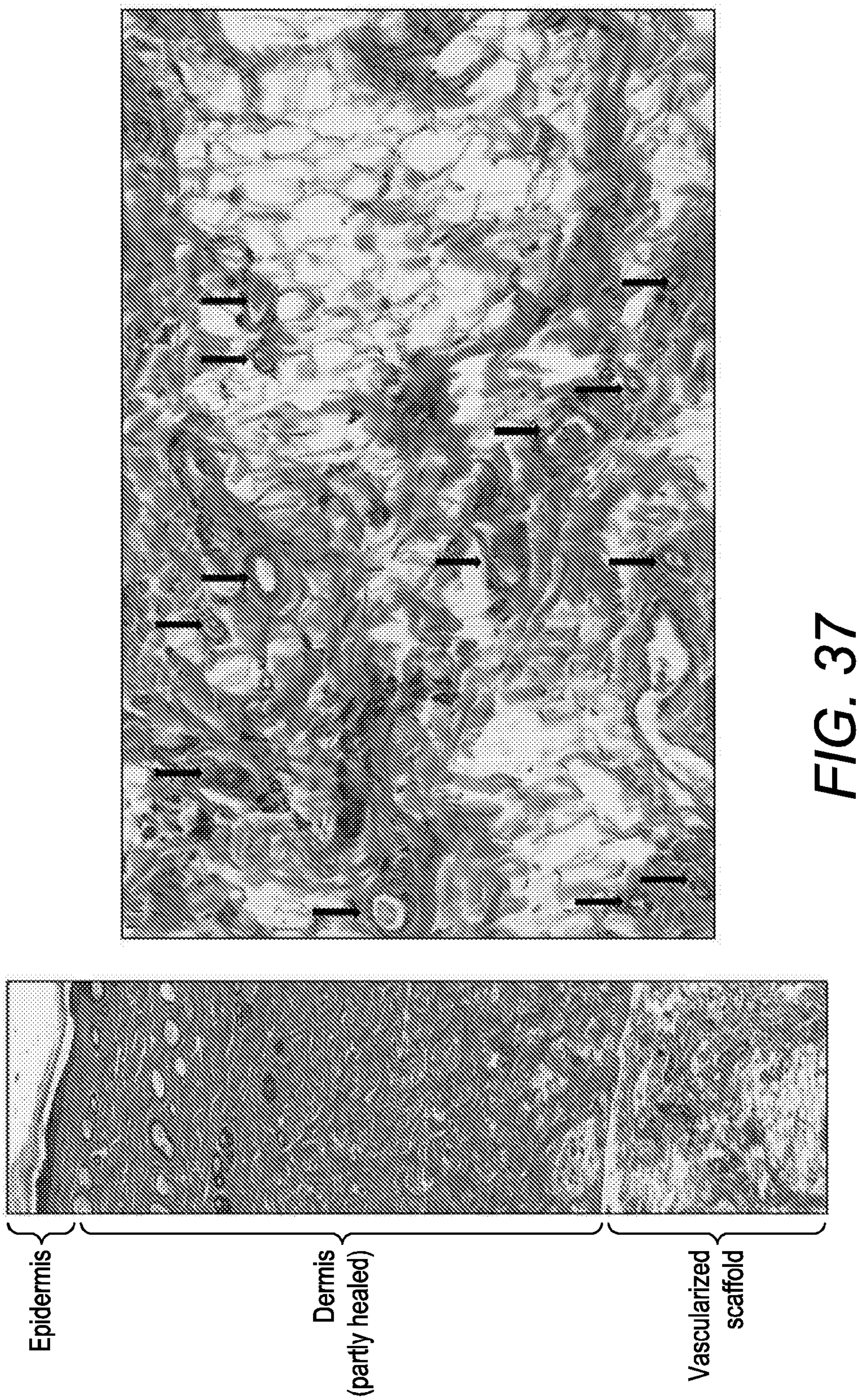


FIG. 37

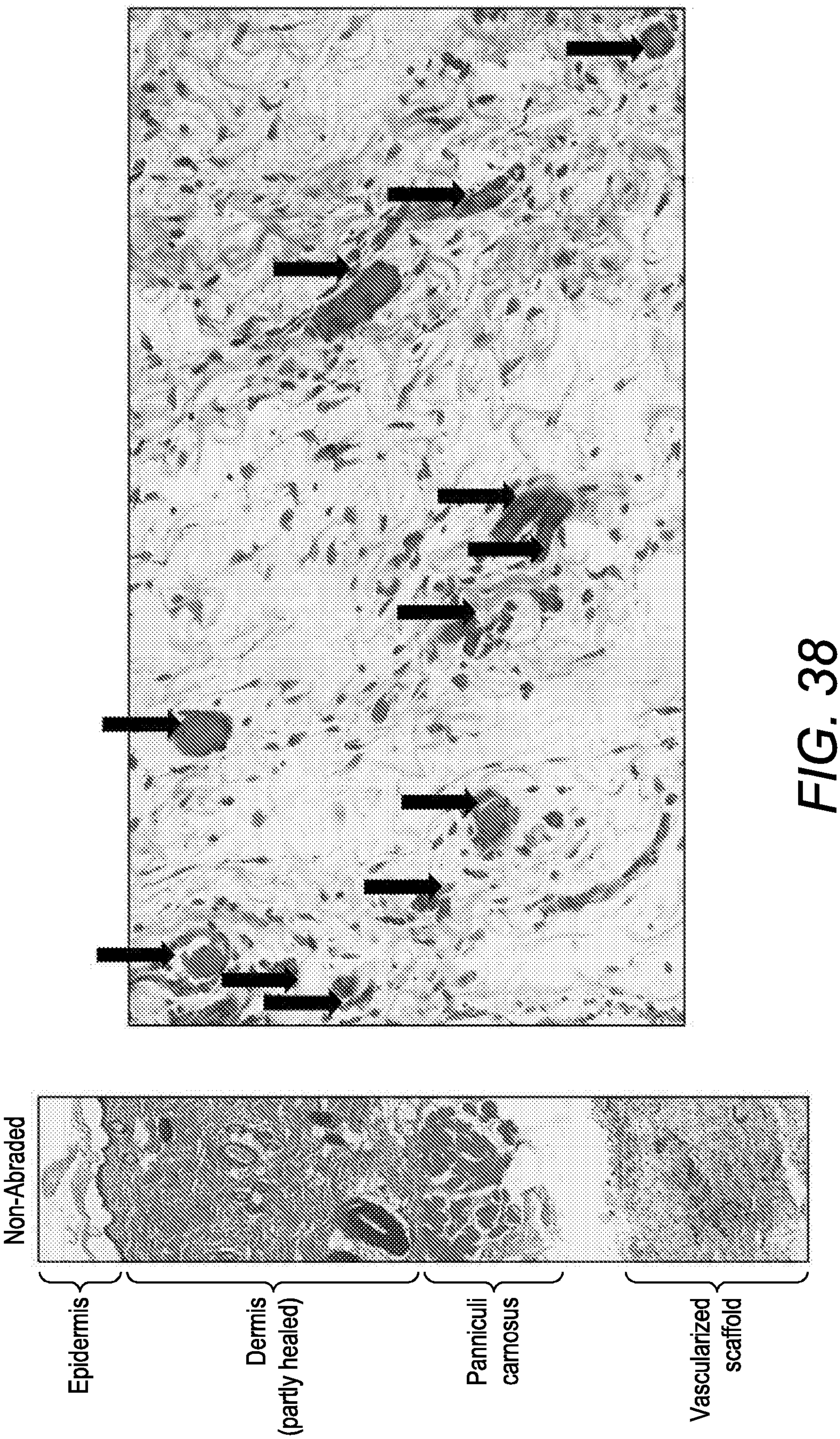

Non-Abraded
Epidermis
Dermis
(partly healed)
Panniculi
carnosus
Vascularized
scaffold


FIG. 38

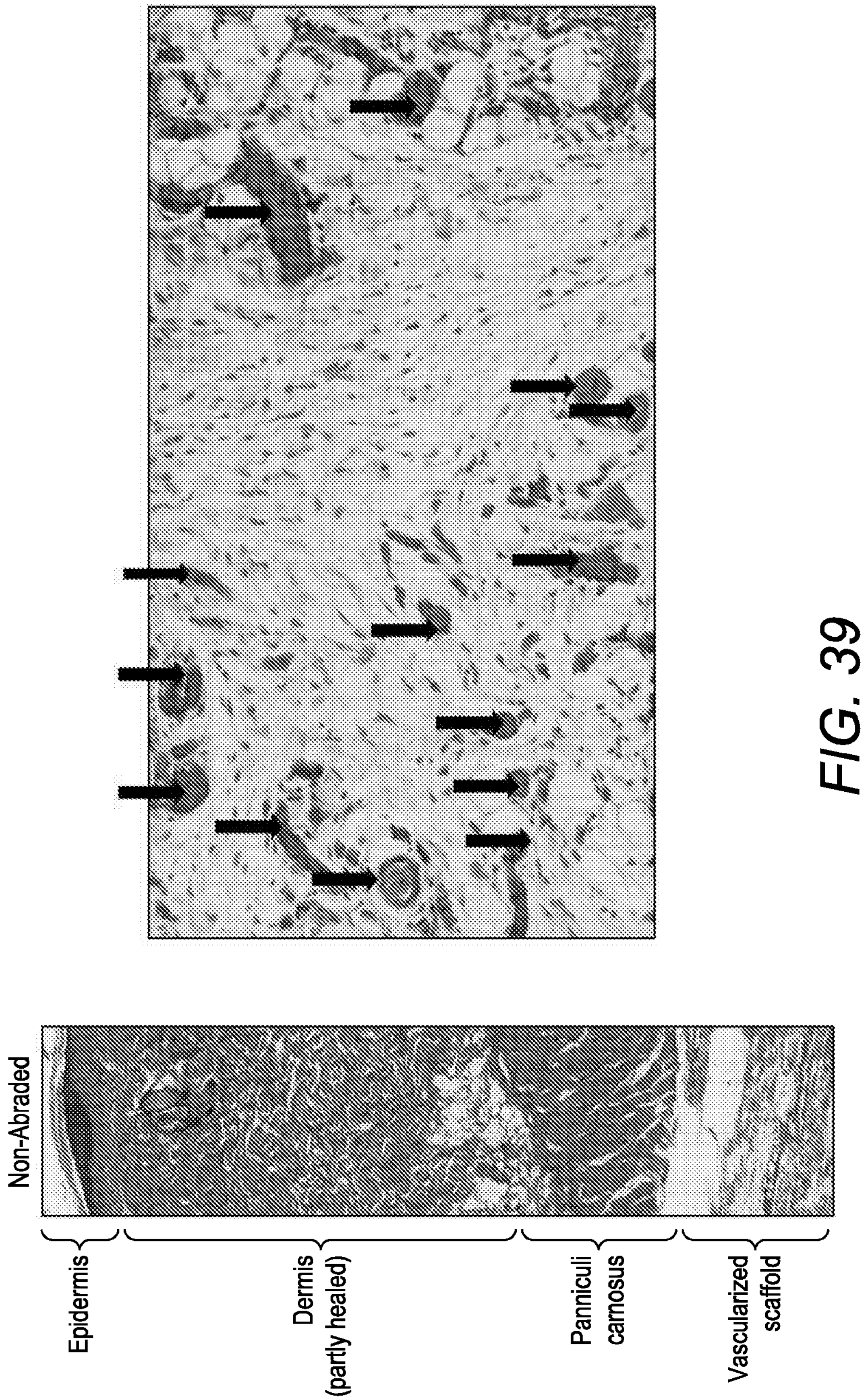
Non-Abraded
Epidermis
Dermis
(partly healed)
Panniculi
carnosus
Vascularized
scaffold

FIG. 39

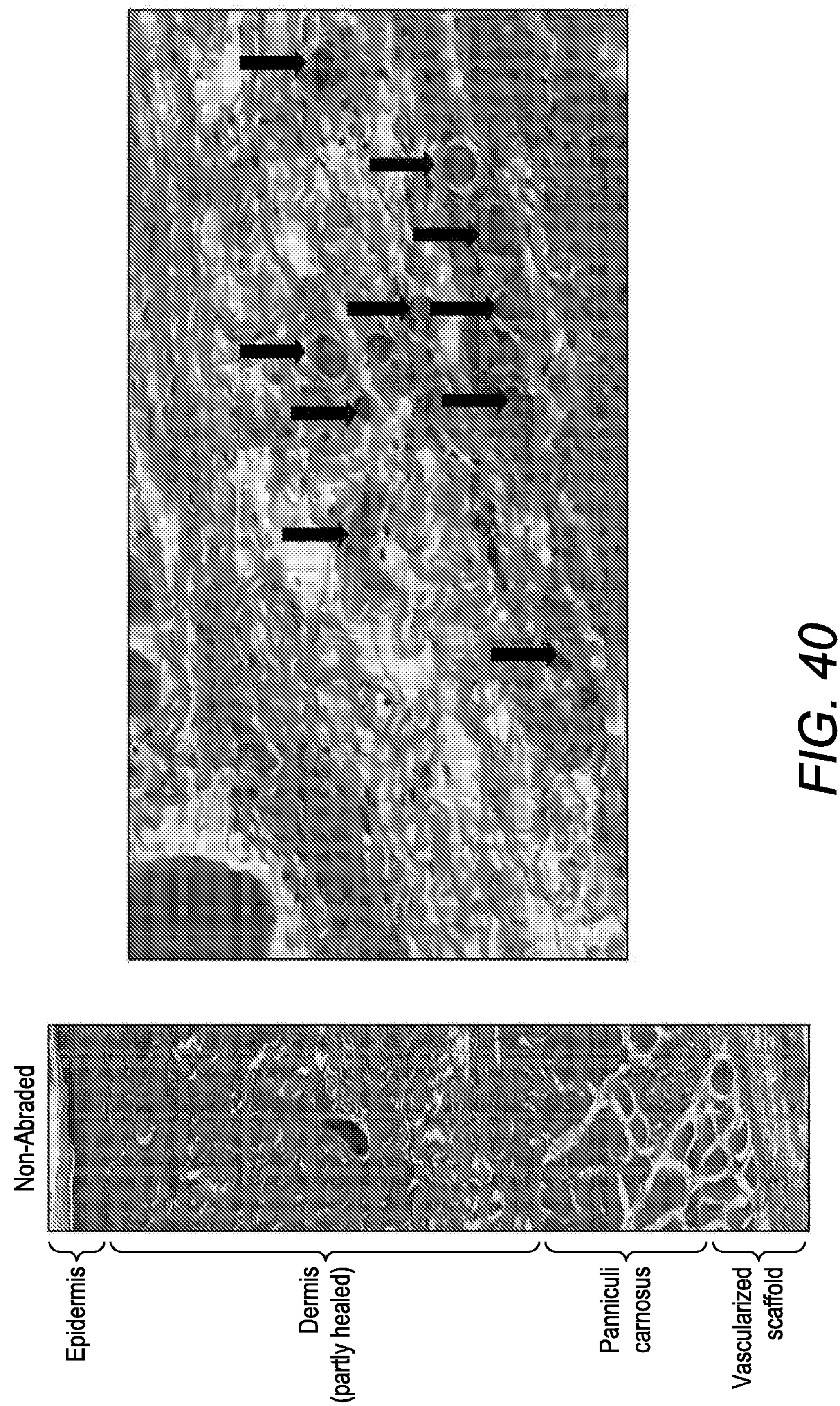
Non-Abraded
Epidermis
Dermis
(partly healed)
Panniculi
carnosus
Vascularized
scaffold

FIG. 40

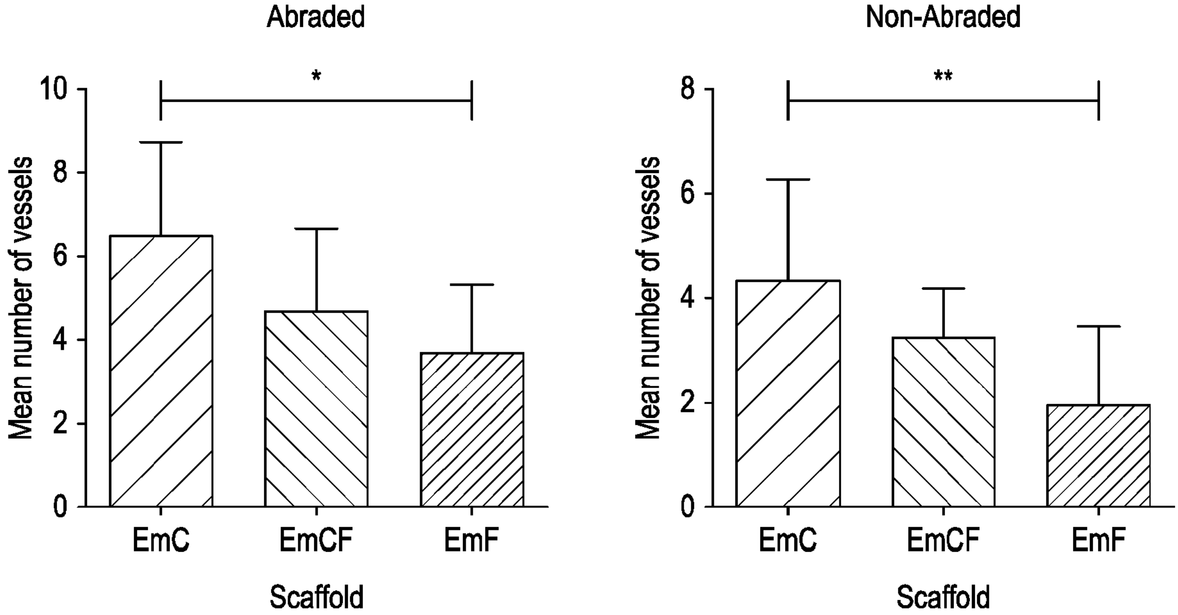
Abraded
Non-Abraded
*
**
Mean number of vessels
10
8
6
4
2
0
EmC
EmCF
EmF
Scaffold

FIG. 41

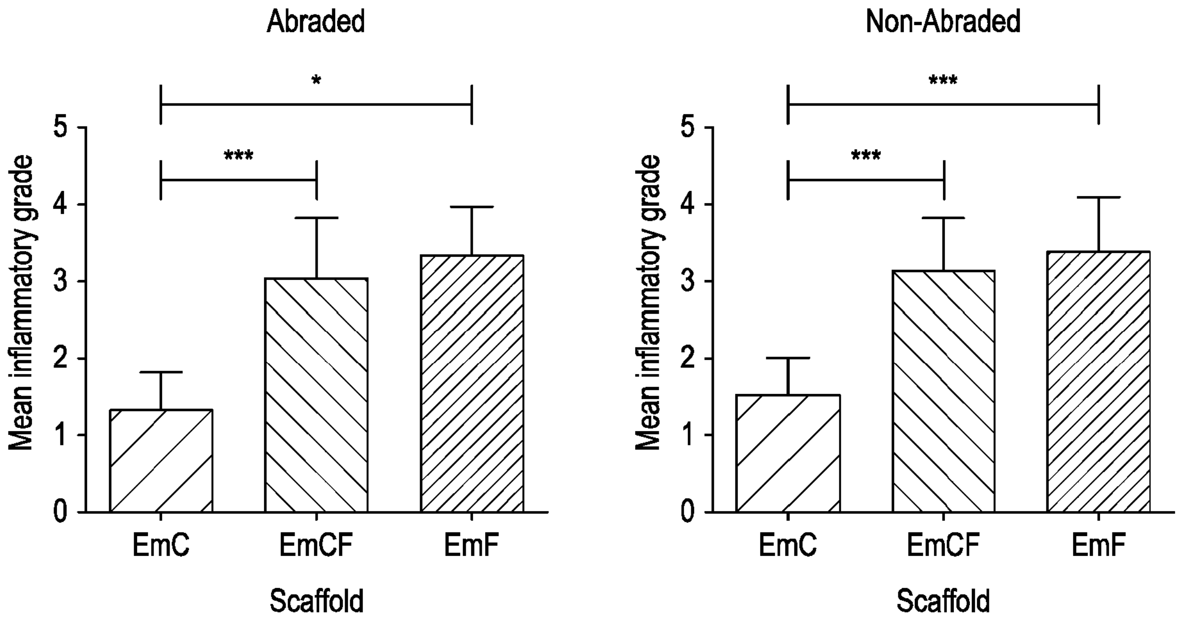
Abraded
Non-Abraded
*
***
Mean inflammatory grade
5
4
3
2
1
0
EmC
EmCF
EmF
Scaffold

FIG. 42

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2009017092 A
- WO 2017182676 A
- WO 2013164635 A
- WO 2017183710 A


### Non-patent literature cited in the description


- **A. BARBETTA et al.** *Biomacromolecules,* 2006, vol. 7 (11), 3059-3068
- **M. DE COLLI et al.** *Biomed. Mater.,* 2012, vol. 7 (5), 055005